(19) Europäisches Patentamt
European Patent Office
Office européen des brevets

(11) **EP 1 624 812 B1**

(12) **EUROPEAN PATENT SPECIFICATION**

(45) Date of publication and mention
of the grant of the patent:
**15.04.2015 Bulletin 2015/16**

(21) Application number: **04709755.5**

(22) Date of filing: **10.02.2004**

(51) Int Cl.:
*A61B 17/15* (2006.01)     *A61B 17/56* (2006.01)
*A61B 19/00* (2006.01)

(86) International application number:
**PCT/JP2004/001440**

(87) International publication number:
**WO 2004/071309 (26.08.2004 Gazette 2004/35)**

(54) **MEMBERS, APPARATUS AND PROGRAM FOR BONE CORRECTION**

ELEMENTE, GERÄT UND PROGRAMM FÜR DIE KNOCHENKORREKTUR

ELEMENTS ET APPAREIL POUR CORRIGER UN OS, ET PROGRAMME POUR DETERMINER UN TEL TRAITEMENT

(84) Designated Contracting States:
**AT BE BG CH CY CZ DE DK EE ES FI FR GB GR HU IE IT LI LU MC NL PT RO SE SI SK TR**

(30) Priority: **12.02.2003 JP 2003001455**

(43) Date of publication of application:
**15.02.2006 Bulletin 2006/07**

(73) Proprietor: **Murase, Tsuyoshi**
**Toyonaka-city,**
**Osaka 5600003 (JP)**

(72) Inventor: **Murase, Tsuyoshi**
**Toyonaka-city,**
**Osaka 5600003 (JP)**

(74) Representative: **BRP Renaud & Partner mbB**
**Rechtsanwälte Patentanwälte**
**Steuerberater**
**Königstraße 28**
**70173 Stuttgart (DE)**

(56) References cited:
WO-A-02/37935        GB-A- 2 334 214
US-A- 4 565 191      US-A- 5 021 056

- **MITTELMEIER W ET AL: "Rapid Prototyping. Modelherstellung zur präoperativen Planung von rekonstruktiven Beckeneingriffen" DER ORTHOPADE. MAR 1997, vol. 26, no. 3, March 1997 (1997-03), pages 273-279, XP002287596 ISSN: 0085-4530**
- **MURPHY S.B. ET AL.: "The planning of orthopaedic reconstructive surgery using computer-aided simulation and design" COMPUTERIZED MEDICAL IMAGING AND GRAPHICS : THE OFFICIAL JOURNAL OF THE COMPUTERIZED MEDICAL IMAGING SOCIETY. 1988 JAN-FEB, vol. 12, no. 1, January 1988 (1988-01), pages 33-45, XP008032671 ISSN: 0895-6111**
- **ECK M. ET AL.: "Three-dimensional determination of an oblique osteotomy in the hip by mathematical optimization fulfilling some anatomical demands" JOURNAL OF BIOMECHANICS. 1990, vol. 23, no. 10, 1990, pages 1061-1067, XP008032680 ISSN: 0021-9290**
- **ZDRAVKOVIC V ET AL: "COMPUTER-ASSISTED PREOPERATIVE PLANNING (CAPP) IN ORTHOPAEDIC SURGERY" COMPUTER METHODS AND PROGRAMS IN BIOMEDICINE, ELSEVIER, AMSTERDAM, NL, vol. 32, no. 2, June 1990 (1990-06), pages 141-146, XP001050306 ISSN: 0169-2607**

**EP 1 624 812 B1**

**Description**

TECHNICAL FIELD

The present invention relates to an osteotomy assisting member used for correcting a bone deformed by fracture or the like into a normal form. The present invention also relates to an apparatus for making an osteotomy assisting member and to a program for making an osteotomy assisting member.

BACKGROUND ART

The following description includes information which is considered to be useful for understanding the present invention.

Conventionally, a bone deformity caused by fracture is healed by: first performing preoperative planning using x-rays, CT (computed tomography) images, perspective drawings which are two-dimensional images, and then cutting and correcting bones. Bone is deformed three-dimensionally, and it is difficult to accurately simulate three-dimensional correction osteotomy surgical operations. Currently, actual surgical operations have many defects that, for example, the bone is cut at an inappropriate position, and correction is insufficient.

US 4,565,191 discloses an osteotomy assisting member comprising a drill jig, a osteotomy guide and a osteometric compression plate, wherein the osteotomy guide can be placed on the drill jig by means of drill bits in order to define a cutting slit for an osteometric saw. The preamble of claim 1 is based on the disclosure of this document.

Other osteotomy assisting members are disclosed in WO 02/37935 A and US 5,021,056 A. The preamble of claim 2 is based on the disclosure of WO 02/37935 A.

The present invention has an objective of providing simple and accurate treatment for a bone deformity.

DISCLOSURE OF THE INVENTION

The present invention unexpectedly has realized accurate and easy correction of a deformed bone by creating a bone model directly using three-dimensional data and directly using a difference between a target bone model, representatively showing a shape of a normal bone, and a bone model which is a model of a bone as a subject of treatment (e. g., a malunited bone). According to the present invention, correction is accurately simulated three-dimensionally, and an assisting member for realizing the correction is designed as necessary. It has been found that accurate correction osteotomy surgical operations which were conventionally impossible is realized in this manner.

In more detail, one feature of the disclosed method is that a difference between a target bone model, representatively showing a shape of a normal bone, and a bone model which is a model of a bone as a subject of treatment (e. g., a malunited bone) is calculated by using the Screw Displacement-Axis method or the affine transformation method, and the difference is compensated for by, for example, rotation, graft insertion, or excision. It has been demonstrated that by performing calculations for rotation, graft insertion, excision or the like directly based on the target bone model and the bone model and executing the treatment according to the present invention, the corrected state of the bone is unexpectedly maintained several weeks or even several months later. Thus, the present disclosure provides a simple and more accurate method for performing a surgical operation on a body. The present invention provides technology for correcting an abnormal bone such as a deformed bone into a normal shape by cutting the bone substantially once.

The present invention provides an osteotomy assisting member according to claim 1, an apparatus for making an osteotomy assisting member according to claim 2, and a program for making an osteotomy assisting member according to claim 4.

BRIEF DESCRIPTION OF THE DRAWINGS

Figure 1 is a front view of an osteotomy assisting member according in an example according to the present invention.

Figure 2 is a rear view of the osteotomy assisting member in the example.

Figure 3 is a side view of a rod in the example.

Figure 4 is a perpective view of a correction guide in the form of a block in the example.

Figures 5 through 11 illustrate a method for producing an osteotomy assisting member in the example.

Figure 12 illustrates how to produce a block in the example.

Figures 13 through 17 illustrate a procedure for performing a correction osteotomy surgical operation using the osteotomy assisting member in the example.

Figure 18 shows a state of a bone after the correction osteotomy surgical operation performed using the osteotomy assisting member in the example.

Figure **19** shows a state of a bone before the correction osteotomy surgical operation performed using the osteotomy assisting member in the example.

Figure **20** shows a simulation for finding a correction position of a bone in another example according to the present invention.

Figure **21** shows an example of a preoperative computer simulation.

Figure **22A** shows two-dimensional data of a CR or MRI image of both forearm.

Figure **22B** shows an exemplary procedure for semi-automatically marking and extracting a bone as a subject of treatment.

Figure **22C** shows a segmented model.

Figure **22D** shows an example of a created three-dimensional surface model of the bone.

Figure 23 shows an exemplary optimum cutting position of the bone and correction amount which have been determined.

Figure **24** shows an exemplary procedure performed with reference to the site and amount of deformity obtained by the simulation and computer images.

Figure **25A** shows an exemplary cutting position of the bone and correction amount which have been determined based on CT data.

Figure **25B** shows designing of the osteotomy template.

Figure **25C** shows a photo printout model.

Figure **26** is a table summarizing the results of group A and group B in Example 1.

Figure **27** shows x-rays of the affected site of a patient in Example 2 (Left photo shows a front view, and right photo shows a side view.)

Figure **28** shows a progress of hyper-extension in the cast.

Figure **29** shows the state of the patient in Example 2 years after the initial treatment.

Figure **30** shows an aftereffect of hyper-extension and varus deformity.

Figure **31** shows an exemplary surgical operation performed on the patient in Example 2 using a three-dimensional model produced based on MRI.

Figure **32** shows a photo printout model produced in Example 2.

Figure **33** shows intraoperative photos of the malunited bone performed on the patient in Example 2.

Figure **34** shows bone cutting performed by a bone saw.

Figure **35** shows a state after the bone cutting.

Figure **36** shows excision of an extra bone portion.

Figure **37** shows bone correction in Example 2.

Figure **38** shows x-rays immediately after the surgical operation in Example 2.

Figure **39** shows a movable range of the elbow immediately after the surgical operation in Example 2.

Figure **40** shows an external appearance immediately after the surgical operation in Example 2.

Figure **41** shows x-rays 1 year after the surgical operation in Example 2.

Figure **42** shows external appearances of the patient in Example 2.

Figure **43** shows an affected site of a 21-year-old male patient having a fraction malunion on the forearm in Example 3.

Figure **44** shows a curve and restriction of supination of forearm of the patient in Example 3.

Figure **45** shows a computer simulation in Example 3.

Figure **46** shows designing of a template in Example 3.

Figure **47** shows the malunited bone which is exposed in Example 3.

Figure **48** shows attachment of the template in Example 3.

Figure **49** shows bone cutting in Example 3.

Figure **50** shows removal of the template in Example 3.

Figure **51** shows bone correction by a correction guide in Example 3.

Figure **52** is an x-ray showing the correction result in Example 3.

Figure **53** shows implantation of a graft in Example 3.

Figure **54** shows fixation of a bone and a graft using a template in Example 3.

Figure **55** shows removal of Kirschner wires from the patient in Example 3.

Figure **56** shows x-rays immediately after the surgical operation in Example 3.

Figure **57** shows x-rays 9 months after the surgical operation in Example 3.

Figure **58** shows x-rays 1 year and 1 month after the surgical operation in Example 3.

Figure **59** shows external appearances of the patient 1 year and 1 month after the surgical operation in Example 3.

Figure **60** shows x-rays of a 48-year-old female patient having a fracture malunion on the distal end of left radius in Example 4.

Figure **61** shows an external deformity and a disorder in the movable range of the left wrist joint of the patient in Example 4.

Figure **62** shows a three-dimensional simulation on the bone in Example 4.

Figure **63** shows an osteotomy template designed in Example 4.

Figure **64** shows a photo printout model in Example 4.

Figure **65** shows exposure of the malunited bone in Example 4.

Figure **66** shows fixation of Kirschnerwires in Example 4.

Figure **67** shows bone correction in Example 4.

Figure **68** shows a graft obtained by shaping hydroxyapatite using CAD used in Example 4.

Figure **69** shows postoperative x-rays in Example 4.

Figure **70** shows x-rays 4 months after the surgical operation in Example 4.

Figure **71** shows external appearances of the patient 4 months after the surgical operation in Example 4.

Figure **72** shows x-rays of a 67-year-old female patient having a fracture malunion on the distal end of the radius in Example 5.

Figure **73** shows an external appearance of the patient in Example 5.

Figure **74** shows the disorder of the patient in Example 5.

Figure **75** shows a three-dimensional osteotomy simulation in Example 5.

Figure **76** shows designing of an osteotomy template in Example 5.

Figure **77** shows designing of a correction guide in Example 5.

Figure **78** shows a model of the corrected state in Example 5.

Figure **79** shows exposure of the malunited bone in Example 5.

Figures **80** and **81** show attachment of the template in Example 5.

Figure **82** shows bone cutting in Example 5.

Figures **83** and **84** show graft implantation in Example 5.

Figure **85** shows x-rays after the template is fixed in Example 5.

Figure **86** shows x-rays 4 months after the surgical operation in Example 5.

Figure **87** shows an image of a normal wrist joint in Example 5.

Figure 88 shows x-rays of a patient in Example 6 having a fracture on the distal end of the radius as a result of a motorbike accident.

Figure **89** shows a deformity of the wrist joint of the patient in Example 6.

Figure **90** shows a restricted forearm rotation of the patient in Example 6.

Figure **91** shows a simulation of treatment of the patient in Example 6.

Figure **92** shows a method for finding a screw axis in the simulation of treatment of the patient in Example 6.

Figure **93** shows osteotomy accompanying distraction in Example 6.

Figure **94** shows the states before and after the correction in Example 6.

Figure **95** shows designing of a template in Example 6.

Figure **96** shows designing of a correction guide in Example 6.

Figure **97** shows a photo printout model produced in Example 6.

Figure **98** shows exposure of the malunited bone in Example 6.

Figure **99** shows fixation of the template to the bone in Example 6.

Figure **100** shows bone cutting (left photo) and fixation with the correction guide (right photo) in Example 6.

Figure **101** shows molding of a graft in Example 6.

Figure **102** shows insertion (left photo) and fixation (right photo) of the graft in Example 6.

Figure **103** shows x-rays immediately after the surgical operation in Example 6.

Figure **104** shows x-rays 9 months after the surgical operation in Example 6.

Figure **105** shows external appearances 1 year after the surgical operation in Example 6.

Figure **106** shows recovery of the movable range 1 year after the surgical operation in Example 6.

Figure **107** shows x-rays of a 13-year-old male patient having a fracture malunion on the left radius diaphysis in Example 7.

Figure **108** shows a pronation disorder of the patient in Example 7.

Figure **109** shows an osteotomy simulation in Example 7.

Figure **110** shows a rotational correction plan in Example 7.

Figures **111** through **114** show designing of a guide for rotational osteotomy in Example 7.

Figure **115** shows external appearances of an osteotomy assisting member on a computer in Example 7.

Figure **116** shows a photo printout produced in Example 7.

Figure **117** shows exposure of a malunited bone in Example 7.

Figure **118** shows fixation of the template in Example 7.

Figure **119** shows the state after the template is removed in Example 7.

Figure **120** shows x-rays after the correction guide is attached in Example 7.

Figure **121** shows fixation of the template in Example 7.

Figure **122** shows x-rays immediately after the surgical operation in Example 7.

Figure **123** shows x-rays 6 months after the surgical operation in Example 7.

Figure **124** shows the symptom of a patient in Example 8.

Figure **125** shows x-rays of the patient in Example 8.

Figure **126** shows an osteotomy simulation in Example 8.

Figure **127** shows designing of a template in Example 8.

Figures **128** and **129** show designing of a correction guide in Example 8.

Figure **130** shows designing of osteotomy in Example 8.

Figure **131** shows a photo printout model produced by in Example 8.

Figure **132** shows a correction guide in Example 8.

Figure **133** shows exposure of a malunited bone in Example 8.

Figure **134** shows attachment of the template in Example 8.

Figure **135** shows bone cutting in Example 8.

Figure **136** shows excision of a wedge-shaped bone in Example 8.

Figure **137** shows restoration after the bone is excised in Example 8.

Figure **138** shows the postoperative correction state in Example 8.

Figure **139** shows the correction state immediately after the surgical operation, 2 month after, and 6 months after the surgical operation in Example 8.

Figure **140** shows a 7-year-old male patient having a fracture of the distal end of the radius in Example 9.

Figure **141** shows the progress of the bone dislocation of the patient in Example 9.

Figure **142** shows the state 1 month after the surgical operation in Example 9.

Figure **143** shows an x-ray 8 months after the injury in Example 9.

Figure 144 shows x-rays 5 years after the injury in Example 9.

Figure **145** shows external appearances of the patient in Example 9.

Figure **146** shows the volar flexion of the wrist joint of the patient in Example 9.

Figure **147** shows a simulation in Example 9.

Figure **148** shows an osteotomy model in Example 9.

Figure **149** shows an external fixation device used in Example 9.

Figure **150** shows parts of the external fixation device used in Example 9. The upper left photo shows a top part of the device, the lower left view is of a bone, and the right photo shows the bone fixed by wires.

Figure **151** shows the external fixation device used in Example 9. The left photo shows the state before correction, and the right photo shows the state after correction.

Figure **152** shows a surgical operation of the surgical operation in Example 9.

Figure **153** shows the state immediately after the surgical operation in Example 9.

Figure 154 shows an x-ray after the surgical operation in Example 9.

Figure **155** shows the state immediately after the distraction started in Example 9.

Figure **156** shows the state immediately after the distraction finished in Example 9.

Figure **157** shows an x-ray 2.5 months after the distraction started in Example 9.

Figure **158** shows removal of the external fixation device 3.5 months after the surgical operation in Example 9.

Figure **159** show comparison of x-rays 4 months after the surgical operation and before the surgical operation in Example 9.

Figure **160** shows comparison of external appearances before the surgical operation and 5 months after the surgical operation in Example 9.

Figure **161** shows improvement of volar flexion of the wrist joint in Example 9, and also shows that the patient has recovered from the restriction of the movable range.

Figure **162A** shows a three-dimensional surface model of the schaphoid re-constructed on a computer in case 3 in Example 10. The left view shows a frame model, and the right view shows a surface model.

Figure **162B** shows a surf ace image obtained by matching the distal portion and the proximal portion of the nonunion model (left) to the opposite normal scaphoid model (right) for simulating restoration of the deformed scaphoid in Example 10. The deformity to be restored is represented as rotation about the Screw Displacement-Axis (center).

Figures **162C** and **163D** show a simulation of estimation of the bone defect (arrow) and screw insertion (arrow) in Example 10. An appropriate site and direction of screw insertion were determined by observing the restored scaphoid model by a transparent mode from various angles.

Figure **163A** shows a photo printout model (hard model) of the scaphoid. The left model is a scaphoid nonunion model, the central model shows a restoration model obtained by appropriate insertion model, and the right model is an mirror image model of the normal scaphoid on the opposite side.

Figure **163B** shows a model of an estimated bone defect.

Figure **164A** shows the scaphoid nonunion of the carpus in the surgical operation in case 7 in Example 10.

Figure **164B** shows an image of the dorsal rotation of the lunate bone seen from the side of the three-dimensional model in the surgical operation in Example 10.

Figure **164C** shows comparison of the nonunited site with that of the hard model regarding the surgical operation in Example 10.

Figures **164D** and **164E** show molding of iliac bone graft performed using a hard model as a reference for the surgical operation in Example 10.

Figure **164F** shows that after the insertion of the iliac bone graft, the site and direction of screw insertion were determined using a hard model for the surgical operation in Example 10.

Figure **164G** shows an x-ray immediately after the surgical operation in Example 10.

Figures **164H** and **164I** show x-rays of anteroposterior and side views 6 weeks after the surgical operation in Example 10.

Figure **165** shows a screw axis of the deformed scaphoid in the case shown in Figures **162A** and **162B** (case 3 in Example 10).

Figure **166A** shows that, in case 4 in Example 10, osteophyte was observed on the dorsal side of the scaphoid in the three-dimensional model but was not clear in the simple x-ray before the surgical operation.

Figure **166B** shows that, in case 4 in Example 10, osteophyte was observed on the dorsal side of the scaphoid in the three-dimensional model but was not clear in the simple x-ray after the surgical operation.

Figure **167** shows the three-dimensional images used for case 4 in Example 10.

Figure **168** shows an x-ray of the left forearm, in which the ulna is internally curved than in the normal state (arrow).

Figure **169** shows an x-ray of the normal side.

Figure **170** is a photo of a patient of Example 11, showing the supination of the left forearm is impossible.

Figure **171** is a photo showing that the pronation of the patient is slightly restricted.

Figure **172** shows a three-dimensional model of the affected side in Example 11.

Figure **173** shows a mirror image model of the healthy side in Example 11.

Figure **174A** shows closed wedge osteotomy planned for the ulna in Example 11. The dot represents the screw axis.

Figure **174B** shows the step of cutting the bone along the plane passing through the screw axis and excising a wedge-shaped bone portion having an angle of 13 degrees in the closed wedge osteotomy in Example 11.

Figure **174C** shows the correction osteotomy.

Figures **174D** and **174E** show the step of implanting a wedge-shaped graft in the defect which is generated after the correction in the closed wedge osteotomy in Example 11.

Figure **175A** shows the osteotomy design by which the bone is cut along an appropriate arch having the screw axis at the center to provide a dome-shaped bone fragment.

Figure **175B** shows that the upper bone fragment is rotated at 13 degrees, and thus correction is completed.

Figure **176A** shows an osteotomy template seen from the dorsal side in Example 11.

Figure **176B** shows an osteotomy template seen from the volar side in Example 11.

Figure **176C** shows a correction guide seen from the dorsal side in Example 11.

Figure **176D** shows the correction guide seen from the volar side in Example 11.

Figure **177A** shows the intraoperative step of exposing the deformed site in an osteotomy operation performed on the ulna.

Figure **177B** shows the step of applying an osteotomy template to the site and fixing the template with Kirschner wires angled at 13 degrees in the osteotomy operation performed on the ulna.

Figure **177C** shows the step of arranging the Kirschner wires to be parallel to each other after the cutting in the osteotomy operation performed on the ulna.

Figure **177D** shows the step of fixing the template in the state shown in Figure 177C and filling a bone defect generated on the side closer to the operator with a wedge-shaped bone excised from the deeper side in the osteotomy operation performed on the ulna.

Figure **178** shows an x-ray after osteotomy was performed by a combination of closed wedge osteotomy and open wedge osteotomy.

Figure **179** shows an exemplary structure of a computer 1000 for executing processing for determining a treatment process to be performed on a bone.

Figure **180** shows an exemplary procedure of processing for determining the treatment process to be performed on the bone.

Figure **181** shows an exemplary procedure of processing for performing the step **2030** shown in Figure **180.**

Figure **182** shows an exemplary procedure of processing for determining a direction and an amount of movement of a distal bone fragment model with reference to a proximal bone fragment model.

BEST MODE FOR CARRYING OUT THE INVENTION

The present invention will be described below. It should be understood throughout the present specification that articles for singular forms include the concept of their plurality unless otherwise mentioned. Therefore, articles or adjectives for singular forms (e.g., "a" , "an" , "the", and the like in English) include the concept of their plurality unless otherwise specified. Also, it should be also understood that terms as used herein have definitions ordinarily used in the art unless otherwise mentioned. Therefore, all technical and scientific terms used herein have the same meanings as commonly understood by those skilled in the relevant art. Otherwise, the usage in this specification (including definitions) takes precedence.

(Definitions)

Hereinafter, definitions of the terms specifically used in this specification will be described.
As used herein, the term "bone model" used for bones refers to a model representing a current state of a bone as a subject of treatment. A bone model is usually represented three-dimensionally.
As used herein, the term "target bone model" refers to an image showing a shape which should be obtained after the treatment performed by the method for treating a bone according to the present disclosure. A target bone model is, for example, a normal bone model, but is not limited to this. A target bone model is usually represented three-dimensionally by the same representation technique as that of the bone model. An existing model is usable as a target bone model. Alternatively, a target bone model may be manually created, but usually created using a computer.
In this specification, the "three-dimensional representation" is usually performed using an orthogonal system, but any system is usable as long as three-dimensional representation is possible.
As used herein, the term "bone" refers to a supportive organ of vertebrate which is an individual component of an endoskeleton. Bones of vertebrate are mainly composed of bones except for marsipobranch and cartilage fish. Herein, the term "bone" encompasses cartilage. Herein, a hard connective tissue which forms the majority of the skeleton of vertebrate is specifically referred to as a "hard bone". This specification describes bones as an example, but it should be understood that treatment may be designed and carried out by the present invention for other parts of the body than bones.
The bone used as a subject of treatment according to the present disclosure is often an abnormal bone, and representatively is a bone which has experienced fracture. Fracture is classified into complete fracture and incomplete fracture, or into open fracture which accompanies skin damage and closed fracture. Abnormal bones which have been healed from such a fracture can all be subjects of treatment according to the present disclosure. A bone is usually healed in 6 weeks to 6 months after fracture, during which time the bone is healed by formation of new bone in the crevice or a bone deformity. A bone which is healed by first intention has broken bone fragments tightly fit each other and is difficult to be deformed. When broken bone fragments do not fit each other, ends of broken bone fragments are stimulated and the bone fragments are gradually joined through a cartilage-type callus. This is referred to as "healing by second intention". A bone is healed by second intention is often deformed when healed.
As used herein, the expression "method for treating a bone" involves treatment processes to be performed on a bone, which is a subject of bone treatment and for which a bone model has been created, in order to guide the bone to the target bone model (e.g., cutting, insertion and/or excision, translation, rotation) and the shape of an assisting member required for the method (e.g., template assisting member, correction assisting member, graft to be inserted).
As used herein, the term "assisting member" refers to a member used for surgical operations of a bone performed according to the disclosed method. Examples of the assisting member include, for example, a template assisting member, a graft, a correction position determination assisting member, a fixation assisting member, a correction assisting member, and a translation assisting member, but is not limited to these.
As used herein, the term "parameter" in the three-dimensional direction regarding a bone refers to a factor which represents each of the dimensions in the three-dimensional representation. For example, when a space is represented by a normal orthogonal system, the parameter is a factor regarding each of x, y and z axes (for example, a vector). The space which is represented by x, y and z axes may be alternatively represented by rotation axis, rotation angle and distance.

As used herein, the term "rotation axis" refers to one of the symmetric factors of a point group. For example, when a rigid body (e.g., a bone) is rotated, a set of points which does not move during the rotation is the rotation axis. It is assumed that a construct is entirely rotated at a certain angle using a straight line as an axis. If the post-rotation construct matches the pre-rotation construct, this axis is the rotation axis. In the disclosed surgical operation method , the term "rotation axis" specifically refers to a rotation axis when a portion of a bone model needs to be rotated in order to realize a target bone model.

As used herein, the term "screw displacement" refers to a displacement such that a rotation of a rigid body about an axis accompanies a translation along the axis.

As used herein, the term "Screw Displacement-Axis" is also referred to as a "screw axis". The "Screw Displacement-Axis" refers to a rotation axis of the screw displacement. Such a rotation axis is one of the symmetric factors of a space group. It is assumed that a construct is entirely rotated about a certain straight line as an axis at a certain angle and then is translated parallel to the axis, and this series of operations is repeated. If the resultant construct matches the original construct, the axis is the screw axis. Processing performed using this rotation axis is referred to as the "Screw Displacement-Axis" method. According to the Screw Displacement-Axis method, the displacement can be represented using the vector representation of the rotation axis, the moving distance of the vector, and the like. The Screw Displacement-Axis method, which is one method of representing an object movement, is used in geometry, but is never used for surgical operations.

As used herein, the term "affine transformation method" means a method of representing a displacement, by which translation for moving the origin is added to the linear transformation of a linear space. According to the affine transformation method, a displacement is usually represented by the sum of normal linear transformation and definite vector. In more detail, refer to, for example, http://mailsrv.nara-edu.ac.jp/˜asait/open_gl/linear.htm.

When a translation and a rotation are synthesized together and the coordinate of a point is specified in a plane, the following is obtained in the original coordinate system:

$$
\begin{pmatrix} 1 & 0 & a \\ 0 & 1 & b \\ 0 & 0 & 1 \end{pmatrix} \begin{pmatrix} \cos\theta & -\sin\theta & 0 \\ \sin\theta & \cos\theta & 0 \\ 0 & 0 & 1 \end{pmatrix} \begin{pmatrix} x \\ y \\ 1 \end{pmatrix}
$$

The translation, rotation and the like are more easily understood when interpreted as a movement of the coordinate system. In fact, where the unit vector of the original coordinate system is $\{e_1, e_2\}$, the following calculations can be carried out:

$$(e_1, e_2, 0)\begin{pmatrix} 1 & 0 & a \\ 0 & 1 & b \\ 0 & 0 & 1 \end{pmatrix}\begin{pmatrix} \cos\theta & -\sin\theta & 0 \\ \sin\theta & \cos\theta & 0 \\ 0 & 0 & 1 \end{pmatrix}\begin{pmatrix} x \\ y \\ 1 \end{pmatrix}$$

$$= (e_1, e_2, ae_1 + be_2)\begin{pmatrix} \cos\theta & -\sin\theta & 0 \\ \sin\theta & \cos\theta & 0 \\ 0 & 0 & 1 \end{pmatrix}\begin{pmatrix} x \\ y \\ 1 \end{pmatrix}$$

$$= (\cos\theta e_1 + \sin\theta e_2, \quad -\sin\theta e_1 + \cos\theta e_2, \quad a e_1 + b e_2)\begin{pmatrix} x \\ y \\ 1 \end{pmatrix}$$

$$= (\cos\theta e_1 + \sin\theta e_2)x + (-\sin\theta e_1 + \cos\theta e_2)y + (a e_1 + b e_2)$$

The result can be interpreted as follows:

By giving the coordinate system in the affine space by the group of (unit vector on x axis, unit vector on y axis, position of the origin), it is understood that the movement of the point can be interpreted as the movement of the coordinate system based on the above-shown calculation result.

As used herein, the expression "treatment" of a bone refers to physically acting on the bone, and refers to, for example, rotation, excision, cutting, insertion of a graft, distraction and fixation, but is not limited to these.

As used herein, the term "graft" refer to a homogenous or heterologous tissue, cell group or artifact which should be inserted to a specific site of the body (for example, calcium phosphate construct) and becomes a part of the body after insertion. A graft is, for example, a part of a bone (e.g., natural bone (e.g., autobone, homogenous bone, heterologous bone) or a calcium phosphate construct (e.g., hydroxyapatite)), but is not limited to these. Therefore, the term a "graft" encompasses all which is inserted to a defective site of a portion and used to compensate for the defect. Preferably, a graft which does not cause an immunological rejection reaction is used. As a graft, autograft, allograft or heterograft (e.g., coral to human) is used in accordance with the type of donor, but usable grafts are not limited to these.

As used herein, the term "autograft (bone, tissue, cell, organ, etc.)" used regarding a certain individual refers to a graft derived from that individual (bone, tissue, cell, organ, etc.). As used herein, the term "autograft (bone, tissue, cell, organ, etc.)" may encompass a graft (bone, tissue, cell, organ, etc.) from another individual which is genetically the same (e.g., identical twins) in a broad sense. As used herein, the expression "auto" is used interchangeably with "derived from the test subject". Accordingly, in this specification, the expression "not derived from the subject" has the same meaning as that of "non-auto".

As used herein, the term "allograft (bone, tissue, cell, organ, etc.)" refers to a graft (bone, tissue, cell, organ, etc.) implanted from another individual which is homogenous but genetically heterologous. Since being genetically heterologous, an allograft (bone, tissue, cell, organ, etc.) can cause an immunological reaction in an individual to which the allograft is implanted (recipient). Such a graft is, for example, a graft (bone, tissue, cell, organ, etc.) derived from a parent, but is not limited to this.

As used herein, the term "heterograft (bone, tissue, cell, organ, etc.)" refers to a graft (bone, tissue, cell, organ, etc.) implanted from a heterologous individual. Accordingly, when, for example, a human is a recipient, a graft (bone, tissue, cell, organ, etc.) from a pig or a coral component is referred to as the heterograft (bone, tissue, cell, organ, etc.).

As used herein, the term "artificial" graft (bone, tissue, cell, organ, etc.) refer to a graft which is not derived from a natural organism (e.g. , a construct including artificial calcium phosphate). When the artificial graft is an artificial bone, it is preferable that calcium phosphate is contained. More preferably, the calcium phosphate contains hydroxyapatite.

As used herein, the term "recipient" refers to an individual receiving the graft (bone, tissue, cell, organ, etc.) or a graft body (bone, tissue, cell, organ, etc.), and is also referred to as an "implant". An individual providing the graft (bone, tissue, cell, organ, etc.) or the graft body (bone, tissue, cell, organ, etc.) is referred to as a "donor".

As used herein, the term "test subject" is an organism to which treatment of the present method is to be applied, and is also referred to as a "patient". The patient or test subject may preferably be a human.

The graft used with the present invention may be autograft, allograft or heterograft. An autograft is preferable since an immunological rejection reaction may possibly occur with the other types of graft. Where the immunological rejection reaction is not a problem, allograft is usable. Even a graft which causes an immunological rejection reaction can be made usable by performing a process for solving the rejection reaction as necessary. Methods for avoiding the rejection reaction are known in the art, and are described in, for example, Shin Gekagaku Taikei, Vol. 12, "Zoki Ishoku (heart and lung transplantation: from technological and ethical preparations to practice)" (3rd revised edition) (published by Nakayama Shoten). Such methods include, for example, use of immunosuppressant or steroid. Currently available immunosuppressants include cyclosporine (Sandimmun®/Neoral®), Tacrolimus (Prograph), azathioprine (Imuran), steroid hormone (prednine, methylprednine), and T-cell antibodies (OKT3, ATG). A method used in many facilities throughout the world for prophylactic immunosuppression is to use the three agents: cyclosporine, azathioprine and steroid hormone. An immunosuppressant is preferably administered concurrently with the graft, but it is not necessary. Accordingly, the immunosuppressant may be administered before or after the treatment as long as the immunosuppression effect is provided.

As used herein, the term "reinforce" refers to improving the function of an intended part of the organism.

As used herein, the term "cutting" of a bone refers to a treatment process for dividing a bone into two or more portions. Representatively, bone cutting is performed using a bone cutting device (e.g., bone saw). In this specification, a portion of the bone subjected to bone cutting and the portion which has been cut are referred to as a "cutting portion".

As used herein, the term "rotation" of a bone refers to a treatment process of a bone of, after dividing a bone into two or more portions, rotating the portions with respect to each other about a rotation axis. According to the present invention, the rotation is carried out by, for example, arranging parallel a pair of holes on a template assisting member attached to the bone to be treated (e.g., a hole formed in a distal portion and a hole formed in a proximal portion of the template assisting member). The pair of holes define the rotation axis. In this specification, a bone may be rotated at 0 degrees.

As used herein, the term "excision" of a bone refers to removing a part of the bone. Representatively, excision is carried out by, after the bone is cut once, cutting the bone at least once more at a different position. The excision of a bone is not limited to this.

As used herein, the term "distraction" of a bone means to increasing the length of the bone in at least one direction (representatively, in the direction of a longer axis of the bone). The distraction of the bone is representatively performed by a callus distraction method. According to the callus distraction method, the cutting position is gradually stretched using an external fixation device. More specifically, the bone is distracted by stretching the callus generated at the cutting position. An optimum stretching rate is considered to be about 1 mm per day. The bone is distracted to a target length at this rate over several tens of days, and then the bone is kept fixed using the external fixation device, waiting for the callus to be matured into a bone. Skin, muscle, nervous tissue and the like also elongate by being subjected to a tensile strength. Thus, legs or limbs can be distracted by distracting both the bone and the soft tissue. The external fixation device is available in a single post type and a ring type. After the external fixation device is attached during a surgical operation, the position of the frame is moved little by little every day. Thus, the position of the bone can be precisely moved and controlled.

As used herein, the term "fixation" of a bone refers to, after a certain treatment process is performed on a bone, substantially keeping the post-treatment state. Generally, only the bone as a subject of the treatment is fixed.

As used herein, the term "template assisting member" or "template" refer to an assisting member used in the method for treating a bone. The template assisting member, or the template, specifically indicates the cutting section of the bone, rotation axis, and distance of translation. A template assisting member representatively includes a positioning element to be positioned and attached to a prescribed position of the bone, a cutting section indicating element for indicating an appropriate cutting section of the bone, and an attachment position indicating element. The attachment position indicating element shows a position of each of the bone fragments of the bone at which each of the position determination assisting members is attached. The position determination assisting members are respectively attached to the bone fragments of the bone and can allow for determination on whether the bone fragments of the bone are in a normal positional relationship or not, based on the relative positions of the position determination assisting members. Accordingly, the template assisting member encompasses an osteotomy assisting member.

As used herein, the term "proximal" refers to one of two portions or positions of a bone or the like which is closer to the heart.

As used herein, the term "distal" refers to one of two portions or positions of a bone or the like which is farther from the heart.

As used herein, the term "malunion" refers to a post-healing state or shape which is not normal (i.e., deformed) resultant from fracture or other bone disorders.

As used herein, the expression "partner of a pair of bones" refers to, in a pair of bones including a bone which is a subject of treatment, a bone which is different from bone as the subject. Such a pair of bones are, for example, bones of limbs, but not limited to these.

As used herein, the term "standard" refer to a normal range of a certain group. Representatively, standard means a range which is within $\pm 1$ SD (Standard Deviation) from the average where statistics of a certain group are taken. In the present invention, where one of a pair of bones is to be treated, the other bone of the pair may alternatively be used as the standard.

As used herein, the term "kit" refer to a set of members or devices to be used for a certain purpose. A kit includes , for example, various assisting members (e.g., a template assisting member, a position determination assisting member, a translation assisting member, a correction assisting member, a correction position determination assisting member). A kit may include an instruction sheet which describes how to use the members.

In this specification, the "instruction" describes a method of using a device, member, kit or the like or a method of surgical operation performed using the device, member, kit or the like , such that the users such as a physician, medical practitioner, patient or the like understands such a method, member, kit or the like. The instruction sheet is prepared in conformity with the manner defined by the authoritative governmental office of the country in which the present method is carried out (e.g. , Ministry of Health, Labour and Welfare in Japan, Food and Drug Administration (FDA) in the U.S.A.), and explicitly states that an approval by the authoritative governmental office has been obtained. The instruction sheet is a so-called package insert, and is usually provided in the form of a sheet of paper but is not limited to this. The instruction sheet may be provided in the form of, for example, electronic mediums (e.g., an Internet website, an electronic document in the form of PDF, an electronic mail).

As used herein, the term "positioning element" refers to a portion of an assisting member according to the present invention which is to be attached to a prescribed position of the bone. The positioning element can be represented by molding, or labeling the assisting member with a stain or the like.

As used herein, the term "cutting section indicating element" refers to a portion which indicates a cutting section along which the bone is to be cut in a surgical operation performed using an assisting member according to the present invention. The cutting section indicating element can be represented by, for example, forming a slit-shaped opening in the assisting member or labeling the assisting member with a stain or the like.

As used herein, the term "opening" for rotation is used interchangeably with the term "hole". An opening or hole is formed in an assisting member (e.g., a template assisting member) to guarantee that the treatment is performed without fail. The opening or hole has a shape allowing a correction assisting member to be placed therein.

In this specification, an "assisting member" may be formed of any material. For example, the assisting member may be formed of metal (e.g., stainless steel, titanium), plastics, biocompatible polymers, or biodegradable polymers.

In this specification, an "assisting member" may be provided in various forms. For example, the assisting member may be provided as a template assisting member, a correction position determination assisting member, a correction assisting member, an osteotomy assisting member, a translation assisting member, or the like, but is not limited to these although it is limited to an osteotomy assisting member in embodiments of the invention.

As used herein, the term "correction position determination assisting member" refers to an optional assisting member used for determining a position to be corrected.

As used herein, the term "correction assisting member" refers to an assisting member used for correction treatment. The correction assisting member is usually used with a template assisting member, but is not limited to this.

As used herein, the term "translation assisting member" refers to an assisting member for assisting translation.

As used herein, the term "biocompatible" refers to a property by which a substance can exist in an organism without causing any disorder owing to lack of toxicity or immunological rejection.

As used herein, the term "biocompatible polymer" refers to a polymer having good biocompatibility. Specifically, a biocompatible polymer does not cause toxicity if remaining in an organism. In this specification, whether a polymer is biocompatible or not is determined by use of a test, for example, an implantation test of implanting the polymer subcutaneously in laboratory animals such as rats, rabbits, dogs or the like. When a relatively acute immunoreaction or allergic reaction or the like occurs to cause a swell, flare or fever as a result of implantation, it is visually appreciated that the polymer is low in biocompatibility. When a biocompatible polymer is implanted into a specific affected site, for example, an animal blood vessel, the site of implantation is observed several days or several months later. What are observed are whether there is a take of tissue, and the degree of inflammation, adhesion, thrombosis formation, and the like caused by blood coagulation in the vicinity of the implanted biocompatible polymer. Thus, biocompatibility is determined.

Another method of determining tissue compatibility is performed as follows: A tissue piece of the implanted site is created and the cells are stained by hematoxylin and eosin stain or other stain techniques and observed. It is determined whether or not many granular cells in charge of the immune system have invaded, or whether or not a cicatrix tissue has been formed between a tissue existent before the implantation and the implanted biocompatible polymer so as to separate the two types of tissues. When many granular cells have invaded, or the cicatrix tissue has been formed, the biocompatibility is low.

As used herein, the term "biodegradable" refers to aproperty of a substance, by which the substance is degradable in an organism or by the action of microorganisms. A biodegradable polymer may be degraded, for example, into water, carbon dioxide, methane and the like by hydrolysis. In this specification, whether a substance is biodegradable or not is determined as follows. Bioabsorbability, which is a part of biodegradability, of the substance is determined by performing an implantation test using rats, rabbits, dogs or other laboratory animals for several days to several years. Degradability by microorganisms of the substance is determined by, for example, performing a placing and destruction of a sheet-type polymer in the soil for several days to several years.

In this specification, a "wire" may be formed of any material, for example, metal (e.g., stainless steel, titanium), plastics or the like,

In this specification, "means for obtaining a model" may be any means which can obtain a three-dimensional model of a subject. The means for obtaining a model is, for example, a CCD camera, an optical camera, x-rays, CT, or MRI (magnetic resonance imaging), but is not limited to these.

In this specification, "means for obtaining a bone model" may be any means which can obtain a three-dimensional model of a subject. The means for obtaining a bone model is, for example, software of, for example, computer graphics, but is not limited to this.

As used herein, the expression "determine an assisting member necessary for treatment process to be performed on a bone" refers to determining an assistingmember necessary to perform the treatment process which is to be determined according to the present invention. For this determination, any means which can perform mathematical processing of a three-dimensional model is usable.

As used herein, the term "means for performing a surgical operation" may be any means usually used by those skilled in the art for surgical operations of bones. The operating means is, for example, a bone chisel (and a hammer (e.g., wooden hammer or metal hammer)), an electric or air motion bone saw, but is not limited to these.

Hereinafter, preferred embodiments of the present invention will be described. The following embodiments are provided for a better understanding of the present invention and the scope of the present invention should not be limited to the following description. It will be clearly appreciated by those skilled in the art that variations and modifications can be made without departing from the scope of the present invention with reference to the specification.

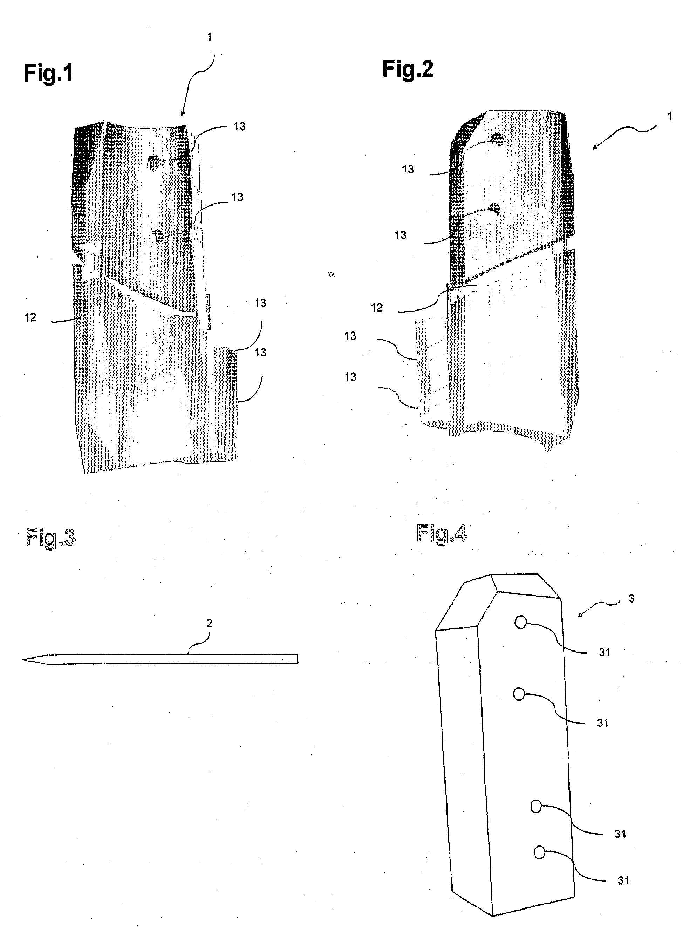

Figures **1** through **3** show an osteotomy assisting member 1 as a template assisting member and a rod **2** as a correction position determination assisting member **2** according to the present invention. The osteotomy assisting member **1** and the rod 2 are used as a pair for osteotomy performed for the purpose of correcting a malunited bone.

In more detail, as shown in Figures **1** and **2,** the osteotomy assisting member **1,** which is formed of a resin block, is produced by rapid prototyping such as photo printout based on three-dimensional model data obtained or created on a computer. The osteotomy assisting member **1** includes a fitting surface **11** (not shown in Figures **1** through **3)** as a positioning element for indicating a position of the osteotomy assisting member **1** which is to be attached to the bone; a slit **12** as a cutting section indicating element for indicating a cutting section along which the bone is to be cut and divided; and guide holes **13** each as an attachment position indicating element for indicating an attachment position of the rods **2.**

The fitting surface **11** is formed to fit a surface feature portion of the bone.

The slit **12** is provided so as to correspond to the cutting section of the bone. The cutting section is defined so as to be such a position that a post-correction bone shape and the shape of a normal bone are closest to each other. The post-correction bone shape is obtained by dividing the bone along the cutting section into a proximal portion and a distal portion and moving and/or rotating the distal portion.

As described above, the guide holes **13** are provided for indicating the attachment positions of the rods **2.** The thickness of the osteotomy assisting member **1** is set such that each guide hole **13** has a sufficient length to have a rod positioning function.

As shown in Figure **3,** each rod **2** is formed of metal and is flexible to some extent. The rod **2** is formed to have, for example, a pointed end so as to pierce a bone. The rod **2** has a diameter which is slightly smaller (for example, 0.1 to 0.2 mm) than that of the guide holes **13.** The rod **2** can be inserted into each guide hole **13** and pierce the bone, so as to be attached to the bone at a desired position in a desired direction while making a hole in the bone. In this example, it is determined that two bone fragments are in a normal positional relationship in the following state: the two bone fragments each having two rods **2** piercing therein are moved until the rods **2** in the two bone fragments are in a prescribed positional relationship (for example, parallel to each other). More specifically, it is determined that the two bone fragments are in a normal positional relationship in the following state: where a block **3** (Figure **4)** is provided as an intermediate member having insertion holes **31** as engaging elements, each of which is capable of receiving the rod **2,** the rods **2** are insertable into each insertion hole **31.**

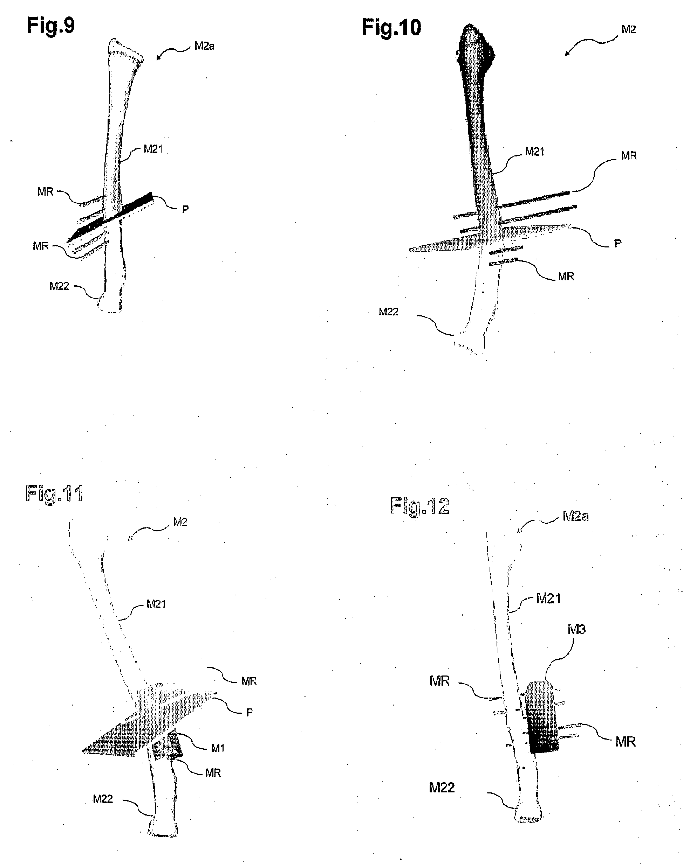

Hereinafter, a method for producing the osteotomy assisting member **1** will be described. The osteotomy assisting member **1** is produced by rapid prototyping such as photo printout based on three-dimensional model data **M1** (Figure **11)** obtained or created on a computer as described below.

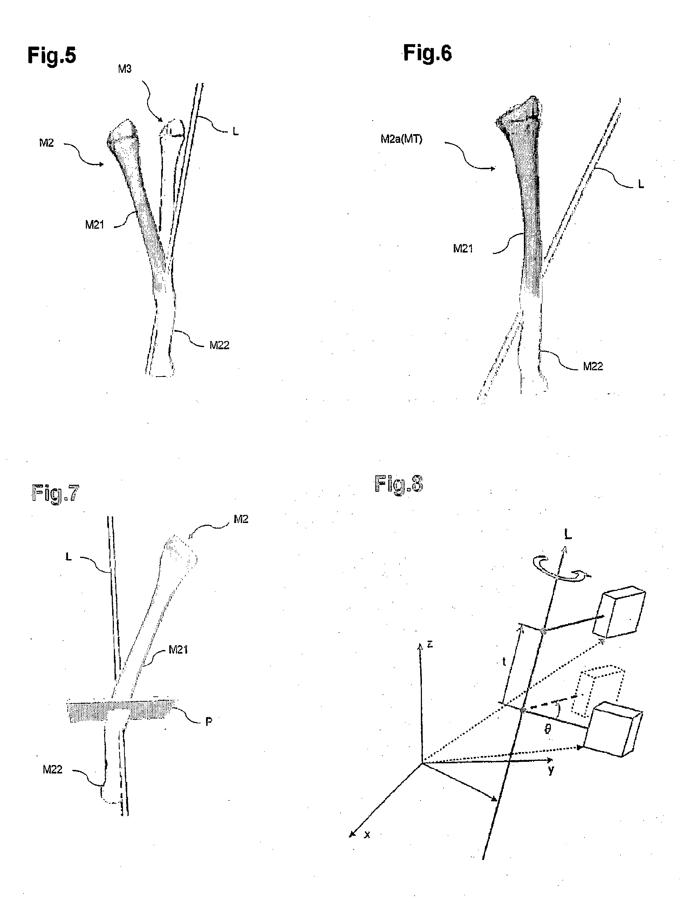

First, as shown in Figure **5,** a three-dimensional surface bone model **M2** is created by a computer based on data obtained by, for example, CT or MRI. A three-dimensional surface bone model **MT** of a normal bone is also created. The proximal portions of the bone models **M2** and **MT** are overlapped. Since this example uses arm bones, the normal bone model **MT** is produced using a mirror image of a bone model of a normal arm. Alternatively, the normal bone model **MT** may be produced using past data of the target bone when it was normal or using various other techniques.

Then, a cutting section **P** (Figure 7) necessary for correction and a moving amount of a distal bone fragment model **M21** obtained by dividing the bone model **M2** into two portions are set. Thus, as shown in Figure **6,** a post-correction bone model **M2a** is determined. Briefly, the cutting section **P** and the moving amount of the distal bone fragment model **M21** are set by arithmetic operations such that the difference in shape between the post-correction bone model **M2a** and the normal bone model **MT** is smallest.

This will be specifically described. In this example, a surface matching method referred to as an "ICP (Iterative Closest Point) algorithm" is used. This is performed as follows. As shown in Figure 7, the bone model **M2** is divided along a surface perpendicular to a virtual screw axis **L** appropriately set into the distal bone fragment model **M21** and the proximal bone fragment model **M22**. The distal bone fragment model **M21** is translated linearly parallel to the screw axis line L and/or rotated about the screw axis line L. A difference between the post-correction bone model **M2a** after the translation/rotation and the normal bone model **MT** is converted into a numerical value by, for example, squaring the data on the corresponding position on the surface of the model. The distal bone fragment model **M21** is translated and/or rotated such that the above-obtained numerical value is minimal.

For defining the screw axis **L,** the Screw Displacement-Axis method, which is one method for representing movement of an object, is used. The principle of the Screw Displacement-Axis method will be described with reference to Figure **8.** There is a unique axis, for one movement of an object in space, for which the following is true: When an object is rotated about the unique axis by an angle $\phi$ and moved parallel to the axis by distance t, the object can be moved to any position. For correcting a deformed bone, the bone is cut at a position into two portions, and the portions are moved with respect to each other into a normal positional relationship. The Screw Displacement-Axis for the movement is obtained. By respectively matching characteristic shapes of the proximal portion and the distal portion of a deformed bone with those of a normal bone, it is found how much the deformed portion (proximal or distal) needs to be moved to realize correction. Thus, the Screw Displacement-Axis for moving the deformed portion by the found amount is obtained by calculation. In many of the clinical uses, the translation component is sufficiently small to be negligible (e.g., within 1 mm), and therefore only a rotation component needs to be considered. Thus, it can be considered that the Screw Displacement-Axis = screw axis.

When the screw axis L is parallel to, or substantially parallel to, the longer axis of the bone as shown in Figure 5, the bone is cut along a plane vertical to the screw axis L at position where the screw axis **L** is closest to the center of the bone, and the distal bone fragment is rotated about the screw axis **L.** Thus, the bone can be corrected with the contact area of the bone fragments being maximal. Thus, this cutting section can be set as an optimum cutting section.

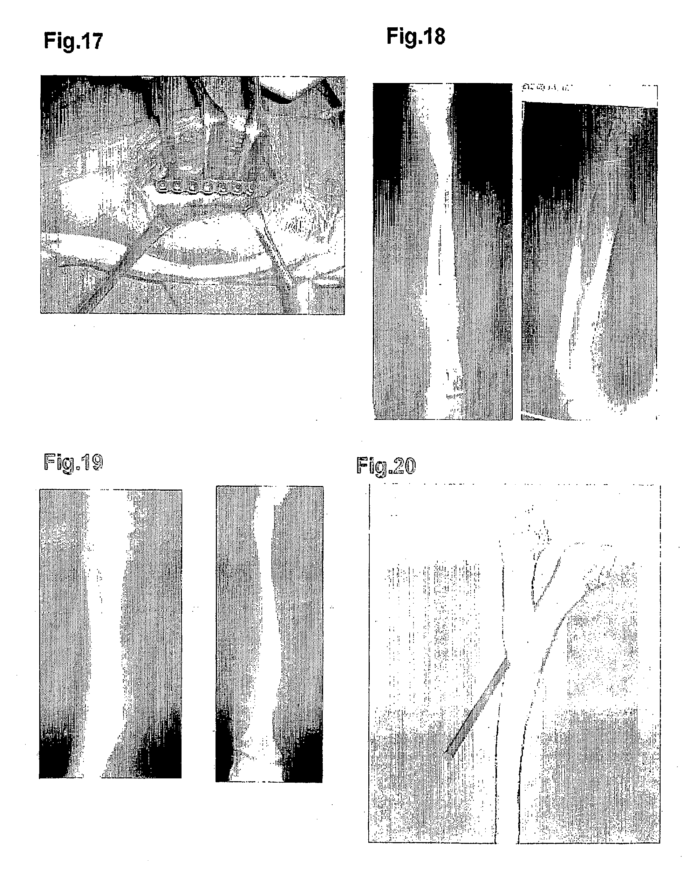

The screw axis **L** varies in accordance with the form of the bone. For example, as shown in Figure **20,** the simulation results in the bone fragments being rotated about the screw axis **L** substantially vertical to the longer axis of the bone. In this case, a defect is created in the bone, and the bone correction is completed by filling in this defect by bone implantation or insertion of a bone filing material.

There are cases where the bone needs to be partially excised, but in many cases, correction simulation using the Screw Displacement-Axis method (= screw axis **L**) is useful.

The above-described simulation is not always used. When, for example, the screw axis **L** passes through a joint, correction osteotomy is not performed unless the joint is cut. Therefore, the above-described method is not usable. In this case, the bone is cut at a convenient position to the operator for simulation.

As shown in Figure **9,** rod models **MR** are respectively attached to each of the bone fragment models **M21** and **M22** of the post-correction bone model **M2a** such that the rod models MR are parallel to each other. As shown in Figure 10, the attachment positions of the rod models **MR** on the bone fragment models **M21** and M22 of the bone model **M2** are obtained by calculation.

As shown in Figure **11,** the three-dimensional model **M1** of the osteotomy assisting member **1** having the fitting surface which can fit the surface feature portion of the bone model is created so as to include the cutting section P determined as described above. At this point, a slit is formed in the model **M1** at a position corresponding to the cutting section **P,** and guide holes are formed in the model **M1** at positions corresponding to the rod models MR.

In this manner, the three-dimensional model **M1** of the osteotomy assistingmember 1 is created having the fitting surface, the slit and guide holes. Based on the data of the model **M1,** the actual osteotomy assisting member 1 is produced.

As shown in Figure **12,** while the rod models **MR** are attached to the bone fragment models **M21** and **M22** of the post-correction bone model **M2a,** a three-dimensional block model **M3** of the block **3** having insertion holes through which the rod models **MR** can pass is created on the computer. Similarly to the osteotomy assisting member 1, the actual block **3** is produced based on the three-dimensional block model **M3** by rapid prototyping such as photo printout for the like.

A method for performing a surgical operation using the osteotomy assisting member **1,** the block **3** and the like thus produced will be described.

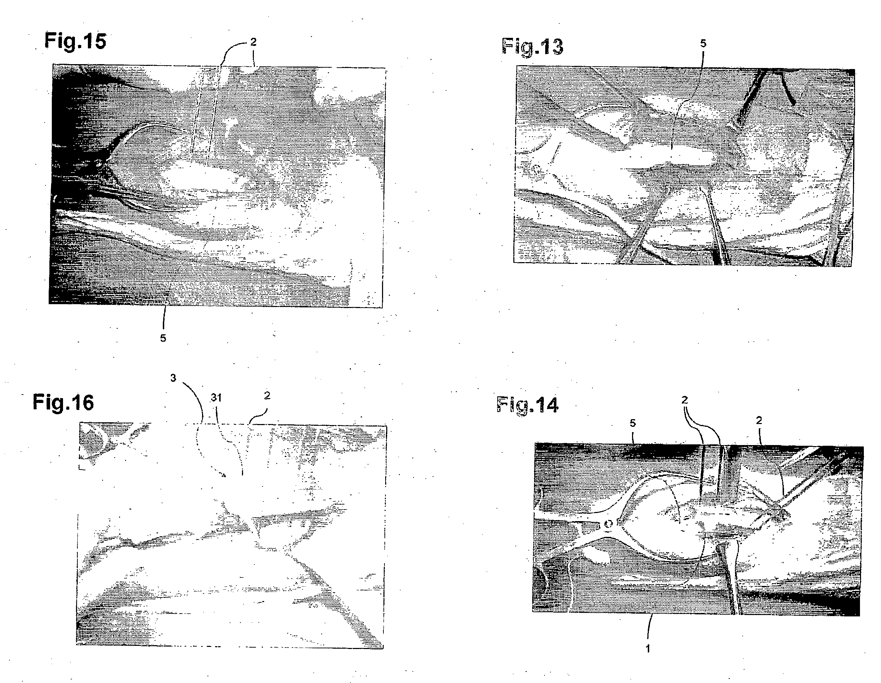

As shown in Figure **13,** an affected site is incised, so as to expose a necessary portion of a bone **5.**

Then, as shown in Figure **14,** the fitting surface of the osteotomy assisting member **1** and the surface feature portion of the bone **5** are made to fit each other, so as to fix the osteotomy assisting member **1** in a closely fit manner. Thus, the osteotomy assisting member **1** is uniquely positioned and attached to the bone **5.** The rods **2** are inserted into the guide holes **13,** and the bone **5** is pierced with the end of each rod **2** so as to attach the rods **2** to the bone **5.**

Next, the bone **5** is cut by moving a cutting jig such as a saw or the like along the slit **12.** Then, as shown in Figure **15,** the osteotomy assisting member **1** is removed while leaving the rods **2** in place.

As shown in Figure **16,** the distal bone fragment and the accompanying tissues are moved to a position at which the other end of each rod **2** is insertable into the respective insertion hole **31** of the block **3 ,** and then the rod **2** is inserted thereinto. This step puts the bone fragments into a normal positional relationship.

Finally, as shown in Figure **17,** the bone fragments are joined to each other in this state using plate and screws or other fixing devices.

In this type of osteotomy surgical operation for bone correction, the osteotomy assisting member **1** and the rod 2 in this example having the above-described structure allow the bone to be easily cut along an appropriate cutting section which is pre-calculated by computer simulation and also allow the post-cut bone fragments to be easily moved to an appropriate position which is also pre-calculated by computer simulation. Accordingly, accurate and easy osteotomy surgical operations for bone correction are realized without relying on the physician's experience or skill. Specifically, the pre-correction bone shown in the x-rays in Figure 19 was superbly corrected to the bone shown in Figure **18.**

The present invention is not limited to the above-described example.

For example, one rod may be inserted into each bone fragment. The correction position determination assisting member is not limited to the rod but may be some other member. For example, a member for fixing the bone while holding the bone between two portions thereof may be more preferable depending on the site of operation.

A plurality of slits may be formed instead of one in the case where the bone needs to be divided into three or more fragments needs to be partially excised.

The assisting members according to the present invention are not limited to those shown in the above-mentioned figures but may be modified in various manners as long as the members can act as intended.

Alternatively, the following procedure may be used.

(i) A proximal portion of a deformed bone is matched to a proximal portion of a normal bone, and the position information thereof (matrix in the sense of affine transformation) is obtained.

(ii) Next, distal portions of the deformed bone and the normal bone, and the matrix thereof is obtained. For the matching, an ICP algorithm may be used (it is not necessary to use the ICP algorithm).

(iii) A difference between the matrices of the proximal portions and the distal portions is obtained. This is called by the present inventor as "relative matrix".

(iv) The relative matrix is transformed into the Screw Displacement-Axis method. Thus, the Screw Displacement-Axis (rotation axis) and the rotation amount about the Screw Displacement-Axis (and the translation amount along the Screw Displacement-Axis) are found.

After the Screw Displacement-Axis is found, it is determined whether rotational osteotomy or closed/open wedge osteotomy is to be used in accordance with whether the Screw Displacement-Axis is parallel or vertical to the longer axis of the bone. Thus, the cutting section of the bone is set. One example of this procedure will be described in Example 12.

(Surgical operation method)

Hereinafter, a specific operation method will be described in detail.

The present disclosure, provides a method for treating a bone (e.g., performing a surgical operation on the bone). The method includes the steps of: (A) obtaining a bone model representing a bone which is a subject of treatment; (B) obtaining a target bone model to which treatment aims; (C) determining a treatment process which is to be performed on the bone (e.g., determining an assisting member necessary for the treatment process to be performed on the bone) based on the bone model and the target bone model; and (D) performing a surgical operation using the determined treatment process (e.g., using the assisting member). Conventionally, a malunited bone or the like was gradually corrected by performing surgical operations within a possible range with trial and error. By contrast, it has been unexpectedly found that the present method allows a bone to be healed to a substantially normal state by performing, on principle, one treatments process (for example, cutting, insertion or rotation).

The step of obtaining a bone model representing a bone which is a subject of treatment is performed by using any imaging device, and a data storage and/or processing device usable for the data obtained by the imaging device. Examples of the imaging device include optical imaging devices (e.g., cameras), and CT and MRI imaging devices, but are not limited to these. Examples of the data storage and/or processing device include computers in which software for extracting CT or MRI data on the bone and obtaining or creating a surface model is installed, but are not limited to these. A surface model is representatively stored in the format of VTK or STL, but may be stored in other formats. Examples of software for creating such a surface model include Virtual Place M (Medical School and marketed by Medical Imaging Laboratory, Ltd., Japan), Mimics (Materialise, Belgium), Zview™ (Zview, Inc., Huntington Beach, CA, USA), Realize (Mayo Clinic), but not limited to these.

The step of obtaining a target bone model to which treatment aims can be performed by using any image creating device. The image may be manually created. Usually, the image is created by introducing an image of the subject of the model (e.g. , where one of the arms or legs is to be treated, the other arm or leg) or by using, for example, a model of a normal bone created by a computer (using CAD of other types of software) or stored by the computer.

The step of determining a treatment process which is to be performed on the bone based on the bone model and the target bone model usually includes the step of determining an assisting member necessary for the treatment which is to be performed on the bone. This step can be performed by using any device capable of processing a model. Examples of such a device include computers having image processing software installed therein, but are not limited to these. Such software may have functions of, for example, performing surface matching, deriving movement information, or creating a template. In this example, a program for controlling and managing position information regarding the surface model on a computer (easily created using, for example, an open source referred to as "VTK" (http://public.kitware.com/VTK/)) is usable when necessary.

Surface matching may be performed using, for example, an algorithm suitable to matching such as an ICP (Iterative Closest Point) algorithm, but other techniques may also be used. In surface matching, accurately matching two models (the target bone model and the bone model) is attempted. When a program for controlling and managing position information regarding the surface model on a computer is used, the two models are first manually matched to each other to some extent when necessary, and are then matched using the algorithm as mentioned above. When the bone is deformed, the two models cannot, on principle, match perfectly. Therefore, it is acceptable to ignore the portions which are distanced from each other by an arbitrary threshold (e.g., 1 mm) or more and match the portions which are within the distance effectively within the threshold. Since there are often excessive errors, the two model may be put closer to each other manually and visually, rather than relying on the algorithm.

The movement information can be found through calculation by deriving relative movement information from information on two positions (the positions of the target bone model and the bone model). First, a proximal bone fragment and a distal bone fragment of the bone to be treated are matched to those of the target bone model (e.g., a mirror image of the normal arm or leg). Then, information on a relative deformity can be obtained from the information on the two positions. Here, the Screw Displacement-Axis (rotation axis) and how much the distal bone fragment (or the proximal bone fragment) needs to be rotated about the rotation axis and translated along the rotation axis can be calculated using a Screw Displacement-Axis method. Instead of using the Screw Displacement-Axis method, the movement information on any coordinate axis can be represented using an affine transformation method.

A template model can usually be created using a Boolean operation. The created model can be stored in the STL format (used in photo printout for the like; the model is represented as a surface model filled with triangles), but may be stored in other formats. The Boolean operation is one of the 3D graphic modeling techniques, and performs modeling by an operation referred to as set operation. With the Boolean operation, modeling can be performed by arithmetic operation of: forming a plurality of overlapped models into one mass(sum), deleting the overlapped models (difference), and retrieving only the overlapped portions (logical product). The Boolean operation is adopted in, for example, form·Z (available from autodessys). For creation of the template, form·Z or Magics RP (Materialise) is usable, but other types of software is also usable. Such software is developed for the industrial uses, and is not conventionally used for treating deformed bones.

In a preferred method for treating a bone according to the present invention, the step of determining a treatment process (usually, the step of determining an assisting member necessary for the treatment process to be performed on the bone) directly or indirectly uses parameters in all three-dimensional directions of the bone. In the prior art, the two-dimensional directions are usually considered, and there is no example of considering the three-dimensional directions. The present method, which considers the parameters in the three-dimensional directions, is highly useful in achieving more accurate surgical operations and more satisfactory postoperative progresses.

In a preferred method for treating a bone, the step of determining an assisting member necessary for the treatment process to be performed on the bone comprises the step of determining a rotation axis of the bone.

Preferably in the case where the bone to be treated is deformed, the rotation axis of the bone is found as follows. The bone model is divided into a distal portion and a proximal portion. Then, it is determined by calculation where either the distal portion or the proximal portion overlaps with the target bone model for comparison, whether or not the target bone model is created by rotating the other portion (i.e., by rotating the proximal portion when the distal portion overlaps with the target bone model, and by rotating the distal portion when the proximal portion overlaps with the target bone model). The rotation axis can be found by using, for example, the Screw Displacement-Axis method. Actual calculation may be performed manually, but is usually performed using a computer having a program for executing the Screw Displacement-Axis method installed therein.

Accordingly, in a preferred method for treating a bone, the rotation axis is determined using the Screw Displacement-Axis method. The Screw Displacement-Axis method is described in, for example, S. Ohwovoriole and B. Roth: "An extension of screw theory", Journal of Mechanical Design, Vol. 103, pp. 725-735, Oct. 1981. Using the Screw Displacement-Axis method, bone correction can be realized substantially by cutting the bone once and performing at least one selected from the group consisting of bone rotation, bone excision, bone distraction, and insertion of a graft. It has been unexpectedly demonstrated that the postoperative progress was favorable even several months, or one year or more after the operation. As can be appreciated from this, the present disclosure provides a simple and efficient surgical operation method for bone correction which was unexpected from the prior art.

Accordingly, a preferred method for treating a bone includes at least one selected from the group consisting of bone rotation, bone excision, bone distraction, and insertion of a graft. Which one or ones of the bone rotation, bone excision, bone distraction, and insertion of a graft should be performed can be easily determined by those skilled in the art by the degree of deformity (i.e., a difference between the target bone model and the bone model). The difference can be found by calculation using any mathematical technique well known in the art. Each of bone rotation, bone excision, bone distraction, and insertion of a graft is preferably performed once. In the prior art, there is no example in which it is necessary to perform each of the rotation, excision, insertion, implantation and the like only once. Thus, the present method has realized the simplicity, which was conventionally impossible.

In a preferred method for treating a bone, a template assisting member is used. In a conventional treatment of an abnormal bone, such as a deformed bone, the bone is cut, at an approximately proper position determined by visual inspection, into bone fragments and then the bone fragments were joined in an approximately proper manner determined by visual inspection. Use of a template assisting member realizes simple and accurate correction. The template assisting member can be produced by creating a model by, for example, a computer-assisted image creation method described below in this specification and then molding a material (e.g., metal, plastics, ceramics) by any molding method such as photo printout.

Optical formation is a technique for producing a precise object substantially the same as that of 3D data in a short time. By photo printout, a liquid ultraviolet curable resin (a liquid which is cured by reacting to ultraviolet rays) is cured by ultraviolet laser light from an photo printout apparatus and laminating the cured layers.

Specifically, data of a three-dimensional model designed by, for example, CAD is output in the STL format, and the three-dimensional model is sliced at a pitch of 0.1 to 0.2 mm. Thus, contour data for photo printout is obtained. Based on the contour data, the ultraviolet laser light from a semiconductor laser device draws a shape of a cross-section on a surface of a liquid resin in a tank. The portion irradiated with the laser light is chemically reacted and is cured. When one layer is formed, the substrate bearing the formed laminate sinks by the slicing pitch, and a shape of the next cross-section is drawn and formed into a layer by the laser scanning, and laminated on the first layer. By laminating such thin cross-sections sequentially in this manner, a three-dimensional model is formed below the liquid surface. Then, the three-dimensional model is washed or subjected to other processing, and the template assisting member is completed. The service of producing such template assisting members is available from many various providers including PLAMOS of the Daicel Group, Kida Industry Co., Ltd., and CMET Co., Ltd.

The template assisting member used in an embodiment of the present invention preferably includes at least one element selected from the group consisting of a positioning element for indicating a position at which the template assisting member is to be located; a cutting section indicating element for indicating a cutting section along which the bone is to be cut; and an attachment position indicating element for indicating a position at which a correction position determination assisting member is to be attached. More preferably, the template assisting member includes at least the cutting section indicating element. Owing to the cutting section indicating element, the cutting position of the bone can be easily identified so that the bone can be cut accurately. Still more preferably, the template assisting member includes the attachment position indicating element. Owing to the attachment position indicating element, the template assisting member can be more smoothly and more accurately moved after the bone is cut. The positioning element can be easily realized by molding, or labeling the template assisting member with a label (e.g., a stain), but may be realized by other methods. The cutting section indicating element can also be easily realized by molding, or labeling the template assisting member with a label (e.g., a stain), but may be realized by other methods. Advantageously, the cutting section indicating element is realized by physically forming a slit, since the slit allows the cutting device such as a bone saw to be operated more easily. The attachment position indicating element can also be easily realized by molding, or labeling the template assisting member with a label (e.g., a stain), but may be realized by other methods. The attachment position indicating element is preferably an opening, since the correction position determination assisting member is preferably a wire.

More preferably, the template assisting member used in an embodiment of the present invention includes all of the positioning element, the cutting section indicating element (preferably, a slit), and the attachment position indicating element (preferably, an opening or a hole). When including all of these elements, a single template assisting member can perform positioning of the template assisting member and bone cutting.

In a preferred method for treating a bone according to the present invention, the step of performing a surgical operation includes the steps of: (A) cutting the bone at at least one position into bone fragments; (B) performing at least one selected from the group consisting of: (i) performing the bone rotation when necessary, (ii) performing the insertion of a graft when necessary, and (iii) performing the bone excision when necessary; and (C) joining the bone fragments.

The step of cutting the bone can be performed using any cutting device. Preferably, an assisting member (e.g., a template assisting member of the present invention) is used. The bone may be cut an unlimited number of times, but preferably once. The present method realizes correction of an abnormal bone by cutting the bone only once and performing the subsequent treatment processes.

The step of performing at least one treatment process can be performed using any device and the hands of a physician or other medical practitioners. Preferably, an assisting member (e.g., a template assisting member of the present invention) is used. Each of the treatment processes (i.e. , bone rotation, insertion of a graft, and bone) maybe performed an unlimited number of times, but preferably once. The present method realizes correction of an abnormal bone by performing each of at least one of the treatment processes only once. Thus, the present invention realizes simple and accurate bone correction. A graft to be inserted can be designed by a computer. An actual graft is produced based on the computer-designed graft model by molding a material (e.g., metal, plastics, ceramics) using any molding method such as 3D cutting, NC machining or the like. The cutting can be performed using a commercially available cutting tool such as, for example, the 3D cutting tool by Roland DG (e.g. , MDX series including MDX-20, PNC-3200).

In a preferred method for treating a bone, the treatment process includes bone rotation. Advantageously, the bone is rotated about one rotation axis. There is no conventional technique for healing an abnormal bone by rotating the bone about a rotation axis. It was not conventionally expected that a deformed bone can be corrected by using rotation technique in a surgical operation. The present method which demonstrates that simple and accurate correction can be realized by rotation, is useful for various types of bone correction.

The step of joining the cut bone fragments can be performed using any device well known in the art. The bone fragments can be joined using, for example, an internal fixation device such as a plate, screw, wire, intramedullary nail or the like. A defect of the bone may be filled in by calcium phosphate (e.g., hydroxyapatite, βTCP, calcium phosphate paste) or other biocompatible materials. Alternatively, the defect of the bone may be filled in by bone implantation.

After the bone fragments are joined, it is preferable to maintain the state for a long time. In this manner, the corrected state is retained and the effect of correction is guaranteed. When appropriate, the bone may be distracted rather than joined. Such distraction may be performed using a callus distraction method.

In a preferred method for treating a bone, the bone rotation, the insertion of a graft and the bone excision are defined by the Screw Displacement-Axis method or an affine transformation method. Surprisingly, it has been demonstrated that bone correction is realized by simply performing a surgical operation with a difference calculated by such a mathematical technique being reflected on the bone rotation, insertion of a graft, bone excision, bone distraction of the like, and that the effect of correction is maintained for one year or longer for the first time in history. In order to closely follow the difference calculated by the above mathematical technique, it is preferable to use a template assisting member. Using the template assisting member, the operator can simply follow the instruction line on the template assisting member to perform the cutting, rotation, or other processes. Such a method of surgical operation brings an epoch-making change in the field of surgery, in which many of the operation procedures conventionally needed to be conducted manually relying on only the experience of the operator.

In one method for treating a bone , the bone excision is defined by a template assisting member, but the present invention is not limited to this. The template assisting member may be attached after the bone is cut.

In one method for treating a bone , the step of performing the bone rotation representatively includes the step of arranging correction assisting members passing through at least a pair of openings which define a deformity amount, the pair of openings being on the template assisting member. In this case, the template assisting member is designed such that a target rotation is achieved by arranging the correction assisting members in parallel. Such a design can be realized by, for example, the Screw Displacement-Axis method or the affine transformation method.

The graft to be inserted is preferably defined and produced by the Screw Displacement-Axis method or the affine transformation method. By such methods, a difference between the bone model and the target bone model can be obtained and a model of the graft can be created from the difference. Based on the created model, an actual graft can be produced by, for example, 3D shaving.

In a preferred method for treating bone, the step of cutting the bone includes the step of cutting the bone at one position into a proximal bone fragment and a distal bone fragment, and the step of performing a surgical operation includes fixing either the proximal bone fragment or the distal bone fragment of the bone. The target bone model is preferably created based on a proximal portion and a distal portion of the bone, since one of the bone fragments usually has a normal shape.

The bone to be treated using the present invention is usually a bone of a limb, but is not limited to this. Any bone can be treated . These bones include, for example, long bones (for example, bones of limbs), short bones, flat bones (e.g., sterna, costae, scapulae, ilia), sesamoid bones (e.g., patellae), and irregular bones (viscerocranium, vertebrae), but also include other types of bones. For the present invention, correction of long bones (for example, bones of limbs) is suitable. A subject of correction using the present invention is representatively a malunited bone.

In one method for treating a bone , a graft to be inserted is produced using a natural bone or an artificial bone. The natural bone may be, for example, autobone, homogenous bone, or heterologous bone, but is not limited to these. Autobone is preferable. As the autobone, ilia or the like is usable. When autobone is not easily available, homogenous bone is used. The homogenous bone is available from, for example, the bone bank.

The artificial bone may be formed of any biocompatible material, but preferably of a material compatible to bone, and more preferably of a material which can be a part of the bone. Alternatively, the artificial bone may be formed of a biodegradable material. As a biodegradable material, a material which is gradually degraded (e.g., over several months) is usable, not a material which is rapidly degraded. Preferable materials include, for example, bone-affinitive materials such as bone ceramics, and calcium phosphate, and hydroxyapatite. Hydroxyapatite is especially preferable. Such artificial bone may be, for example, NEOBONE (MMT Co. , LTD., Osaka), but is not limited to this.

A preferred method for treating a bone includes the step of checking whether treatment was performed properly or not after the step of performing a surgical operation. Such a checking can be performed using any device or technique well known in the art; for example, by using a correction position determination assisting member of the present invention. Alternatively, the checking can be performed by taking an x-ray of the post-correction bone, obtaining a model of the bone, and comparing the target bone model and the model of the post-correction bone to determine the difference. Such a comparison can be performed using any program known in the art.

The target bone model used in the present invention is defined based on a partner of a pair of bones including the bone represented by the bone model. Such a pair of bones are, for example, bones of limbs, but not limited to these.

Alternatively, the target bone model used in the present invention is defined based on a standard of patients having the bone to be treated. The standard can be found by investigating a set of patients, processing the results statistically, and setting a range of the average $\pm$ 1 SD as the standard.

(Simulation)

Also disclosed is a method for simulating bone treatment (e.g., surgical operation). The method includes the steps of: (A) obtaining a bone model representing a bone which is a subject of treatment; (B) obtaining a target bone model to which the treatment aims; (C) determining a treatment process which is to be performed on the bone (e.g., determining an assisting member necessary for the treatment process to be performed on the bone) based on the bone model and the target bone model; and (D) creating a production model based on the bone model and performing a simulation of the bone treatment based on the determined treatment process (e.g., using the assisting member). Conventionally, a malunited bone or the like was gradually corrected by performing surgical operations within a possible range with trial and error. By contrast, the present method realizes a simulation of the treatment process to be performed. Thus, even a beginner can easily perform the treatment processes and become familiar with the technique. Any of the steps included in the method for simulating bone treatment can be performed using any of the devices or techniques described in the "Surgical operation method" section above.

For simulation, it is possible to actually produce a production model (of, for example, hydroxyapatite) and an assisting member when necessary, and perform a simulation of the surgical operation in accordance with the determined treatment process.

(Treatment kit)

Also disclosed is a bone treatment kit used for treating a bone. The kit includes: (A) a template assisting member including a positioning element for indicating a position of the template assisting member which is to be attached to the bone, a cutting section indicating element for indicating a cutting section along which the bone is to be cut, and at least one opening through which the correction assisting member for rotation is to be inserted; and (B) a correction position determination assisting member. The template assisting member used here may be in any form or material which is described above in detail in this specification. The correction position determination assisting member may also be in any form described above.

Preferably, the template assisting member includes at least two openings. With two openings, it is easily determined whether or not the correction position determination assisting members are parallel to each other. More preferably, the template assistingmember includes three openings, and still more preferably four openings. With four openings, it is more easily determined by visual inspection whether or not the correction position determination assisting members are parallel to each other. The correction position determination assisting member is preferably a wire having a translation assisting function, but is not limited to this as long as the member has a translation assisting function. The wire may be formed of any material, preferably stainless steel.

In a preferred bone treatment kit , the correction position determination assisting member has at least one function selected from the group consisting of a translation assisting function for translation and a rotation assisting function for rotation. Alternatively, the bone treatment kit may include a plurality of correction position determination assisting members, each of which has both the translation assisting function and the rotation assisting function.

It is advantageous that the bone treatment kit has both the translation assisting function and the rotation assisting function. The translation assisting function can be realized by marking a member extending in a direction of a longer axis of the bone, for example, a wire. The rotation assisting function may be realized using a linearly extending member, for example, a wire.

Preferably, the bone treatment kit further includes a fixation assisting member for fixing the bone after a surgical operation. Such a fixation assisting member may be any member. For example, a conventionally used device such as an external fixation device or the like may be used.

(Simulation kit or system)

Also disclosed is a kit or system for simulating bone treatment (e.g., surgical operation). The kit includes (A) means for obtaining a bone model representing a bone which is a subject of treatment; (B) means for obtaining a target bone model to which the treatment aims; (C) means for determining a treatment process which is to be performed on the bone (e. g. , determining an assisting member necessary for the treatment process to be performed on the bone) based on the bone model and the target bone model; and (D) means for performing a simulation of the bone treatment based on the determined treatment process (e. g. , using the assisting member). The kit may include a production model of the bone, an assisting member when necessary, and an instruction sheet which indicates the determined treatment process. Conventionally, a malunited bone or the like was gradually corrected by performing surgical operations within a possible range with trial and error. By contrast, the present kit or sytem realizes a simulation of the treatment process to be performed. Thus, even a beginner can easily perform the treatment processes and become familiar with the technique. Any of the means included in the kit for simulating bone treatment can be in any form described in the "Treatment kit" section above.

For simulation, it is possible to actually produce a production model (of, for example, hydroxyapatite) and an assisting member when necessary, and perform a simulation of the surgical operation in accordance with the determined treatment process.

(Graft)

Also disclosed is a graft used for treating a bone. The graft has a shape substantially representing a difference between a bone model representing a bone which is a subject of treatment and a target bone model and a target bone model to which the treatment aims. The shape representing such a difference is realized by any molding method. The molding method may be 3D shaving, RP, or NC processing, but not limited to these. 3D shaving is preferable since 3D shaving can realize substantially any shape. Preferably, the shape of the graft is determined by a difference between the target bone model and the bone model defined by the Screw Displacement-Axis method or the affine transformation method.

(Treatment system)

Also disclosed is a system for treating a bone. The system includes (A) means for obtaining a bone model representing a bone which is a subject of treatment; (B) means for obtaining a target bone model to which treatment aims; (C) means for determining a treatment process which is to be performed on the bone (e.g., determining an assisting member necessary for the treatment process to be performed on the bone) based on the bone model and the target bone model; and (D) means for performing a surgical operation using the determined treatment process (e.g., using the assisting member).

The means for obtaining a bone model, the means for obtaining a target bone model, and the means for determining a treatment process, and the means for performing a surgical operation may be in any means described in the "Surgical operation method" section above.

(Simulation system)

Also disclosed is a system for simulating bone treatment (e.g., surgical operation). The system includes: (A) means for obtaining a bone model representing a bone which is a subject of treatment; (B) means for obtaining a target bone model to which the treatment aims; (C) means for determining a treatment process which is to be performed on the bone (e.g., determining an assisting member necessary for the treatment process to be performed on the bone) based on the bone model and the target bone model; and (D) means for performing a simulation of the bone treatment based on the determined treatment process (e.g., using the assisting member). Conventionally, a malunited bone or the like was gradually corrected by performing surgical operations within a possible range with trial and error. By contrast, the present system realizes a simulation of the treatment process to be performed. Thus, even a beginner can easily perform the treatment processes and become familiar with the technique. Any of the means included in the system for simulating bone treatment can be in any form described in the "Treatment system" section above.

For simulation, it is possible to actually produce a production model (of, for example, hydroxyapatite) and an assisting member when necessary, and perform a simulation of the surgical operation in accordance with the determined treatment process.

(Computer program)

The present invention, in an aspect thereof, provides a program for making a computer execute processing for determining a treatment process to be performed on a bone. The processing includes the steps of: (A) obtaining a bone model representing a bone which is a subject of treatment; (B) obtaining a target bone model to which treatment aims; and (C) determining a treatment process to be performed on the bone based on the bone model and the target bone model. The program according to the present invention can allow the treatment process to be performed on the bone (e.g., bone cutting, movement of bone fragments) to be determined in order to achieve a desired purpose (e.g., bone correction). Thus, even a physician with little experience can perform the treatment appropriately.

By executing the program for making a computer execute processing for determining a treatment process to be performed on a bone, the computer provides a method for determining the treatment process. By executing the program for making a computer execute processing for determining a treatment process to be performed on a bone, the computer acts as an apparatus for determining the treatment process.

The above description is provided regarding bones, but it should be understood that the present methods are applicable to portions of a body other than bones (e.g., soft portions such as skin).

The following examples are provided for the purpose of illustration. Only Example 7 is an example of the invention, but the remainder assist in understanding of the invention.

EXAMPLES

In the following examples, experiments on humans were performed after obtaining informed consent in compliance with the criteria defined by the Ministry of Health, Labour and Welfare in Japan or equivalent authoritative governmental offices.

(Example 1: Production of template assisting members and application thereof)

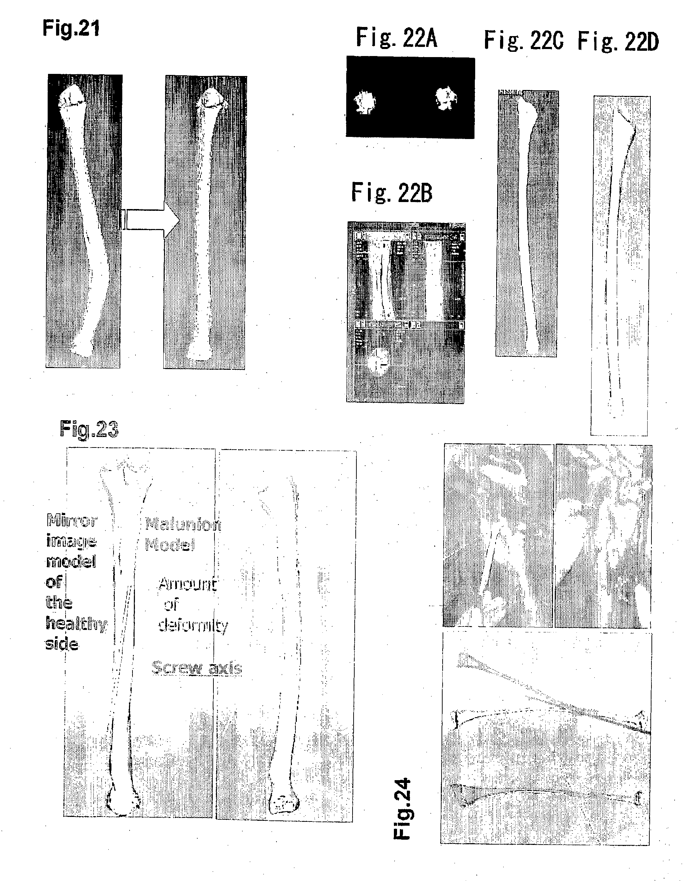

In this example, template assisting members were produced after performing a preoperative simulation (Figure 21) using actual deformed bones as a model. In the case of a fracture malunion on the forearm, the degree of deformity of the radius and the ulna is one of the main factors of restriction of pronation and supination. Pronation refers to rotating the forearm such that the palm is directed downward, and the supination refers to rotating the forearm such that the palm is directed upward (such that the palm is to receive something). It is desirable to correct the deformity as accurately as possible. The present inventor performed accurate correction osteotomy through a preoperative simulation using CR or MRI three-dimensional images. The methods and results will be described below.

There were 6 cases, and the patients were all male in the age range of 12 to 31 years with the average age of 19.2 years. The correction osteotomy was performed on a deformity in radius diaphysis in 5 cases and radius distal in 1 case. The time period from injury to osteotomy was 11.6 months on average. The preoperative angle of deformity on x-ray was 22.8 degrees, the preoperative movable range of the forearm was limited to 62 degrees for pronation and 25 degrees for supination. The average period of follow-up survey was 6.6 months.

(Creation of a model)

For each of these cases, a subject bone model was extracted by semi-automatic marking (Figure 22B) from CT or MRI two-dimensional image data of both forearm (Figure 22A) using specialized software , and segmented (Figure 22C). Thus, a three-dimensional bone surface model was created (Figure 22D).

The forearm bone model of each patient was overlapped with the proximal portion and the distal portion of the mirror image of the healthy arm, so that the screw axis of the deformity and the deformity amount were calculated, and an optimal site for osteotomy and correction amount were determined (e.g., Figure 23).

For the first 3 cases, the surgical operation was performed with reference to the deformed site and the deformity amount obtained by the simulation, the computer images and the like. These cases were grouped as group A (Figure 24).

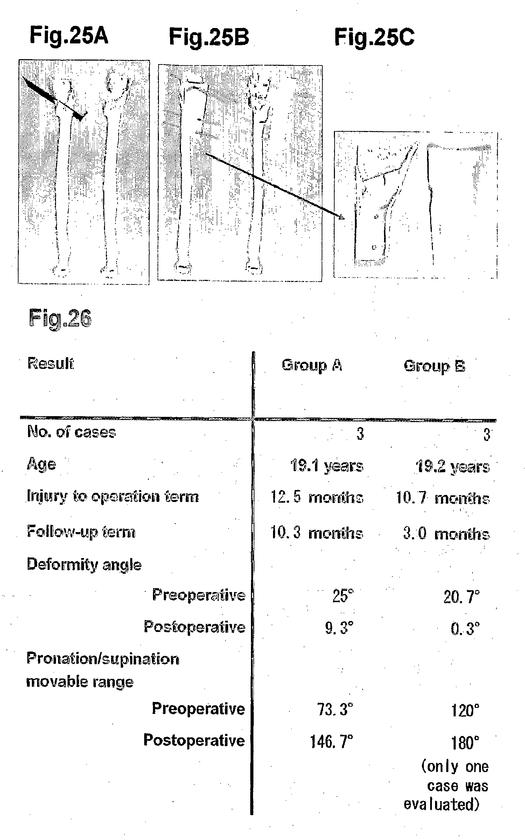

For the second 3 cases, the site for osteotomy and the correction amount were determined based on the CT data (Figure 25A), and an osteotomy template was designed on the computer. The osteotomy template designed was such that it fits snugly the bone surface of the malunited site and has a guide hole, through which Kirschner wires (also referred to as the "K-wires") which would be parallel to each other when the bone is corrected, can be inserted (Figure 25B). The template also has a bone cutting clit. A guide for retaining the restored state with a K-wire passing therethrough was also designed, and an actual production model of the guide was produced by photo printout and used for the surgical operation (Figure 25C). These cases were grouped as group B.

(Results)

Both groups were substantially the same in age, time period from injury to the surgical operation, and the preoperative degree of deformity. After the operation, the deformity angle of group A was insufficient at 9.3 degrees, whereas the deformity angle of group B was 0.3 degrees, which means almost complete correction. The movable range for pronation and supination was changed from 73 degrees (preoperative) to 146 degrees (postoperative) in group A. In group B, the movable range for pronation and supination was changed from 120 degrees (preoperative) to 180 degrees (postoperative) although the result was evaluated in only one case 6 months after the surgical operation. The results are shown in Figure 26.

(Example 2: 8-year-old male child; closed wedge osteotomy for supracondylar fracture of the humerus)

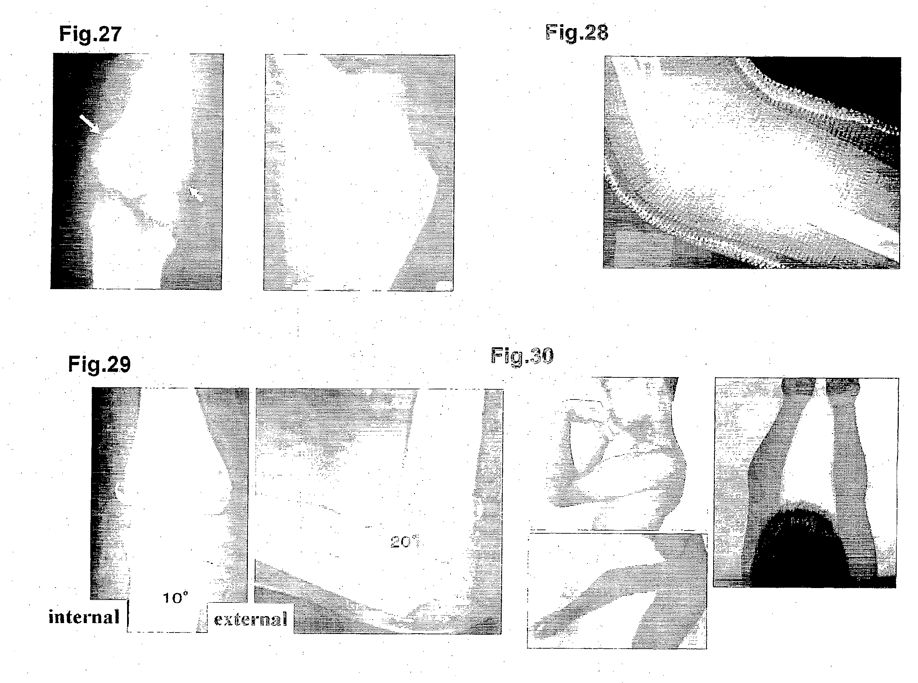

In this example, it will be demonstrated that the closed wedge osteotomy is applicable using the present methods A surgical operation was performed by the disclosed method on an 8-year-old male patient having a supracondylar fracture of the humerus, which is a representative fracture around the elbow joint of children. This case is known as easily resulting in cubitus varus. Figure 27 shows x-rays illustrating the affected site (the left x-ray shows a front view, and the right x-ray shows a side view thereof).

Figure 28 shows the progress of hyper-extension in the cast caused by the initial treatment. It is shown that dorsiflexion occurs since no appropriate treatment was performed.

Figure 29 shows the state of this patient 2 years after the initial treatment. It is shown that varus deformity and hyper-extension is left and natural correction is not sufficient even 2 years after the bone union. In the front-view x-ray (left in Figure 29), the elbow exhibits about 10° of varus (the forearm is slightly internally flexed; normally, the forearm should be externally flexed at about 10 degrees). In the side-view x-ray (right in Figure 29), the distal humerus exhibits about 20° dorsiflexion with respect to the axis of the humerus. Normally, the distal humerus should exhibit about 20° anterior flexion.

Figure 30 shows an aftereffect of hyper-extension and varus deformity. The upper left photo shows a flexion disorder, by which the arm can bend at up to only 90 degrees. Normally, the arm should bend at up to 130 to 140 degrees. The lower left photo shows 30° hyper-extension . Normally, the arm can only extend at about 10 degrees. The right photo shows cubitus varus.

(Correction osteotomy simulation using an MRI model)

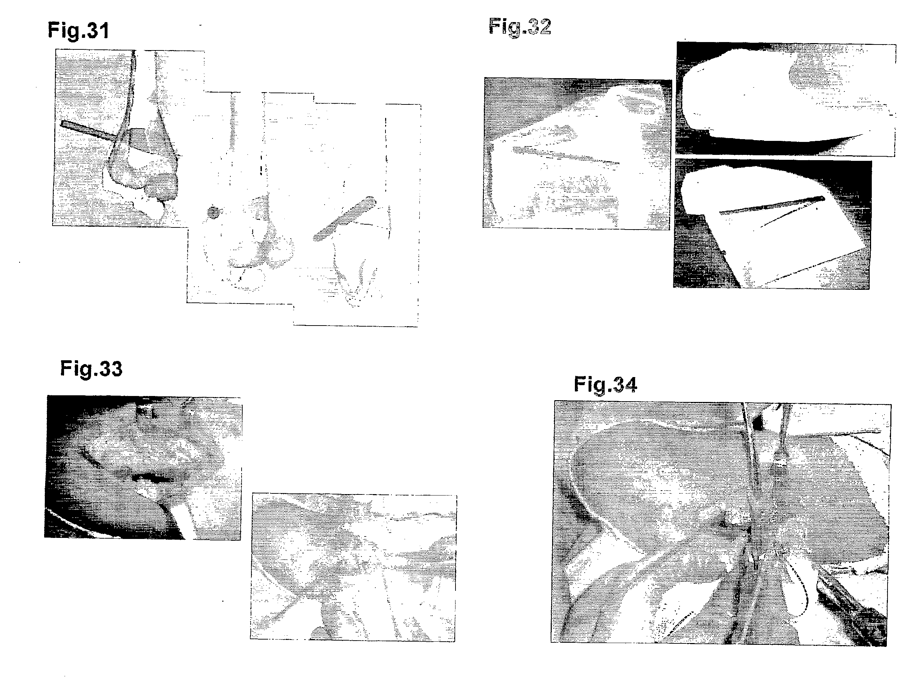

On this patient, a surgical operation was performed using a three-dimensional model created from MRI data (see Figure **31**). The three-dimensional model was matched to the mirror image of the healthy arm as a target bone model, so that the screw axis and angle of deformity were found. The deformity can be accurately corrected by excising a wedge-shaped bone having the screw axis as an apex and closing the cutting portion. It is impossible for the operator to accurately determine the direction and angle of osteotomy by visual inspection during the surgical operation.

The matching may be performed either manually or by use of a computer using an ICP (Iterative Closest Point) algorithm.

(Photo printout)

Based on a template produced by matching, a photo printout model was produced. The model was described in the STL format. (The STL format is one format of three-dimensional CAD data. By the STL format, the data representing the surface is polygonized using triangles. "STL" represents Standard Triangulation Language. The STL format is especially used in photo printout as a standard file format.) The STL design was converted into cross-sectional data at a slicing pitch of 0.1 mm. Based on the sliced data, the surface of the photocurable resin was scanned and thus cured by an LD excited UV solid-state laser light via a galvanometer mirror. Layers each having a thickness of 0.1 mm were laminated, so as to produce a three-dimensional model. The model was washed and completely cured by a post-cure device. As the photo printout apparatus, Rapid Meister 3000 available from CMET Co., Ltd. was used.

Figure **32** shows a photo printout procedure.

(Surgical operation)

Using the template, the surgical operation was performed to correct the deformity. Figure 33 shows the outer side of the left arm during the surgical operation. The shoulder is on the right and the hand is on the left of the photos. The template was fit to the lateral condyle of the humerus. The left photo of Figure **33** shows the state immediately after the operation site (arm) was exposed by incision and retraction. The right photo of Figure **33** shows the state after the template and wires were set after the site was exposed.

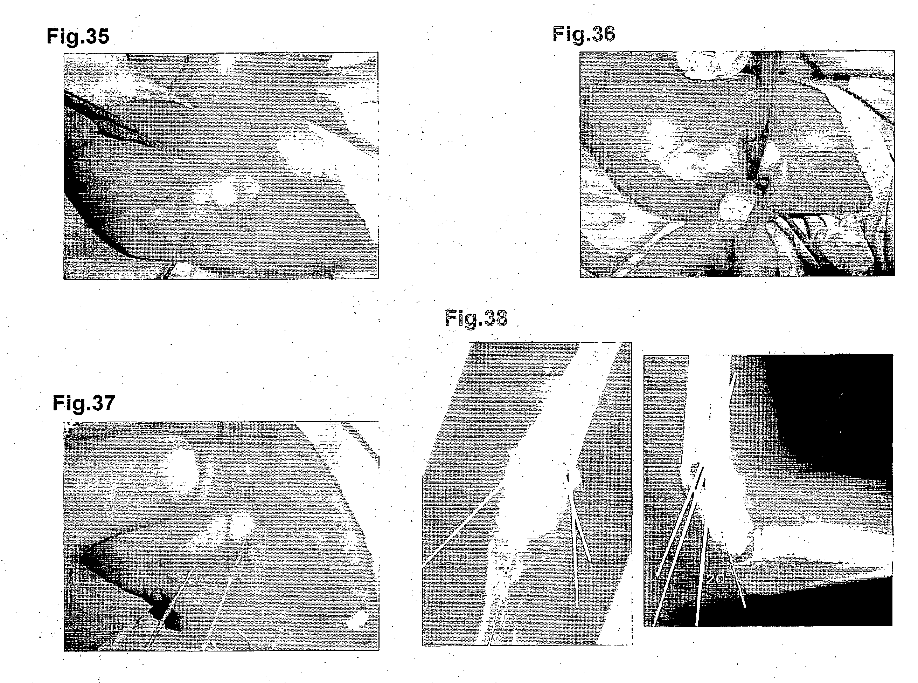

After setting the template and the like, the bone was cut using a bone saw (Figure **34**). Figure 35 shows the state after the bone was cut. As shown, here, it is appreciated that the method allows the bone to be accurately cut at the first trial, which is impossible by an operation performed merely manually.

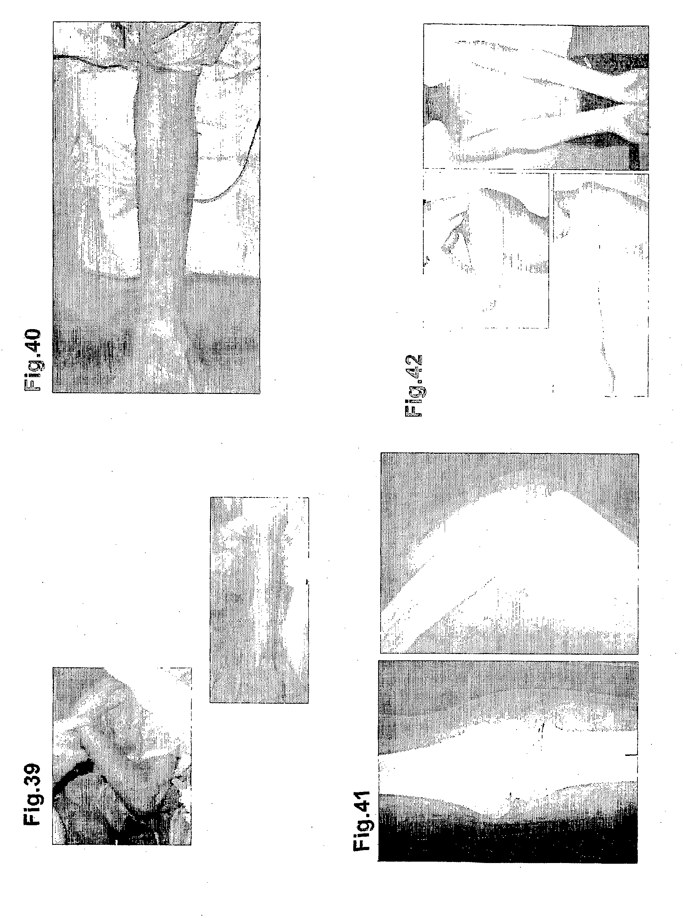

After the bone was cut, an excessive bone portion was excised as shown in Figure 36. After the bone portion was excised, the positions of the bone fragments were corrected such that the wires were parallel to each other (Figure **37).** Figure **38** shows x-rays immediately after the surgical operation. The bone is fixed by the K-wires. As shown here, fine correction and fixation are realized by the treatment. The left photo of Figure 38 shows 5° valgus, and the right photo shows 20° anteversion of the distal humerus. The movable range of the elbow was normalized in both flexion and extension immediately after the surgical operation. Even during the surgical operation, it was shown that the flexion was at 130 degrees and the extension was at 10 degrees (Figure **39**). Figure **40** shows an external appearance immediately after the surgical operation. The values of flexion and extension are close to normal values. It was demonstrated that the disclosed method for treating a bone provides the effect of recovering substantially normal functions.

Figure **41** shows x-rays 1 year after the surgical operation, and Figure **42** shows an external appearance of the patient 1 year after the surgical operation. Surprisingly, it is understood that the normal state is kept even 1 year after the surgical operation, or rather the patient has almost completely recovered with the postoperative unevenness disappearing.

This example is directed to the case where excision and rotation are necessary.

The above-described effect was not expected from the prior art, and it is demonstrated and understood that the present invention is highly useful in medical practice.

(Example 3: 21-year-old male; open wedge osteotomy for a left forearm fracture malunion)

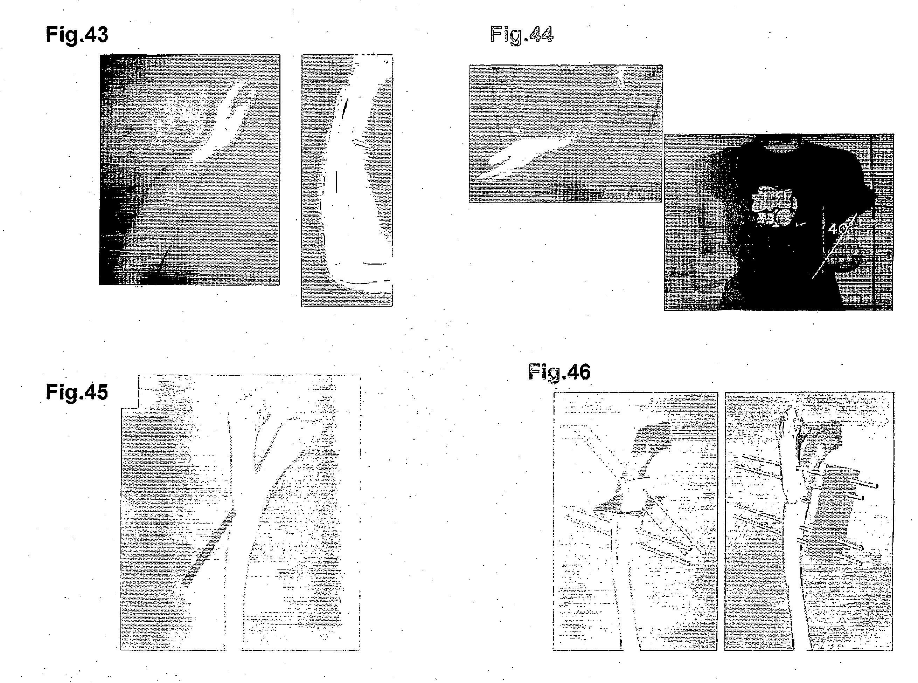

In this example, it will be demonstrated that open wedge osteotomy is applicable to the present invention. As an example, a case of a 21-year-old male patient with a left forearm fracture malunion was used. This patient showed an excessive deformity of the radius (Figure **43**). An forearm fracture is often seen among men in early youth to youth, and is considered to often cause a malunion when treated in a cast.

As shown in Figure **44,** this patient complained of a deformity and restriction of supination of forearm (i.e. , such that the palm is to receive something). The supination was restricted to about 40 degrees (normally, 80 to 90 degrees).

It was found by computer simulation that the deformed bone was matched to the healthy bone by rotating the bone at 30 degrees about the screw axis (Figure **45**). A template was designed based on this information. It was found that by subtracting the bone and a pre-produced rectangular parallelepiped shape and cylindrical shape from the block (Figure **46**, left) (Boolean operation), a template which fits the bone surface showing the feature of the malunited portion, which has a cutting section indicating element and which allows wires to be inserted thereto (Figure **46**, right) can be designed. A correction guide was designed in a similar manner.

(Surgical operation)

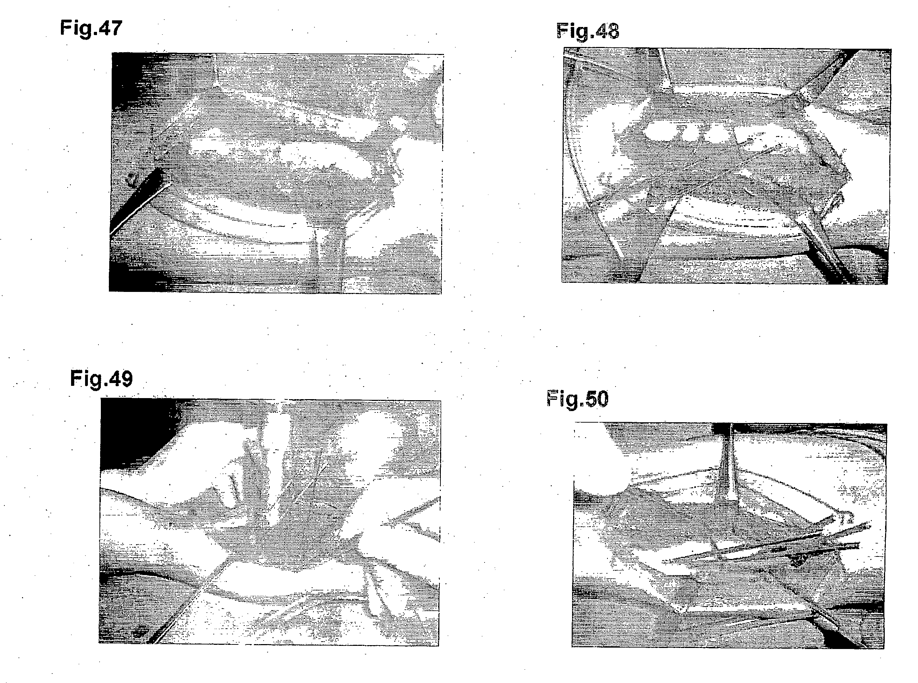

The patient was treated using this template. As shown in Figure 47, the malunited site of the bone was exposed by incision and retraction. Then, the osteotomy template was applied to the malunited site and it was confirmed that the osteotomy template completely fitted the malunited site (Figure **48**). Then, the template was pierced with Kirschner wires (stainless steel wires generally used in orthopaedics) and fixed. Then, as shown in Figure **49,** the bone was cut along the slit of the template. As shown in Figure **50,** the osteotomy was completed and the template was removed.

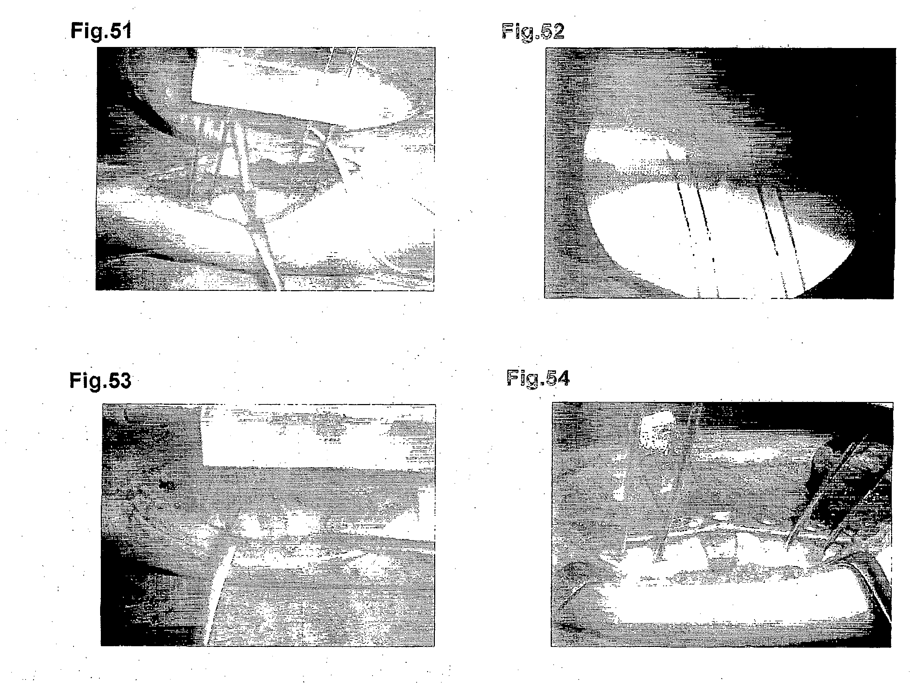

As shown in Figure **51,** the correction guide was outserted into the wires to perform correction. The cutting portion was opened. Although it was not clear whether the deformity was corrected or not by a visual inspection of the site of operation (the surface is seen as extremely projecting forward), it was confirmed by an x-ray during the operation that the deformity was accurately corrected (Figure **52).**

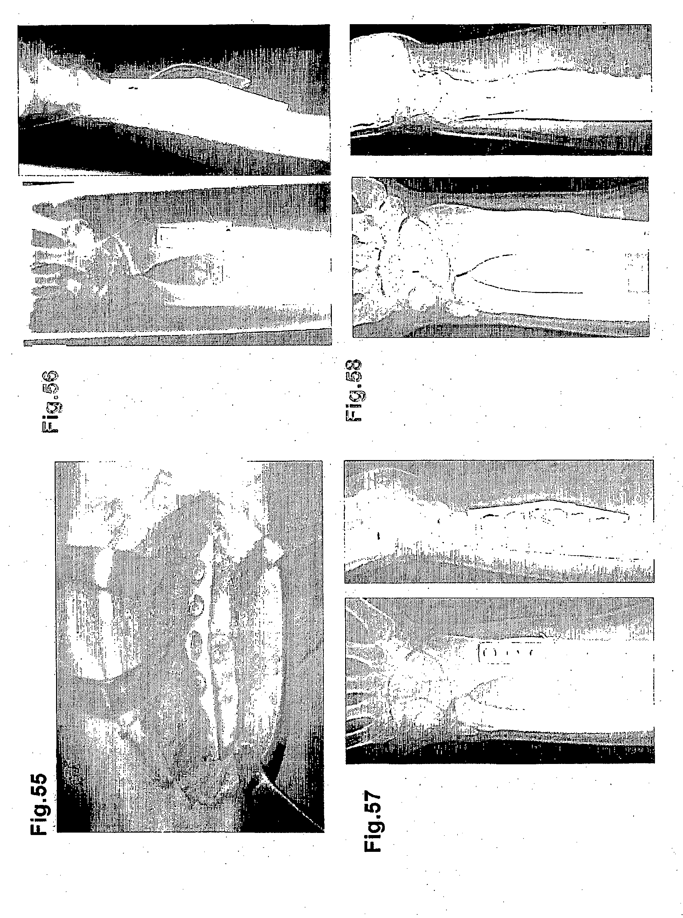

Then, as shown in Figure 53, the iliac bone was shaped into a wedge-shape and implanted. After this, the bone and the bone graft were fixed with a titanium plate and screws (Figure **54**). The Kirschner wires were then removed (Figure **55**). A postoperative x-ray shows that fine correction was realized (Figure **56).**

(Postoperative progress)

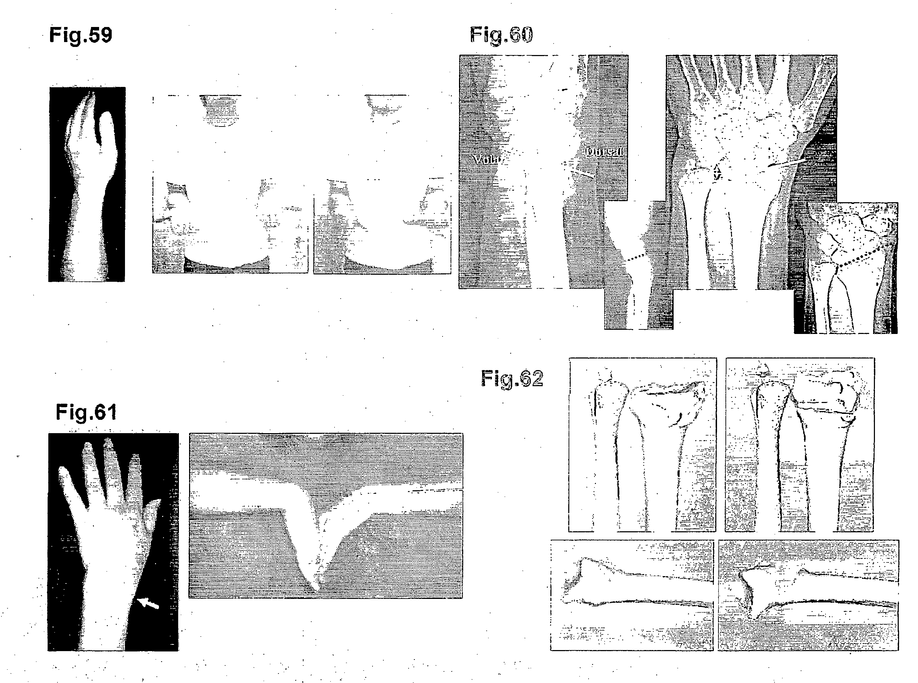

This patient was examined 9 months after the surgical operation. As shown in Figure 57, the bone was united while the deformity of the radius was corrected to an almost normal state. 1 year and 1 month after the surgical operation, the plate was removed (Figure **58**). The external deformity had disappeared, and the forearm had been recovered from the rotation disorder (Figure **59**, left). The supination was possible to up to 90 degrees (Figure 59, center). The pronation was normal (Figure **59,** right).

(Example 4: 48-year-old female; a fracture malunion on the distal end of the left radius)

It will be demonstrated that the deformity can be corrected even for a middle-aged woman. As an example, a 48-year-old female patient having a fracture malunion on the distal end of left radius was used.

This patient had a fracture malunion on the distal end of left radius as shown in Figure **60**. The left photo of Figure **60** shows a left view thereof. As shown, the articular surface shows dorsiflexion. The solid line represents a tilt of the articular surface, which shows about 20° dorsiflexion. In a normal state, as represented with the dotted line in Figure **60,** lower center, the articular surface shows 10 to 20° volar flexion. The upper center photo of Figure **60** shows a front view of the affected side. The radius is shortened by about 4 mm as represented with the arrow, and the tilt of the articular surface of about 10 degrees is smaller than the normal tilt of 20 to 30 degrees. The left photo of Figure **60** is a reversed photo of the healthy side as reference. The line represents a tilt of the artificial surface of 25 degrees.

The fracture of the distal end of the radius suffered by this patient is a representative fracture among middle- and senior-aged women, and is likely to result in a malunion. A slight malunion is often left without treatment since the bone is united in a fine manner, but patients of or under 50 years of age, and older patients actively doing physical exercise, often complain of a disorder in the movable range and pain during physical exercise.

This patient has an external deformity and disorder of the movable range of the left wrist joint as shown in Figure **61**. Such a deformity of the distal end of the radius is referred to as a fork-type deformity since it looks like a fork seen from the projecting side. As shown in Figure **60**, right, the volar flexion is restricted to about 35 degrees. The normal angle of volar flexion is 60 to 80 degrees. This patient also complained of pain of the wrist joint during physical exercise.

(Three-dimensional simulation)

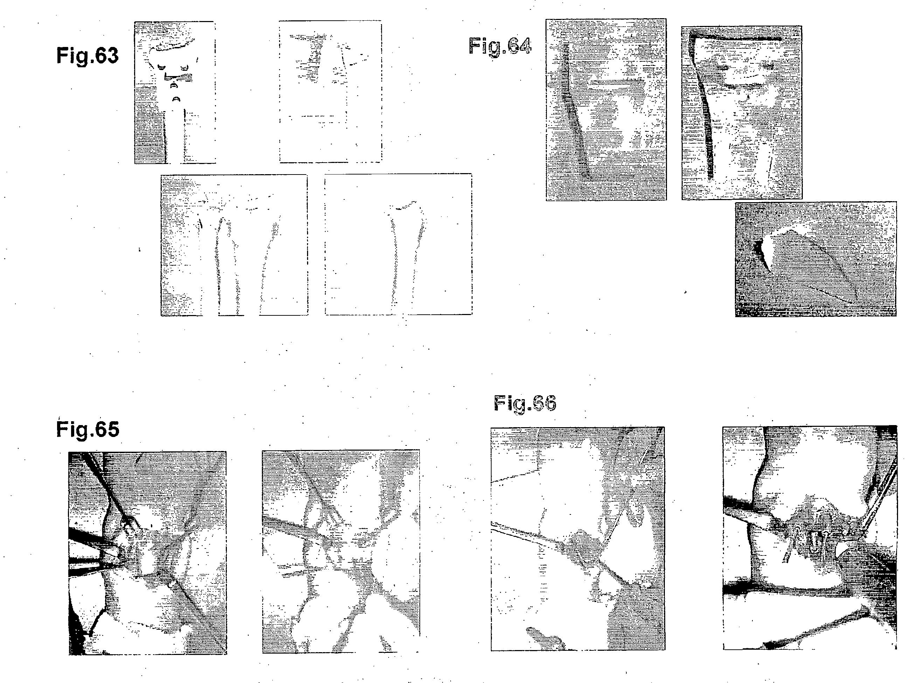

A three-dimensional simulation of the bone of this patient was performed (Figure **62). As** a result, it was calculated that 30° open wedge osteotomy was necessary. Based on the calculation result, an osteotomy template, a correction guide and a bone graft were designed (Figure **63**). Figure **64** shows the procedure for producing the template or the like by photo printout.

(Surgical operation)

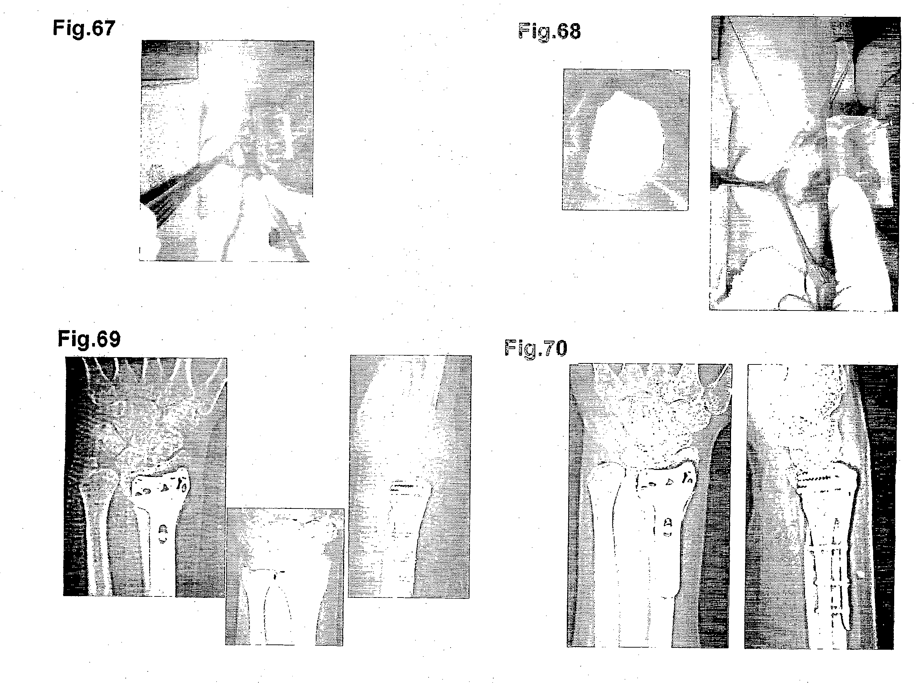

A surgical operation was performed on the patient using the template and the like. The left photo of Figure **65** shows the malunited bone was exposed by incision and retraction. As shown in Figure **65,** right, the template was fit to the site. As shown in Figure **66,** left, the template was fixed with Kirschner wires and, and as shown in Figure **66,** right, the bone was cut with a bone saw. As a correction guide, a stainless steel guide (cylindrical guide having slits) was used so as not to damage the resin. The deformity was corrected as shown in Figure 67 using the guide. A graft which was obtained by shaping hydroxyapatite using CAD before the surgical operation was used (Figure **68).** Such an artificial graft contributes to simplification and higher accuracy of the surgical operation.

Then, a postoperative x-ray was taken after the bone was fixed by the template (Figure **69).** As shown, the correction is finely done. The central photo of Figure 69 is a reversed image of the healthy side. Through the comparison, it was confirmed that the deformity was corrected to the state which is substantially the same as that of the healthy side.

Figure **70** shows a state 4 months after the surgical operation. As is clear from the photo, the corrected state was maintained. The artificial bone and the surrounding bones were being united to each other.

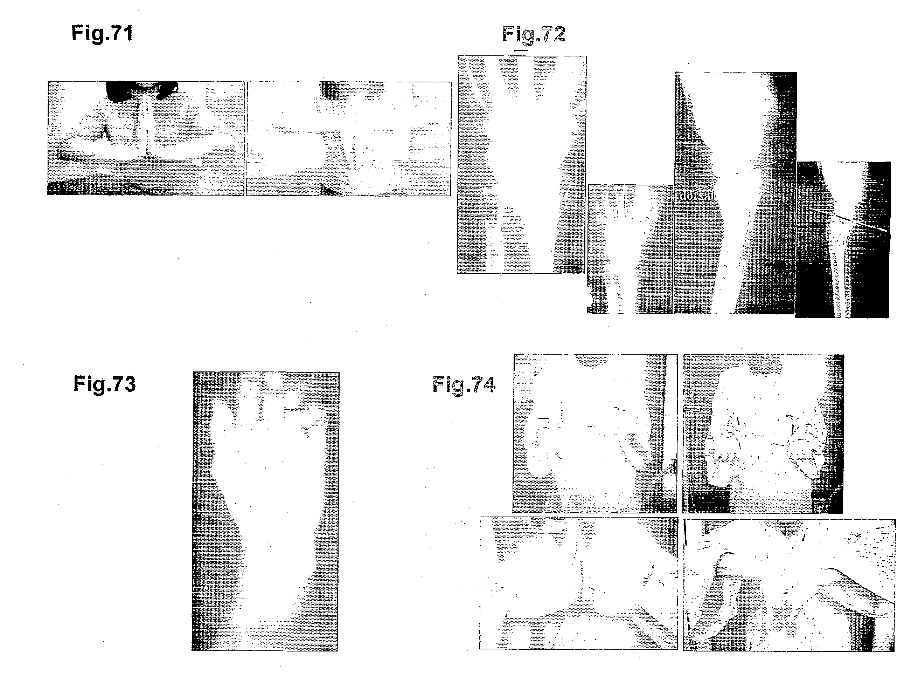

Figure **71** shows the patient 4 months after the surgical operation. The restricted volar flexion of the left wrist joint had been recovered to 80 degrees. The external deformity and the pain at the wrist joint disappeared, and the patient was highly satisfied.

It was demonstrated that the present method provides the effect of recovering the external appearance and mobility to the patient's satisfaction.

(Example 5: 67-year-old female; a fracture malunion on the distal end of the radius)

Here, it will be demonstrated that a similar case of a fracture malunion on the distal end of the radius can be corrected for an older patient (67-year-old woman). In Figure 72, the two lower photos each show a reversed photo of the healthy side. A side view of the affected side shows dorsiflexion of the articular surface, although a front view does not show an extreme deformity. Figure **73** shows the external deformity of the patient. Figure **74** shows a mobility disorder. As shown here, the left hand has a pronation disorder (upper left photo) of the left hand and a volar flexion disorder (lower right photo) of the left wrist joint. This patient also complained of numbness of the hand due to nerve disturbance. In this type of case, nerve disturbance may be caused by deformity.

(Simulation)

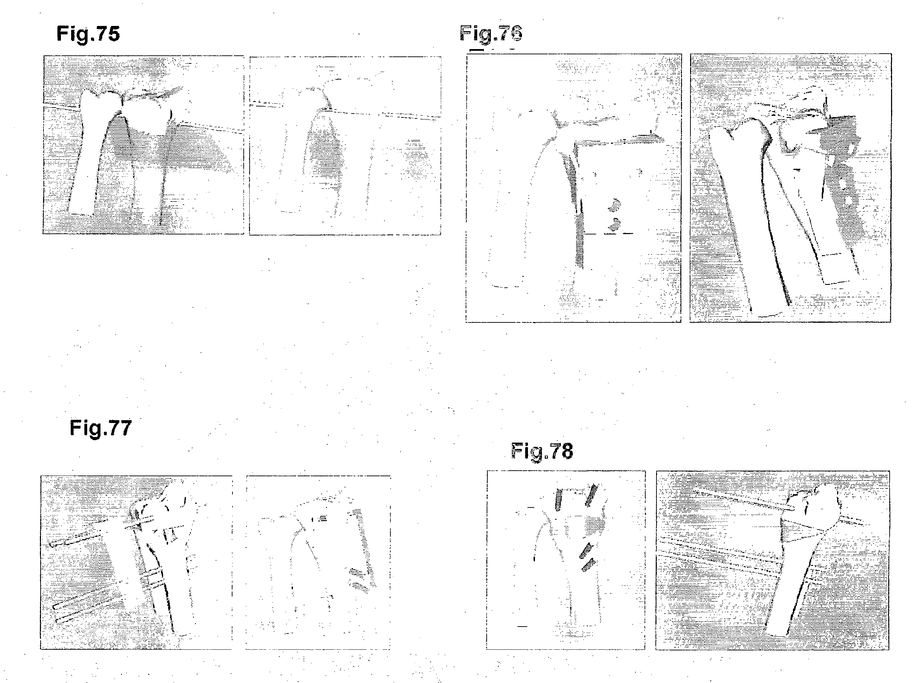

A model was created from this patient, and a three-dimensional simulation of osteotomy was performed as shown in Figure **75.** It was found that by rotating the bone fragment about the axis shown in Figure **75,** the deformity can be corrected. Based on the model, an osteotomy template was designed (Figure **76**), and a correction guide was designed (Figure **77).** Figure **78** shows the corrected state which is expected by this correction method.

(Surgical operation)

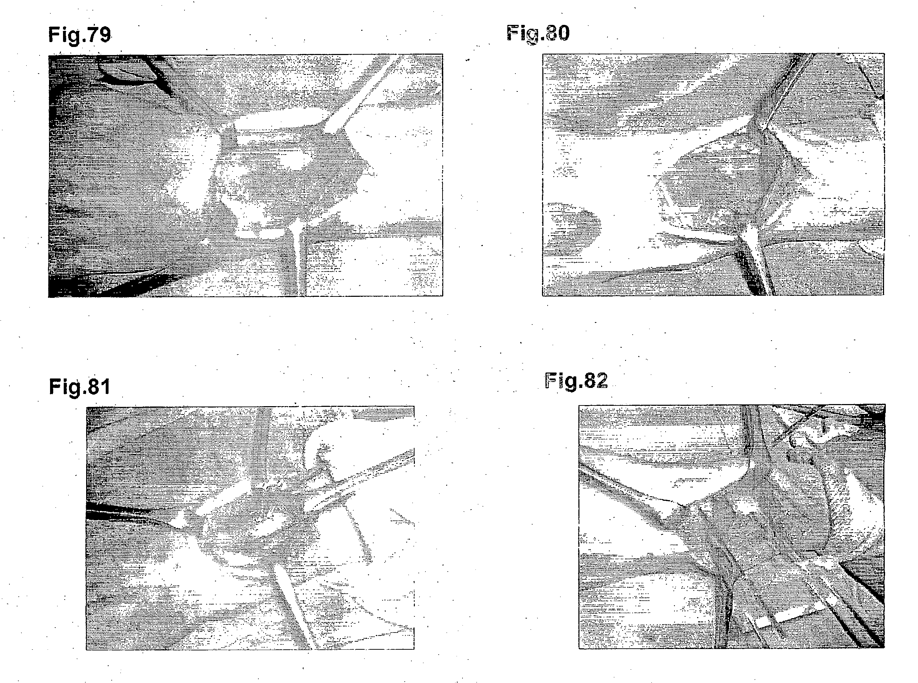

A surgical operation was performed on the patient using the template and the like. As shown in Figure **79,** the malunited bone was exposed by incision and retraction. As shown in Figures **80** and **81,** the template was set. Then, the bone was cut, and the osteotomy was finished as shown in Figure **82.**

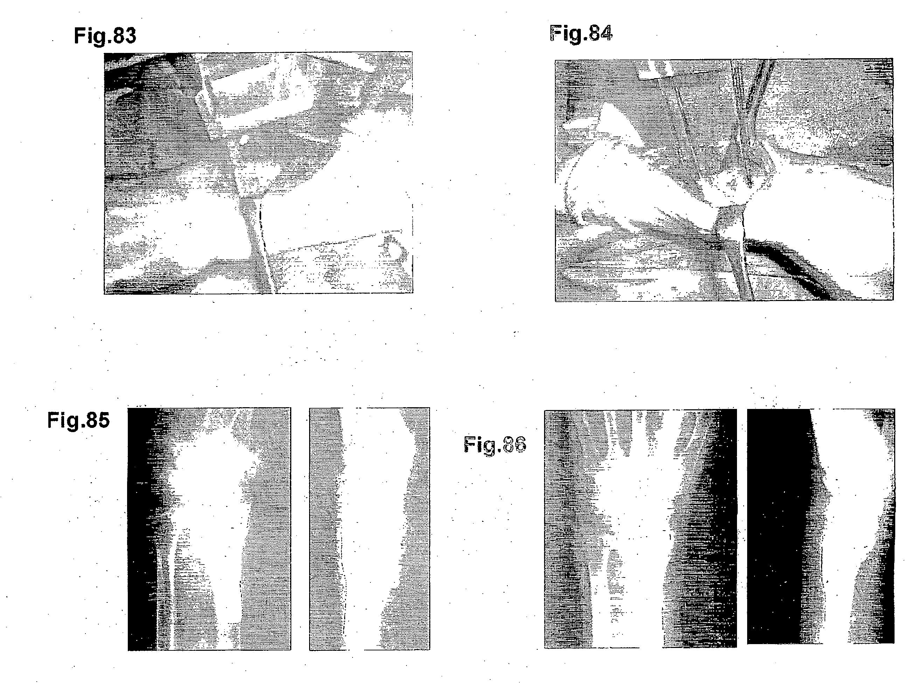

Then, a bone graft was implanted in this patient. Since the patient was relatively old and was considered to be poor in bone union capability, an autobone (her own iliac bone), which is more advantageous for bone union, was used (Figure **83**). Figures **83** and **84** show the bone implantation. Figure 85 shows x-rays taken after the bone was fixed with the titanium plate.

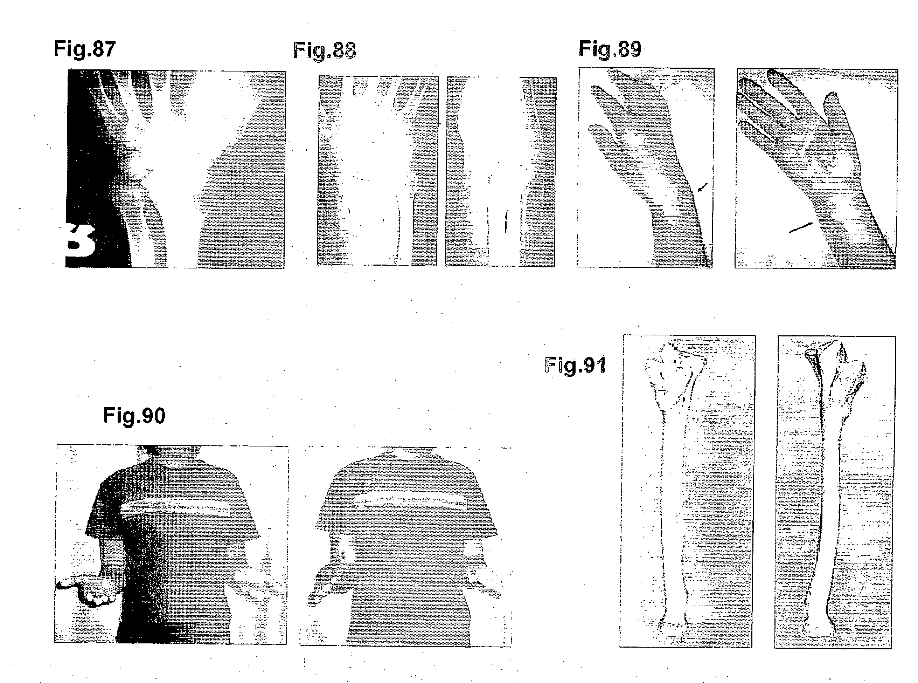

Figure **86** shows x-rays taken **4** months after the surgical operation. As is clear, the bone was united while the corrected state was maintained. The numbness and ache were alleviated, and the movable range of the wrist joint was normalized as demonstrated by the volar flexion of 65 degrees, the dorsiflexion of 64 degrees, the pronation of 80 degrees, and the supination of 90 degrees. Figure **87** shows a front view of a normal wrist joint.

Thus, it was demonstrated that the present method is effective even to correction of malunited bones of old patients. Accordingly, it was found that the bone correction can be performed on the middle-aged and senior-aged people, for whom bone correction conventionally had been abandoned.

(Example 6: Radius distal was fractured by a motorbike accident received conservative treatment by a previous physician, resulting in a malunion; open wedge osteotomy accompanying distraction)

It will be demonstrated that the present method is effective even to osteotomy accompanying distraction. The patient in this example had a fractured distal end of the radius in a motorbike accident, and received conservative treatment by a previous physician. As a result, the bone was malunited (Figure **88) .** This patient had a deformity at the wrist joint (Figure **89**) and a rotational disorder of the right forearm (Figure **90**). The supination is normal, but the pronation is restricted to 50 degrees (Figure **90**). The patient complained of pain in the wrist joint during physical exercise.

(Simulation)

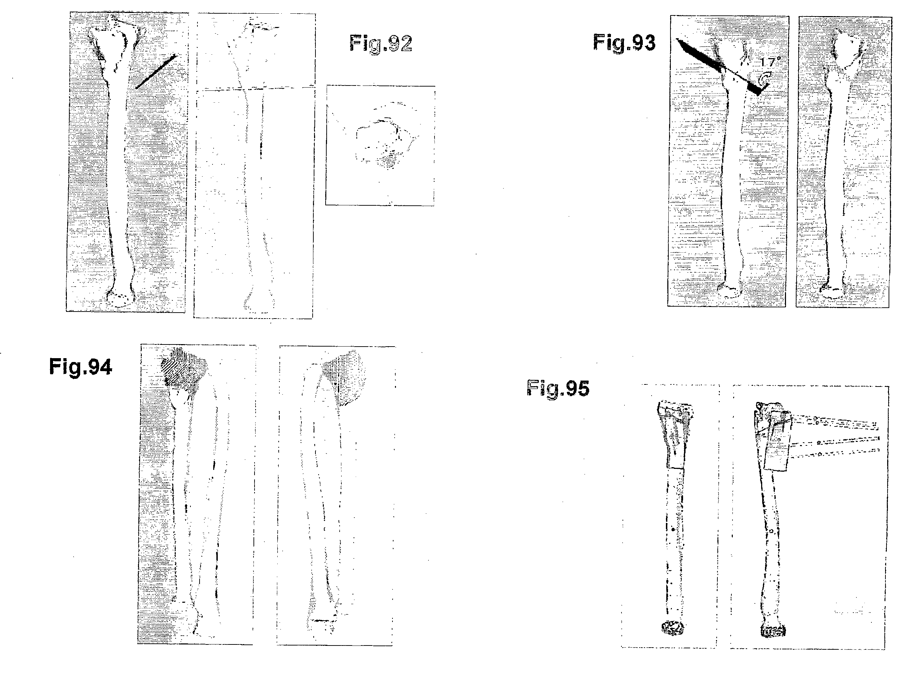

A treatment simulation of this patient was performed (Figure **91).** A three-dimensional model of the radius was matched to the mirror image of the healthy side. It is observed that the distal end is shortened and the articular surface is tilted. Thus, the screw axis was obtained (Figure **92).** Since the axis was distanced from the bone, the correction was to be performed while distracting the bone. In the case where the screw axis is distanced from the bone like this, osteotomy accompanies distraction (or may accompany shortening) (Figure **93**). Figure **94** shows the pre-correction state and the post-correction state. The hatched portion (in the left figure; the left side of the overlapping portion, and in the right figure, the right side of the overlapping portion) shows the pre-correction state. The vertical-lined portion (in the left figure; the right side of the overlapping portion, and in the right figure, the left side of the overlapping portion) shows the post-correction state.

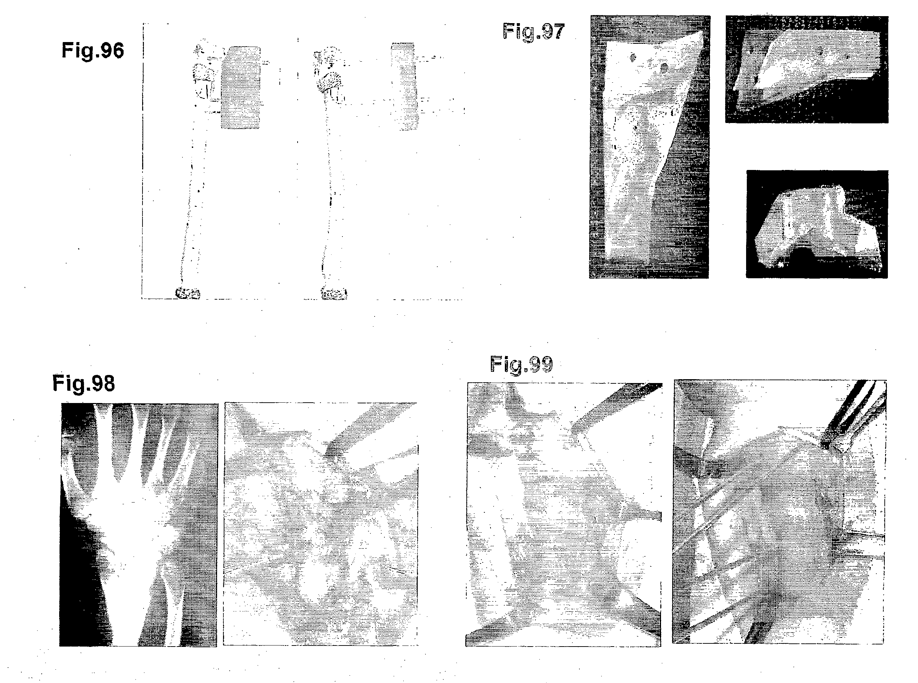

Based on this information, an osteotomy template was designed (Figure **95**). Next, a correction guide was designed (Figure **96**). The oblique-lined portion shows the shape of the bone graft to be implanted. The guide and the like were produced by photo printout (Figure 97).

(Surgical operation)

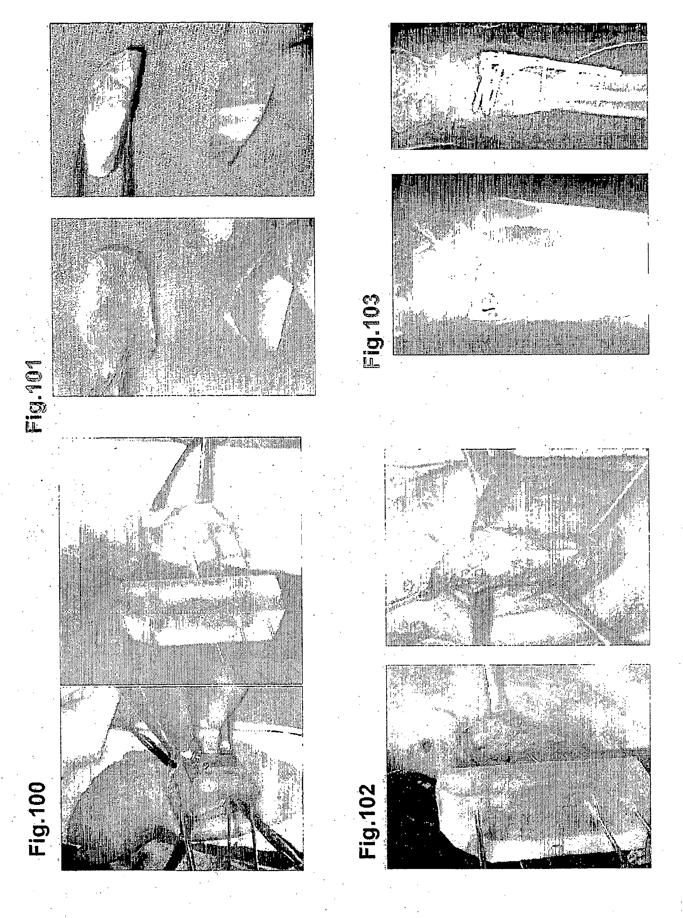

A surgical operation was performed on the patient using the template and the like. As shown in Figure 98, the malunited portion of the bone was exposed by incision and retraction. The template was fit to the site and fixed with Kirschner wires (Figure **99**). Then, the bone was cut as shown in Figure **100.** The cutting portion was opened and the bone fragments were arranged such that the Kirschner wires were parallel to each other. The Kirschner wires were fixed with the correction guide. As shown in Figure **101,** a bone graft (iliac bone) was shaped with reference to the estimated bone defect. As shown in Figure **102,** left, the bone graft was inserted, and as shown in Figure **102,** right, the bone fragments were fixed with a titanium plate. Figure **103** shows the state immediately after the surgical operation. Although the portion around the cutting portion appears slightly abnormal due to the callus which had been generated before the surgical operation, the position and tilt of the articular surface are normal.

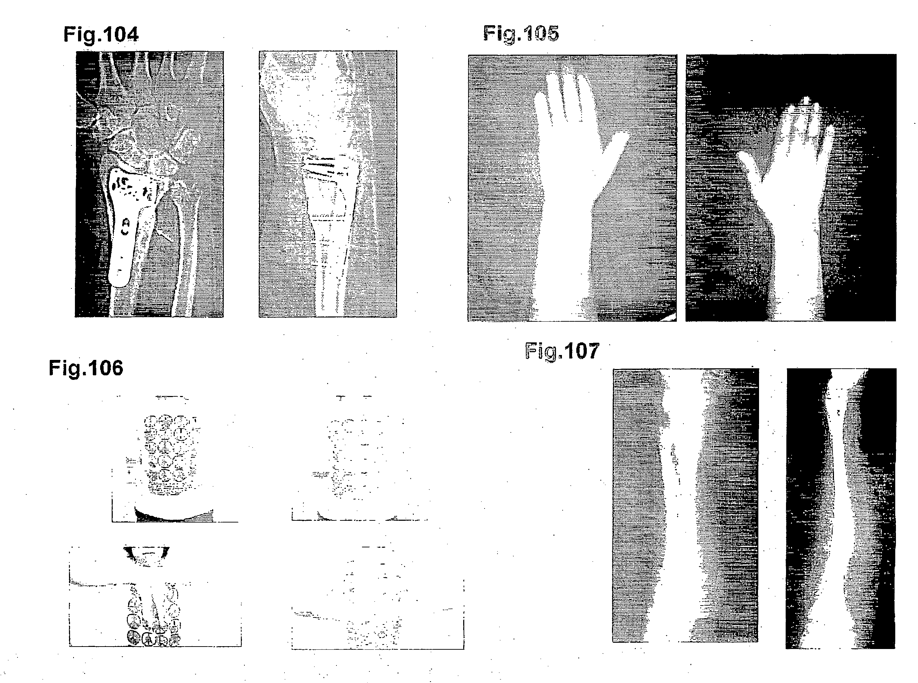

The postoperative progress was fine. As shown in Figure **104,** 9 months after the surgical operation, the bone union was realized and the portion around the bone graft was progressively remodeled for fine correction (see the arrow). The external deformity was finely corrected (Figure **105).** The movable range was completely normalized (Figure **106).**

(Example **7:** 13-year-old male; rotational osteotomy for a fracture malunion on left radius diaphysis)

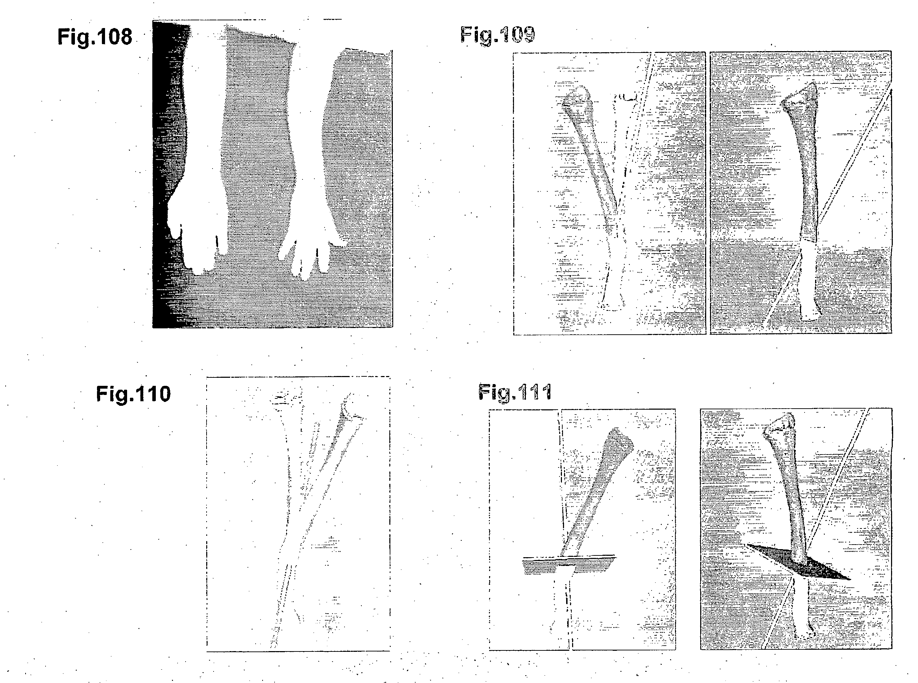

It was investigated whether or not treatment would be possible using rotational osteotomy. As an example, a 13-year-old boy having a fracture malunion on the left radius diaphysis was used (Figure **107**). About 30° deformity was seen in radius diaphysis . It was also seen that the left forearm had a supination disorder (Figure **108**). The pronation was restricted to 60 degrees, and the supination was restricted to -20 degrees. Pronation refers to rotating the forearm such that the palm is directed downward, and the supination refers to rotating the forearm such that the palm is directed upward (such that the palm is to receive something). Normally, the pronation and supination both are possible up to 80 to 90 degrees. This case is seen as a simple angular deformity by x-rays, but by measurement using three-dimensional models, it is understood as in Figure **109** as a 45° rotation deformity about an oblique line.

As shown in Figure **110,** it was planned to cut the bone along one plane and rotating the bone about the plane. This corrects the rotation deformity as shown above in the x-ray.

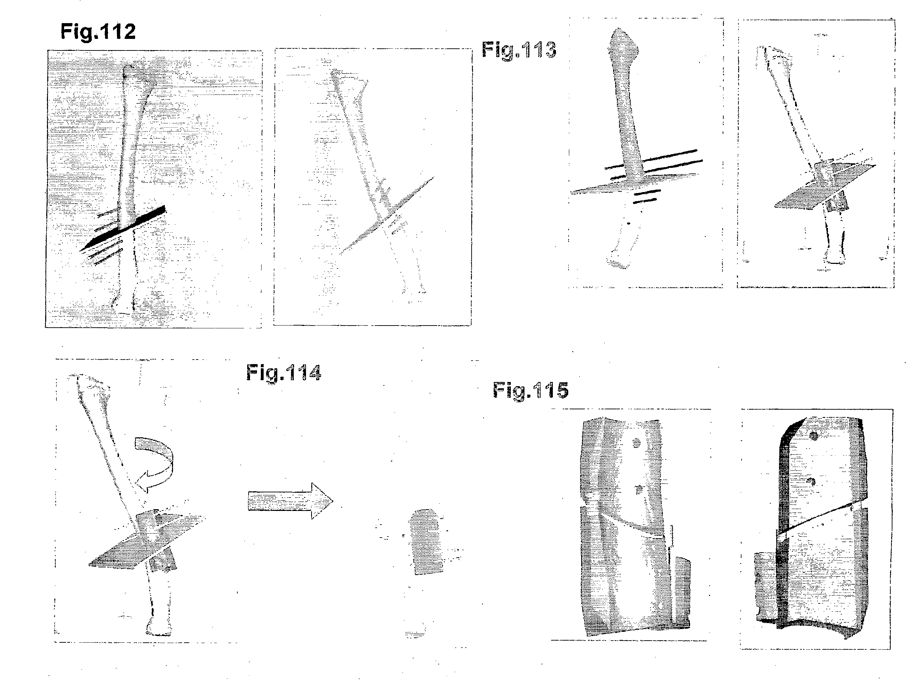

Next, a first guide for rotational osteotomy was designed as follows. First, a cross-section of the bone, which is vertical to the screw axis at the position where the screw axis is closest to the center of the bone, was set as the cutting section (Figure **111**). Next, as shown in Figure **112,** four cylindrical wires parallel to each other were set in a corrected state. Then, as shown in Figure **113,** the corrected state was returned to the initial deformed state. The cylindrical wires were axially offset. As shown in Figure **113**, right, the block was applied to the bone in this state, and the bone, the cutting section, and the cylindrical model were subtracted from the block (Boolean operation). Then, as shown in Figure **114,** by cutting the bone as indicated by the template and arranging the wires (cylindrical wires) parallel to each other, the deformity is corrected. Figure **114,** right, shows a guide for keeping the wires parallel to each other.

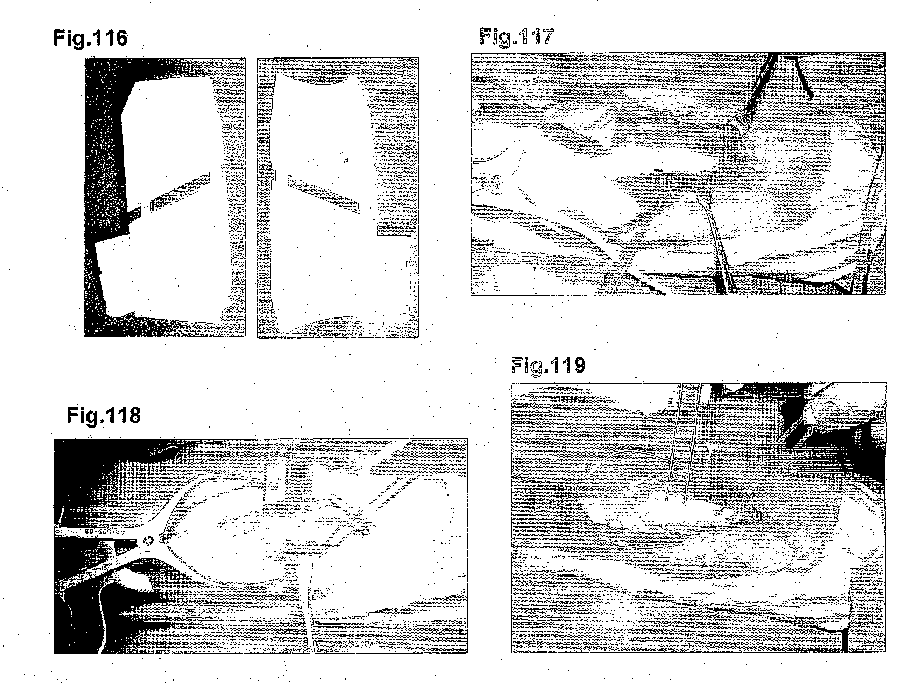

Figure **115** shows general views of the osteotomy template shown on the computer. It is clearly seen that the osteotomy template has openings and a slit. The production model of the osteotomy template was produced by photo printout as shown in Figure **116.**

(Surgical operation)

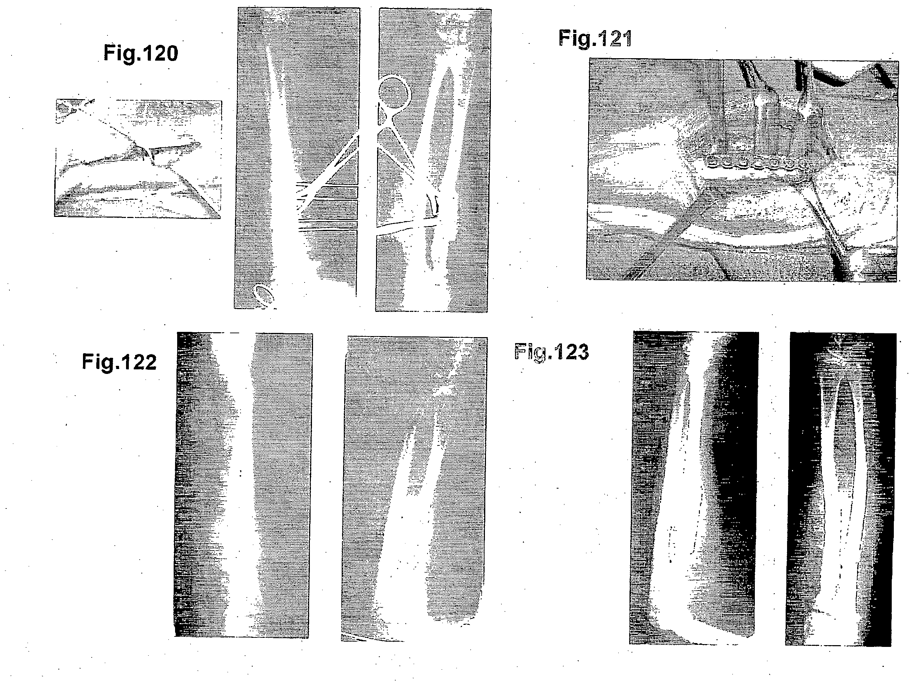

As shown in Figure **117,** the cutting portion was exposed by incision and retraction. The template was fixed with two wires being inserted into each of two portions of the screwed bone (Figure **118**). As shown in Figure **119,** the osteotomy was finished and the template was removed. Then, the correction was performed with the correction guide, and the corrected state was confirmed with an x-ray (Figure **120**). The rotational deformity was finely corrected merely by rotation. As shown in Figure **121,** the bone fragments were well fixed with a titanium plate. Figure **122** shows x-rays immediately after the surgical operation. It is shown that the bone is fixed in the corrected state. X-rays taken 6 months after the surgical operation (Figure 123) show that fine bone union was realized while the corrected state was maintained. Clinically, both the pronation and the supination were improved respectively to 70 degrees. Thus, it was demonstrated that the present invention provides the effect even when the distraction is necessary.

(Example 8: Case where comparison with the healthy side is impossible; affected on both sides of the body)

Next, it will be demonstrated that the present method is also effective in the case where comparison with the healthy side is impossible since both sides of the body are affected. In the case where both sides of the body are deformed or where the degree of deformity is excessive and complete correction is not intended, a model created on a computer is manually cut to simulate appropriate correction. A template can be produced based on the simulation. It was demonstrated that the principle of screw axis does not need to be used.

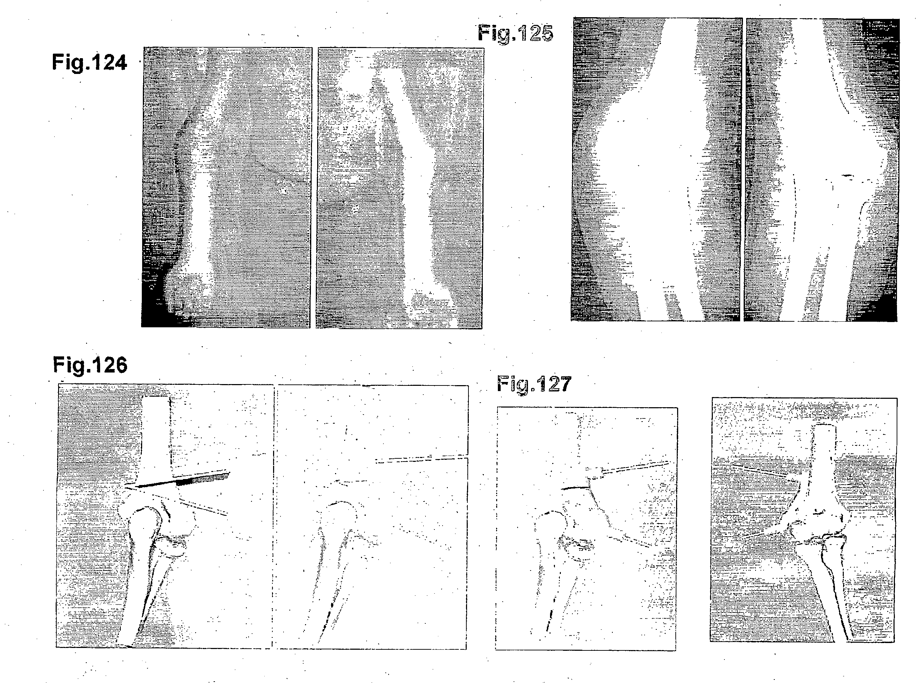

As an example, the patient shown in Figure **124** was used. This patient has cubitus varus on both sides. In such a case, it is impossible to obtain a screw axis by matching the affected side to the healthy side. Figure **125** shows x-rays of the cubitus varus.

For this patient, a cutting section, a correction direction, and a correction amount were arbitrarily determined and planned as shown in Figure **126.** A screw axis was set to be an intersection line of two planes in Figure **126.** It was determined that the bone was cut along the screw axis at 30 degrees.

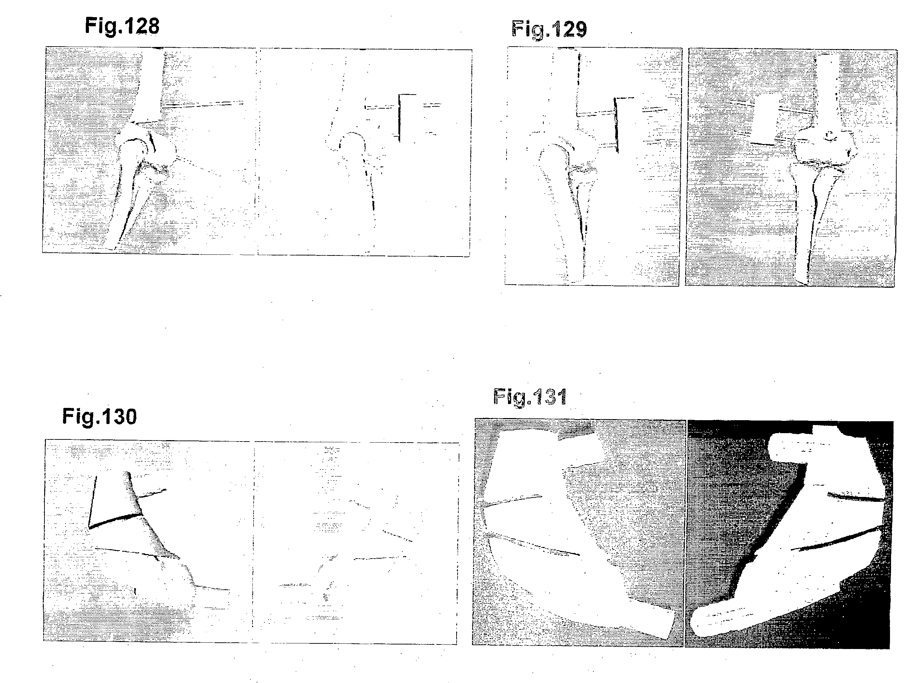

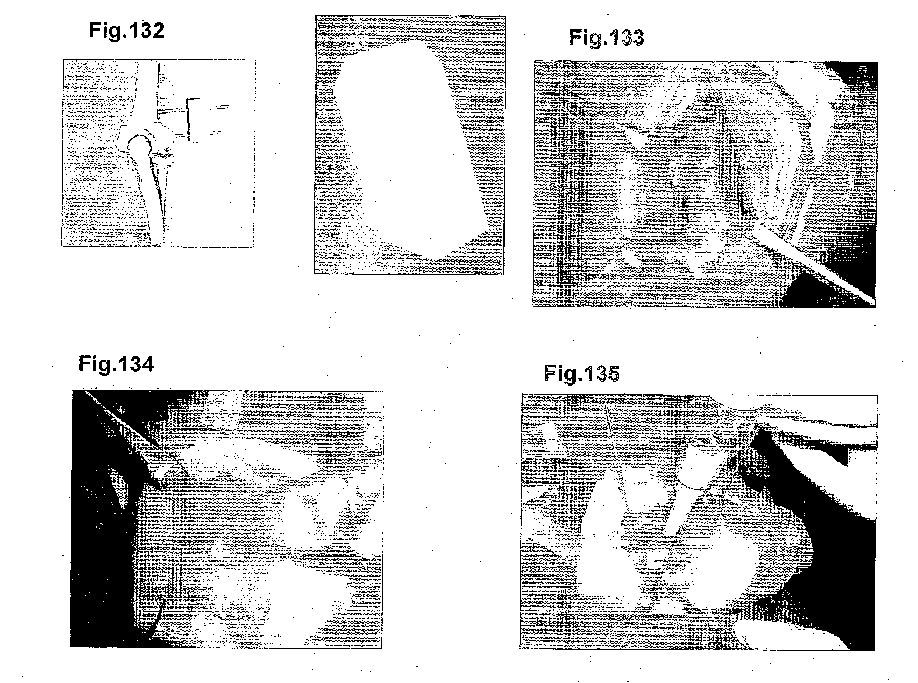

Based on this information, a template was designed (Figure **127**). A correction guide was also designed (Figures **128** and **129).** Then, the cutting section was designed (Figure **130**). Based on this model, an actual template (Figure **131)** and a correction guide (Figure **132)** were produced by photo printout.

(Surgical operation)

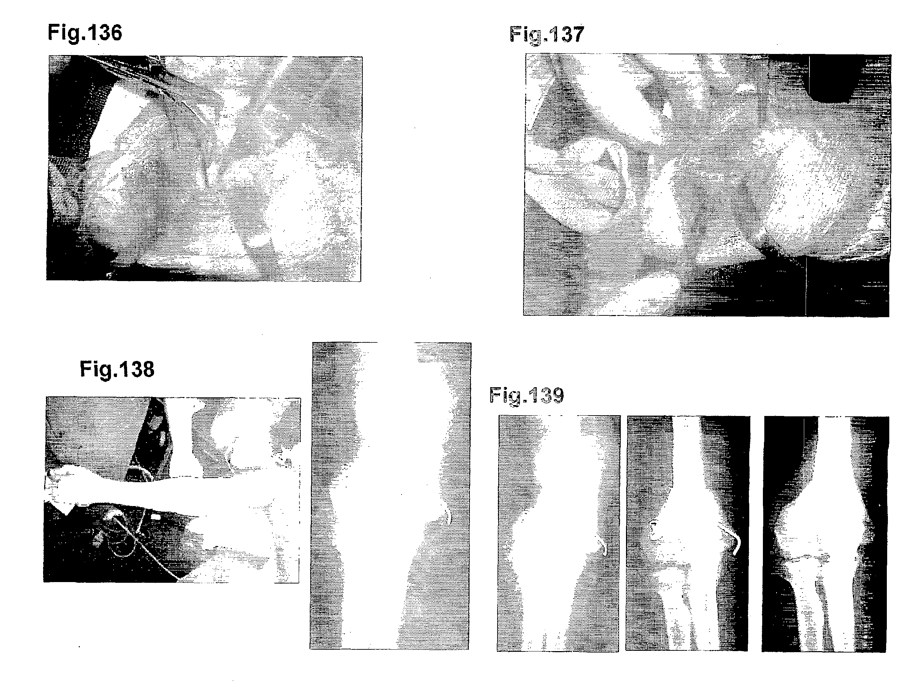

A surgical operation was performed using the template and the like. First, the cutting portion was exposed by incision and retraction(Figure **133**). The template was fit to the site as shown in Figure **134**. The bone was cut as shown in Figure **135,** and a wedge-shaped bone portion was excised as shown in Figure **136.** Figure 137 shows restoration.

After the operation, the correction was performed as expected as shown in Figure **138.** It was demonstrated that osteotomy using a template is possible without a screw axis, as long as the pre-correction and post-correction position information is available. As shown in Figure **139,** the bone of this patient was perfectly corrected by the surgical operation. As shown in Figure **139,** right, a slight dislocation occurred due to the lack of an internal fixation force and a slight deformity was left when the bone was completed united. However, the deformity caused almost no functional problem.

(Example 9: Use of an external fixation device)

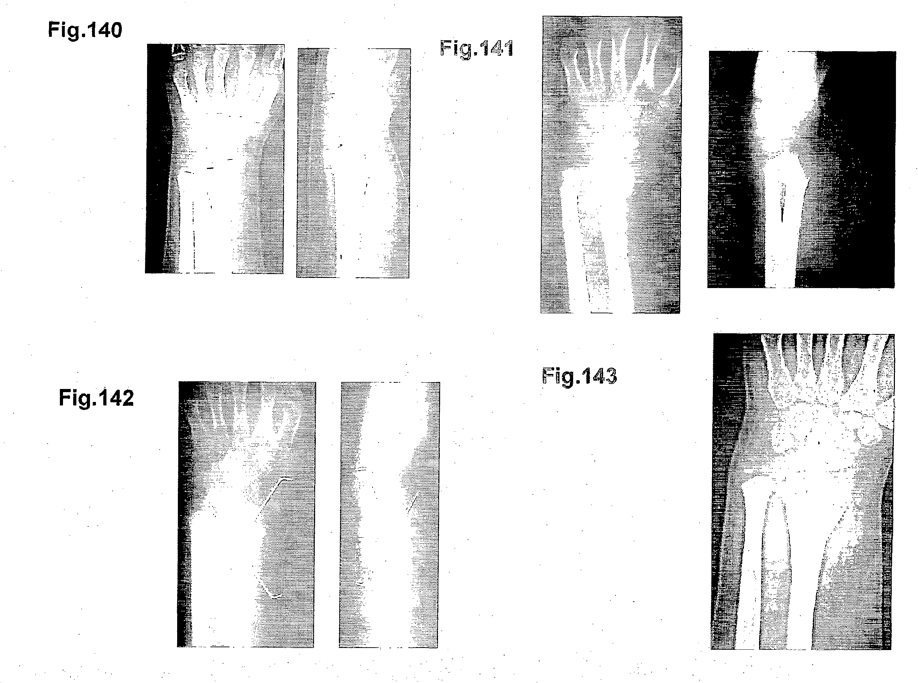

The present method was applied to a technique for gradually correcting a forearm deformity using an Ilizalov external fixation device and by use of a computer simulation. As an example, a 7-year-old boy having a fracture of the distal end of the radius (one representative forearm fracture of children) was used (Figure **140**). As shown here, the dislocation was slight. After this stage, the dislocation increased and deformity progressed in the cast as shown in Figure **141.** This is especially clear from the right photo of Figure **141.**

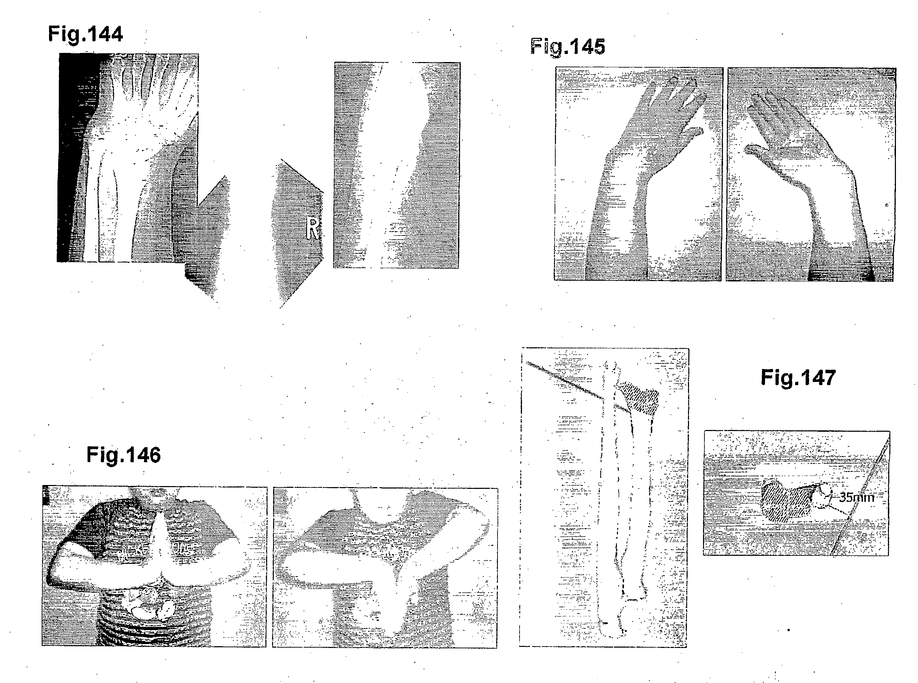

1 month after the injury, a surgical operation was performed (Figure **142**). 8 months after the surgical operation, deformity was observed in an x-ray (Figure **143**). It was a failure to thrive in the radius caused by an epiphysial line disorder. 5 years after the surgical operation, the radius was excessively shortened, and the tilt of the articular surface was abnormal as shown in Figure **144.** The left photo of Figure **144** shows a front view of the wrist joint, and the right photo of Figure **144** shows a side view thereof. As shown in the front view, the radius is shortened and the articular surface is tilted in the opposite direction to the normal tilt. As shown in the side view, the articular surface exhibits dorsiflexion. The central photo of Figure **144** shows a front view of the normal side. Figure **145** shows an external deformity.

As shown in Figure **146,** this patient had a volar flexion disorder at the wrist joint (Figure **146,** right).

(Simulation)

Based on the information on this patient, a surgical operation was simulated (Figure **147**). It was found that the articular surface of the radius was transferred to a substantially normal position by rotating the oblique-lined portion of the radius about the axis at 40 degrees as shown in Figure **147.**

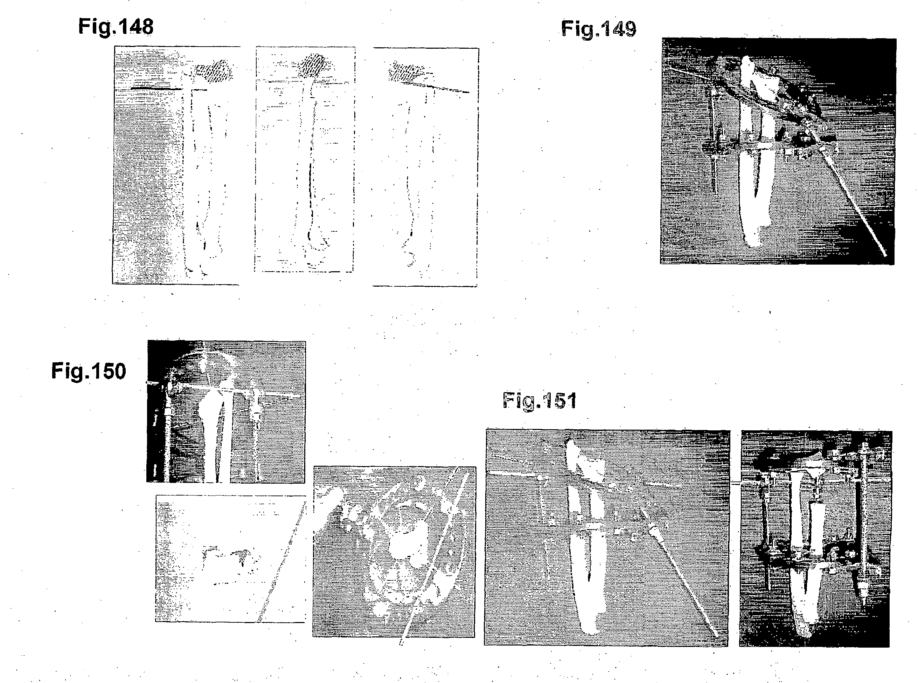

In an actual operation, the screw axis is determined by moving the oblique-lined portion to the normal position and converting the position information at that point (Figure **148**). However, it is impossible to move the bone by that distance in one surgical operation. Thus, gradual correction using an Ilizalov external fixation device was used (Figure **149).**

A full-size photo printout model of the forearm of the patient was produced from the CT data, and a mock surgical operation was performed (Figure **150) .** The radius was fixed to two rings via the wires. The rings were fixed by three supports. The rings were fixed with two supports among the three supports via hinges. The line passing through the two hinges was matched to the screw axis mentioned above. The radius was cut between the two rings.

As shown in Figure **151,** the third support was made to be extended using a nut. First, the two rings were tilted at 40 degrees, such that gradual correction and distraction could be performed about the screw axis by rotating the nut. The right photo of Figure **151** shows the state when the correction was finished. In actuality, the cut portion is distracted by 1 mm per day by callus being generated, and the distracted portion is buried with bone.

(Surgical operation)

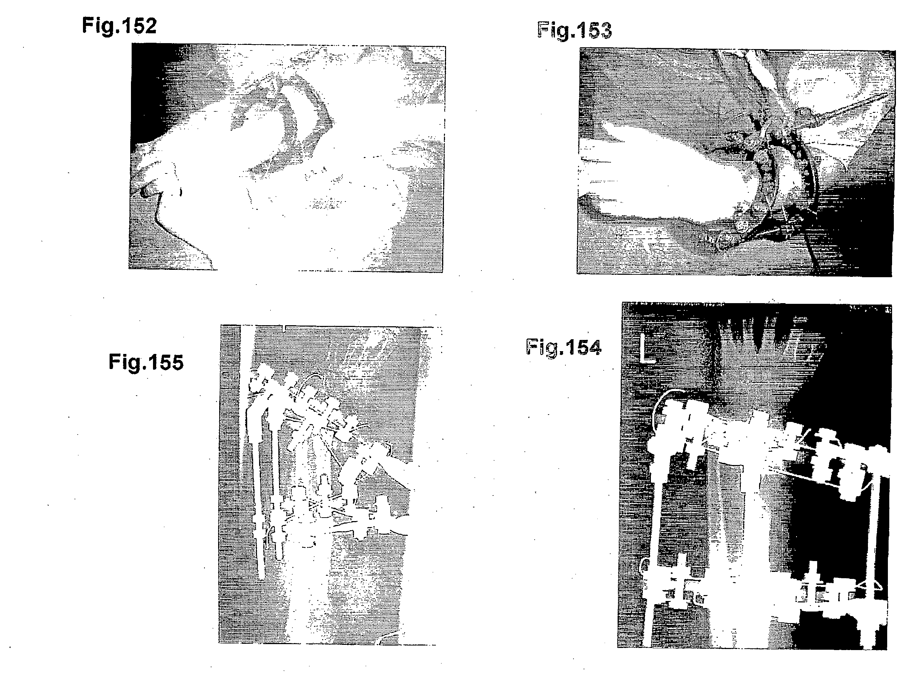

Using the above method, an actual surgical operation was performed as shown in Figure **152.** Figure **153** shows the state immediately after the surgical operation. An x-ray was taken after the surgical operation (Figure **154).** The state is relatively fine. 1 week after the surgical operation, the distraction was started (Figure **155**). It is shown that a thin layer of bone is generated.

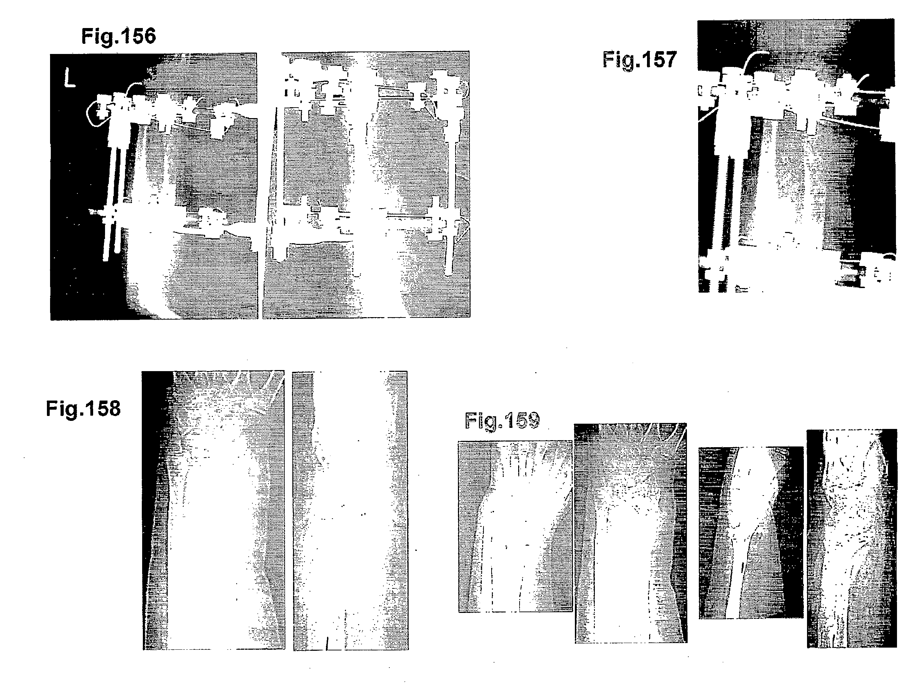

After the distraction was finished, no action was taken until the amount of callus became sufficient (Figure **156**). The distraction was finished 2.5 months after the surgical operation (Figure **157).** As shown in Figure **158,** the external fixation device was removed 3.5 months after the surgical operation. The corrected state was fine.

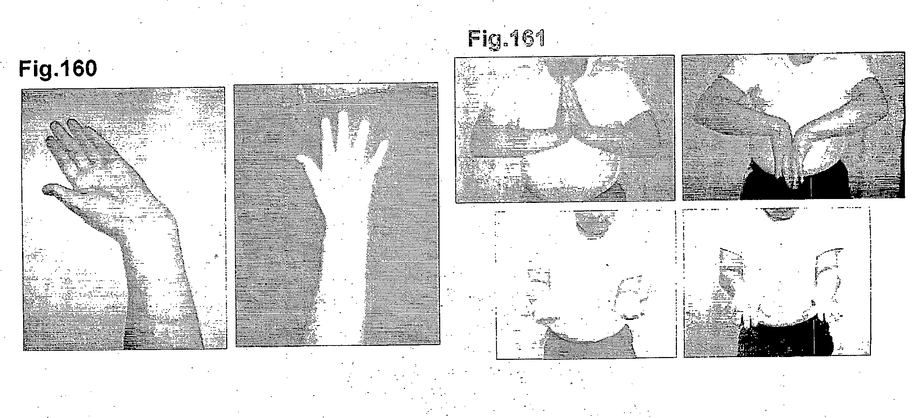

An x-ray was taken 4 months after the surgical operation, and compared with the x-ray before the surgical operation (Figure **159**). The preoperative and postoperative external appearances were compared (Figure **160**). As shown in Figure **161**, the state of the volar flexion of the wrist joint of the patient was improved (upper right photo of Figure 161) and the movable range of other portions were not substantially deteriorated (Figure 161).

Thus, it was found that the present method can be performed without a template. The Ilizalov method and the present method are different in that the present method uses a difference between the target bone model and the bone model. The Ilizalov method corrects the deformity based on a difference between the deformed side and the healthy side but based on a two-dimensional image. The present method has advantages in that correction can be performed accurately based on three-dimensional information; three-dimensional visualization is possible; and a mock operation can be performed using a three-dimensional model (produced by, for example, photo printout) before an actual operation. It was demonstrated for the first time by using the present method that a surgical operation method directly using three-dimensional data like in this example can provide a satisfactory effect.

(Example 10: scaphoid nonunion)

The present inventor developed a system for simulating a surgical operation of a deformed scaphoid nonunion using a three-dimensional image, and applied the system to 8 clinical cases. A surface model of the scaphoid was re-constructed on a computer from CT data of both wrist joints, and a distal portion and a proximal portion of the nonunion model were matched to those of a mirror image of the healthy, opposite side. Thus, an appropriate site and direction of the estimated defect of the bone and screw insertion were obtained by a simulation. Based on the resultant CAD data, a full-size photo printout model of the scaphoid was produced. In an actual surgical operation, restoration, bone implantation and screw insertion were performed using the model as a guide. In all the 8 cases, fine bone union and significant improvement in the clinical symptoms were obtained. The postoperative SL angle and RL angle were normalized. It was found that three-dimensional computer simulation is effective to perform accurate restoration and fixation of a scaphoid nonunion and to maintain the normal arrangement of the hand bone.

(Introduction)

In the scaphoid nonunion, the distal portion is usually volarly rotated and the proximal portion is usually dorsally rotated. As a result, a scaphoid bone causes volar flexion, and as a result, instability of carpus referred to as a DISI (Demonstrable Dorsal Intercalated Segment Instability) pattern. When this deformity is left without being treated, osteoarthritis starts with scaphoid joint of the radius and is finally expanded to the mid-carpal joint.

Main causes of progress of osteoarthritis are considered to be incongruity of the articular surface generated as a result of deformity of the scaphoid nonunion and the instability of the carpus. For the purpose of correcting the carpus malalignment and restoring the scaphoid into a normal anatomy to prevent the scaphoid nonunion from developing to the wrist arthropathy, it has been recommended to implant a volar wedge-shaped bone since the 1970's. In 1984, Fernandez reported a preoperative plan using simple x-rays of the front view and the side view (Fernandez DL, J Hand Surg 1984; 9A: 733-7). His method is a simple method based on two-dimensional information. An actual scaphoid nonunion has a complicated three-dimensional shape. As conventionally described, usual deformity patterns are various degrees of shortening, flexion, ulnar deviation, and pronation of a distal portion with respect to a proximal portion. The scaphoid is occasionally malunited, but when this occurs, the movable range of the wrist joint is reduced and the functions of the radiocarpal joint are spoiled.

For the last 10 years, CT apparatuses and computer technologies have progressed remarkably. Today, orthpaedic simulation using a three-dimensional model has become possible. The present inventor produced a three-dimensional model of carpal bones using CT data in order to simulate a surgical operation on the scaphoid nonunion. In this example, an attempt to simulate restoration of a deformed scaphoid nonunion using a three-dimensional image and application of this technique to 8 clinical cases will be described.

(Cases and methods)

For 8 patients with an average age of 24.3 years (18 to 42 years of age), a three-dimensional computer simulation was performed and then an actual surgical operation was performed. A follow-up survey was performed for 6 months or longer (Table I).

Table I: Patient Data

| Case | Age at surgery (yrs) | Gender | Affected side | Duration of non-union (months) | Follow-up term (months) | Time to bone union (weeks) | Clinical evaluation* | | | | | Radiographic evaluation | | |
|------|------|------|------|------|------|------|------|------|------|------|------|------|------|------|
| | | | | | | | F-E range (F/E)** | R-U range (R/U)*** | Grip strength (affected/ unaffected) | Pain | Wrist score | SL angle (pre./postop.) | RL angle (pre./postop.) | CL angle (pre./postop.) |
| 1 | 22.1 | F | R | 9 | 9.6 | 6 | 150(80/70) | 70(20/50) | 24/24 | none | 100 | 60/50 (45) | 10/14(17) | 5/15(17) |
| 2 | 20.2 | M | L | 3 | 9.4 | 7 | 135(70/65) | 55(15/40) | 46/49 | none | 90 | 85/45(50) | -17/-5(-3) | -18/0(3) |
| 3 | 18.2 | M | R | 6 | 6.1 | 12 | 145(75/70) | 60(20/40) | 48/49 | none | 100 | 85/50(45) | -12/-5(-3) | -10/0(2) |
| 4 | 21.9 | M | L | 13 | 7.1 | 12 | 115(70/45) | 50(15/35) | 36/45 | mild | 65 | 70/55(60) | -13/0(5) | 0/5(7) |
| 5 | 42.4 | M | L | 62 | 9.7 | 10 | 120(60/60) | 65(25/40) | 53/60 | none | 90 | 65/45(50) | 0/10(13) | -2/11(15) |
| 6 | 18.4 | M | L | 3 | 6.5 | 9 | 160(80/80) | 75(25/50) | 37/45 | none | 90 | 50/45(46) | 0/3(5) | 2/5(10) |
| 7 | 24.2 | M | L | 18 | 9.1 | 12 | 135(60/75) | 70(20/50) | 43/50 | none | 90 | 65/45(48) | -5/0(3) | -2/2(3) |
| 8 | 27.3 | M | L | 110 | 8 | 9 | 140(70/70) | 65(15/50) | 27/30 | none | 90 | 75/58(55) | -11/5(3) | -5/10(12) |

*: evaluated at most recent follow-up.

**: F; flexion. E; extension.

***: R; radial deviation. U; ulnar deviation

The patients were 7 males and 1 female. The injury was on the right side in 2 cases and on the left side in 6 cases. The period from the injury to the surgical operation was 28 months on average (3 to 110 months). In 4 cases, no initial treatment was performed. In 2 cases, the scaphoid nonunion was overlooked at the first examination. In 1 case, the initial treatment was discontinued by the patient's own will.

The site of the nonunion was a 1/3 portion at the center of the bone in all 8 cases. Osteoarthritis was not found in the preoperative simple x-ray. In all 8 cases, the patients complained of moderate pain when using the affected limb before the surgical operation.

(Three-dimensional simulation for a surgical operation on the scaphoid nonunion using CT data)

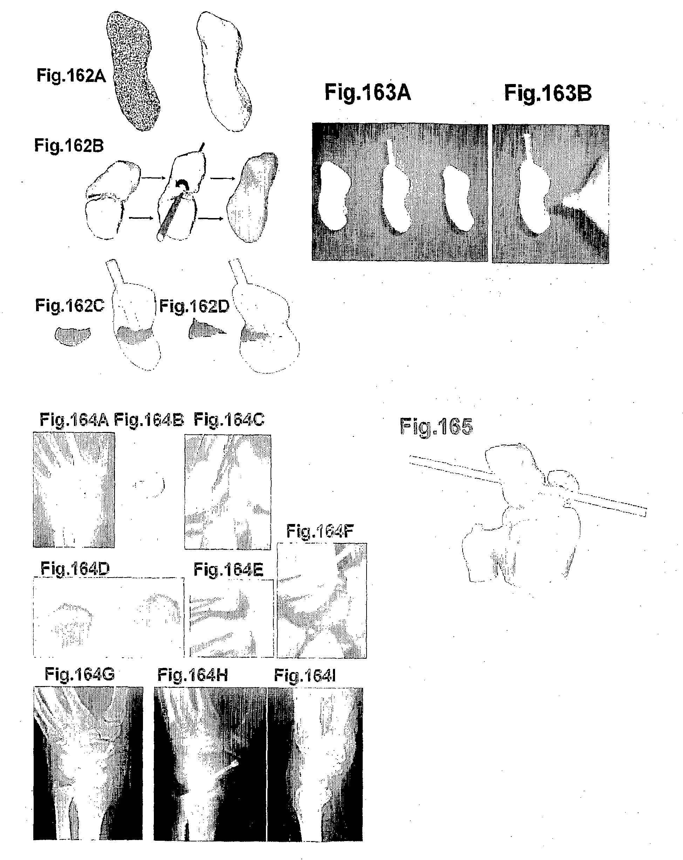

The patient was laid face-down and both arms were raised over the head to take a CT scan image of both the wrist joints at a slice pitch of 0.625 to 1.0 mm (High Speed Advance or LightSpeed Ultra 16 available from General Electrics). The image was stored as DICOM (Digital Imaging and Communications in Medicine) data. In order to maintain the wrist joint at an intermediate position, a splint formed of a radioparent material was attached during the imaging. The profile of the scaphoid was semi-automatically segmented using image analysis software (Virtual Place M®,Medical Imaging Laboratory, Tokyo). A three-dimensional bone surface model was constructed from the surface of the cortical bone using a Marching Cubes algorithm. A three-dimensional model of the scaphoid was visualized using a computer program developed by the present inventor (Figure 162A).

A distal portion and a proximal portion of the nonunion model were matched to an mirror image model of the model of the opposite scaphoid on a graphic workstation using an ICP alrorithm, which is one of the most advanced algorithms for surface matching. In this method, position matching is performed as follows: calculations are started from the initial position at which a three-dimensional model and a set of three-dimensional points are roughly matchedmanually, and a parameter by which the sum of the distances from each three-dimensional points to the surface is minimal. The distal portion and the proximal portion of the nonunion model were matched to the distal portion and the proximal portion of the mirror image of the scaphoid of the healthy side. The rotation of the distal portion with respect to the proximal portion was calculated using the Screw Displacement-Axis method. Thus, the present inventor simulated restoration of the deformed scaphoid nonunion (Figure 162B).

The estimated defect was calculated by subtracting the restored nonunion model from the mirror image of the scaphoid on the opposite, healthy side by the Boolean operation using commercial available computer software (Magics RP®, Materialise, Belgium). Thus, an appropriate site and direction of screw insertion were obtained by a simulation. The simulation was performed by attempting screw insertion on the computer screen and then making the model semi-transparent or visualizing the cross-section of the nonunion model (Figures 162C and 162D).

In order to easily reproduce the computer simulation in an actual surgical operation, full-size photo printout models of the bones (hard models) were produced based on the CAD data and used as guides during the surgical operation (Figures **163A** and **163B)**. The produced photo printout models were a deformed scaphoid nonunion model, a mirror image model of the normal scaphoid, a post-restoration nonunion model after the screw insertion was simulated, and a model of an estimated bone graft. These hard models were produced of an epoxy resin at an accuracy of 0.01 mm (CMET, Co., Ltd., Yokohama, Japan).

(Procedure of surgical operation)

A surgical operation was performed by a volar approach as follows. The articular capsule of the radiocarpal joint was incised in the direction of a longer axis of the bone to expose the volar surface of the scaphoid. The site of nonunion was compared with the hard model (Figures **164A** through **164C**). By extending the wrist joint, the profile of the volar cortical bone of the scaphoid nonunion was matched to the shape of the photo printout model mimicking the restoration. Thus, restoration was performed. The cured bone at the site of nonunion was excised until bleeding was confirmed. Then, a bone graft was sampled from the iliac bone. In consideration of that the defect is larger by the size of the cured bone than the size of the defect calculated based on the simulation, an actual bone graft was molded to be slightly larger than the hard model reproducing the shape of the defect (Figures **164D** and **164E**). The bone graft was trimmed and inserted into the defect, and then a trial Kirschner wire having a diameter of 1.2 mm was inserted at an appropriate position and in an appropriate direction with reference to the screw insertion positions and directions for the hard model (Figure **164F)**. The position of the Kirschner wire was confirmed, and then a second Kirschner wire having a diameter of 1.0 to 1.2 mm was inserted along the first Kirschner wire. The first Kirschner wire was removed and a double threaded screw was inserted into the hole made by the first Kirschner wire to realize internal fixation (Figure **164G).**

The wrist joint was fixed for 4 weeks after the surgical operation, and a wrist joint split was attached until the bone union was confirmed on the x-ray (Figures **164H** and **164I**).

(Evaluation of x-rays)

In order to evaluate the carpal alignment, the radio-lunate angle (RLA), scapho-lunate angle (SLA), and capito-lunate angle (CLA) were measured before and after the surgical operation and at the time of the final examination. If the fracture line disappeared and the bone trabeclae continuity was confirmed, the bone was evaluated as "united". The change in the arthrosis was evaluated before the surgical operation and at the most recent follow-up survey. If the gap of the articulatio became slightly narrower and/or styloid prominence on the radius side was pointed, the arthrosis was classified as "mild". If the gap of the mid-carpal joint was narrowed and pointed, the arthrosis was classified as "moderate". If a screw was inserted along the longer axis of the scaphoid in the postoperative x-ray, it was evaluated that "screw insertion is appropriate".

(Clinical evaluation)

An evaluation system by Cooney et al. was adopted (Cooney, WP, Linscheid RL., Dobyns JH., Wood MB., J Hand Surg 1988; 13A: 635-650). The ache, function, movable range of the wrist joint, and the grip strength of each case were scored with the percentage thereof with respect to the normal values (score of 0 to 25 points). The scores in the four categories were added together. If the sum of the scores is 65 or higher, the result is evaluated as satisfactory. The result of x-ray examination was not included in the scores.

(Results)

(Preoperative three-dimensional analysis)

The distal portion of the scaphoid was rotated with respect to the proximal portion at an average of 39.7° (18.7° to 73.9°). In all the cases, the Screw Displacement-Axis about which the distal portion of the scaphoid was rotated with respect to the proximal portion passed through the head of the capitate and ran from the ulnar side to the radius side (Figure **165**). The estimated bone defect had a prismatic shape having a base on the volar side and the apex on the dorsal side. The defect had an anterior thickness of 5.5 mm on average (3.8 to 7.3 mm), a depth of 9.7 mm on average (7.5 to 12.1 mm), and a width of 10.7 mm on average (9.1 to 12.8 mm) as summarized in Table II.

Table II: Results of three-dimensional computer simulation

**Table II. Results of preoperative 3D computer**

| Case | Volar rotation of the distal fragment (degrees) | Estimated bone defect (mm) | | |
|---|---|---|---|---|
| | | anterior thickness | depth | width |
| 1 | 24.5 | 4 | 8.9 | 9.1 |
| 2 | 70.2 | 6.7 | 8.6 | 12.8 |
| 3 | 28.6 | 4.3 | 8.9 | 10.7 |
| 4 | 36.9 | 6 | 11.3 | 9.2 |
| 5 | 40.8 | 4 | 8.1 | 10 |
| 6 | 23.9 | 6.7 | 12.1 | 12.7 |
| 7 | 18.7 | 5.3 | 11.9 | 11.3 |
| 8 | 73.9 | 7.3 | 7.5 | 9.6 |

The appropriate site for screw insertion was slightly to the ulnar side and to the dorsal side with respect to the center of the tubercle of the scaphoid. The photo printout models were very useful during the surgical operation as good guides for the present inventor. Using the hard models, the present inventor could restore the nonunion, place the graft, and insert the screw during the surgical operation in accordance with the preoperative plan.

(Result of radiographic examination)

In all the cases , fine bone union was obtained before 9.6 weeks on average (8 to 12 weeks) after the surgical operation. Before the surgical operation, the SL angle, RL angle, and CL angle were respectively 69.4°, -6.0°, and -3.8° on average. After the surgical operation, the SL angle, RL angle, and CL angle were respectively 49.1°, 2.8°, and 3.9° (Table I). The SL angle, RL angle, and CL angle of the opposite, normal side were respectively 49.9°, 5.0° and 8.6°(Table I). The parameters of the x-rays did not change even at the most recent follow-up survey. The postoperative x-rays did not show progressive osteoarthritis. In all the cases , the screw was appropriately inserted as planned before the surgical operation.

(Clinical results)

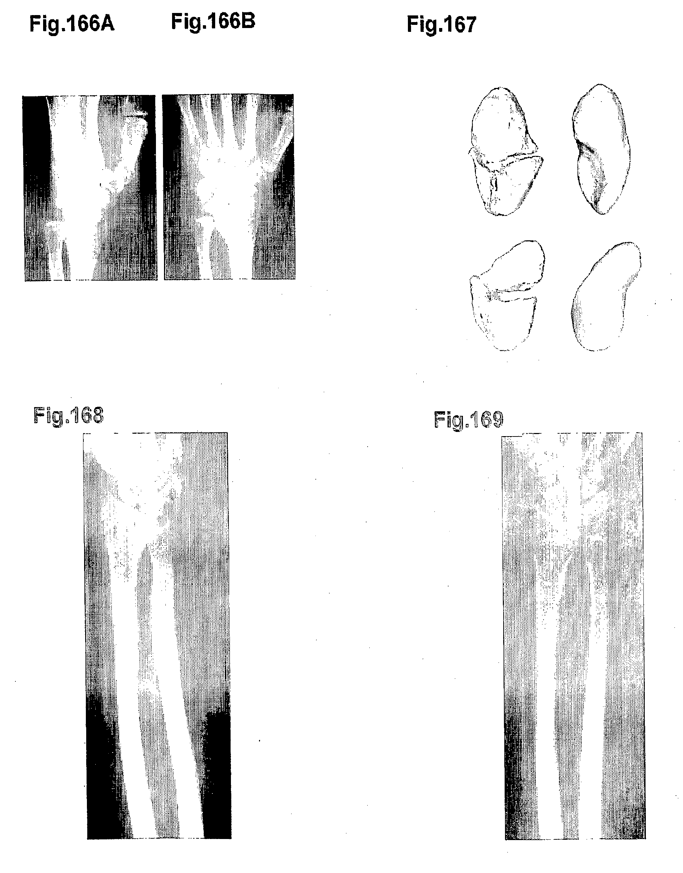

The postoperative score for the wrist joint was 65 to 100, with an average of 89. The movable range of flexion and extension was 138 degrees on average (115 to 160 degrees), and the movable range of the radius and the ulna was 64 degrees on average (50 to 75 degrees). The grip strength was 80 to 100%, witch the average being 90%, of that of the healthy side (Table I). In 1 case (case 4), fine bone union was observed at the most recent follow-up survey but the patient complained of the ache when extending the wrist joint and the restriction of the movable range. In this case, osteophyte was observed on the dorsal side of the scaphoid in the three-dimensional model but was not clear in the simple x-ray (Figures **166A** and **166B** and Figure **167**). This osteophyte was considered to be the cause for the slightly lower clinical score (65) of this case. In the other cases, no ache or functional disorder was observed at the most recent follow-up survey.

(Analysis)

Treatment of deformed scaphoid nonunion is still difficult. It is important to firmly maintain bone union and normal carpal alignment in order to realize good postoperative functions. Conventionally, importance of anatomical restoration has been strongly discussed. Patients having a facture malunion of the scaphoid have a higher incidence of experiencing osteoarthritis and functional disorder of the wrist joint than patients having anatomical union of the scaphoid. It is considered that the radiocarpal joint is made to easily develop into osteoarthritis due to the change in the stress applied on the articulatio and the incongruity of the articular surface. Tsuyuguchi et al. studied patients who experienced a surgical operation of scaphoid nonunion, and reported that the patients having normal carpal alignment after the surgical operation exhibited significantly better functional scores than the patients , whose postoperative SL angle was increased to show DISI deformity (Tsuyuguchi Y, Murase T, Hidaka N, Ohno H, Kawai H, J Hand Surg 1995; 20B: 194-200). Amadio et al. reported that for successful treatment of a fracture of the scaphoid, mere bone union was not sufficient but anatomical restoration of the scaphoid was important (Amadio PC., Berquist TH., Smith DK., Ilstrup DM., Cooney WP., Linscheid RL., JHandSurg1989; 14A: 679-687). In simulation studies using cadaver samples, the extension of the wrist joint was reduced in proportion to the volar deformity of the scaphoid; and the extension of the wrist joint became 0 degrees when the volar deformity of the scaphoid was 30 degrees (Burgress RC, The effect of simulated scaphoid malunion on wrist motion, J Hand Surg 1987; 12A: 774-6).
Comprehensively considering the knowledge obtained by these studies, it is desirable to perform amaximumpossible anatomically accurate restoration for treatment of scaphoid nonunion.
In 1970, Fisk advocated sampling a wedge-shaped bone graft having a volar projection from the styloid prominence using a lateral approach (Fisk GR. , Ann R. , Surg Engl 1970; 46: 63-76). Fernandez et al. improved the surgical method of Fisk and recommended inserting a wedge-shaped or trapezoidal iliac bone graft by a volar approach for internal fixation (Fernandez DL., J Hand Surg 1984; 9A: 733-737). They planned the size of the graft using x-rays of a front view and a side view thereof. Nakamura et al. (Nakamura R. , Hori M., Horii E, Miura T., J Hand Surg 1987; 12A: 1000-1005) and Tomanio et al. (Tomanio MM., King J. , Pizillo M. J Hand Surg 2000; 25A: 322-329) pierced the lunate with a Kirschner wire and filled the defect of the scaphoid, generated by correction of the DISI deformity, with the anterior wedge-shaped bone graft.
The method of Fernandez using the preoperativex-rays is simple, but it is difficult to rely on two-dimensional images to plan a surgical operation on a scaphoid nonunion having a complicated three-dimensional shape. It is reasonable to evaluate the deformity using three-dimensional information in order to plan a surgical operation on scaphoid nonunion.
In 1991, Nakamura et al. reported that three-dimensional CT is useful for evaluating the deformity of a scaphoid nonunion and classifying the deformity patterns into two categories (volar and dorsal) (Nakamura R. , Imaeda T., Miura T., Scaphoid malunion., J Bone Joint Surg [Bg] 1991; 73B: 134-137). Morimoto et al. further investigated the relationship among the fracture site, deformity pattern, generation of DISI deformity, and change in the contact area of the articulatio in the radiocarpal joint using three-dimensional models, and clarified the mechanism of deformed scaphoid nonunion. These researchers qualitatively visualized the fracture pattern and the dislocation of bone fragments using three-dimensional images or three-dimensional models.
Belsole et al. calculated the deformity angle and the volume of the bone defect by overlapping computer images of a normal scaphoid and a fractured scaphoid, and reported that the proximal portion exhibited extension, radial deviation, and supination with respect to the distal portion (Belsole RJ. , Hilberlink DR. , Llewellyn JA. , Dale M. , Greene TL. , Rayhack JM., J Hand Surg 1991; 16A: 899-906). The amount of the bone defect was 6 to 15% of the volume of the scaphoid, and the bone defect had a prismatic shape having a base on the volar side. The three-dimensional technology used by the present inventor in this example fundamentally uses the basic technology used by Belsole et al. Whereas the present inventor used the calculation results for the purpose of treatment, Belsole et al. used the calculation results for understanding the pathologic mechanism of the scaphoid nonunion. Belsole et al. does not suggest the present invention, since Belsole et al. provides no description on the technique for finding the bone defect and does not simulate screw insertion or the like. Accordingly, the present invention would not have been obvious to those skilled in the art based on Belsole et al.

According to the present method, the deformity could be measured three-dimensionally and accurately. The amount of displacement of the distal portion with respect to the proximal portion of the scaphoid nonunion was represented as the rotation about the screw axis, and visualized as a three-dimensional image. The present inventor could know the estimated bone defect and the appropriate site and direction of screw insertion before the surgical operation. The estimated bone defect had a triangular shape having a base on the volar side and a width of 4 to 7 mm. In order to reflect the result of the preoperative simulation on the actual surgical operation, a full-size plastic model produced by photo printout was used as a guide. This guide was found to be useful to obtain the orientation during the surgical operation, restore the deformed nonunion, mold a bone graft, and determine an appropriate position and direction for screw insertion. The carpal alignment in the postoperative x-ray was good in 7 cases. The clinical result was excellent or good except for 1 case (in which slight osteoarthritis was observed in a three-dimensional image).

Another advantage of using a three-dimensional image is that a very small morphological change such as a small osteophyte, which cannot be detected by a simple x-ray, can be observed. In a three-dimensional image in 1 case used in this study, an osteophyte on the dorsal side was observed which cannot be found in a simple x-ray. In this case, the patient complained of a lasting pain at the wrist joint after the surgical operation, despite the DISI deformity shown in an x-ray indicating sufficient correction by the surgical operation. The reason that the symptom was left is considered to be that the radiocarpal joint had already had osteoarthritis before the operation.

A three-dimensional computer simulation was found to be useful to realize correct correction of scaphoid nonunion and maintain the normal carpal alignment in order to obtain good clinical results.

(Example 11: Combination of closed wedge osteotomy and open wedge osteotomy)

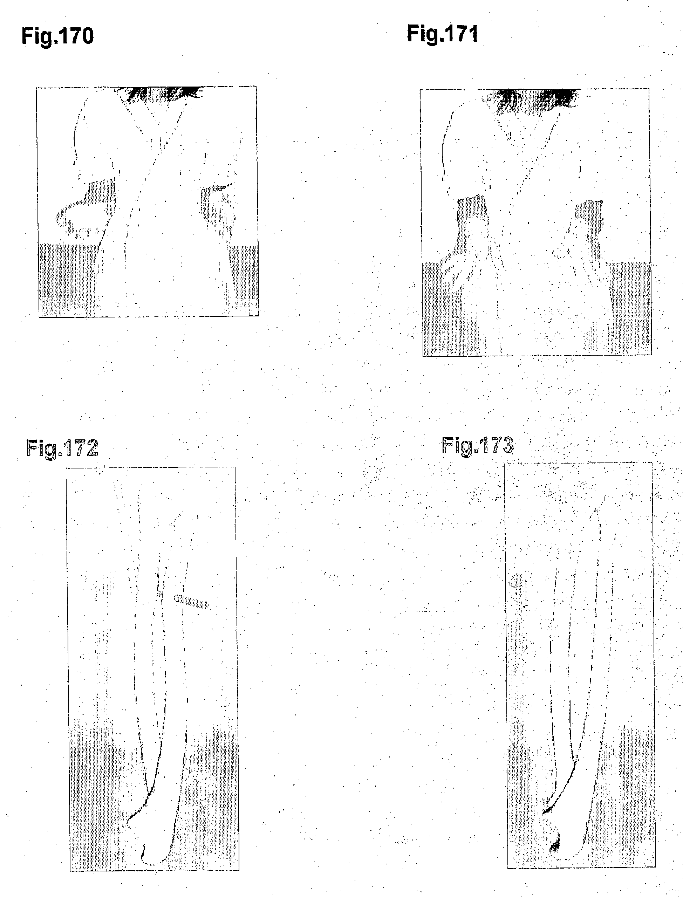

In this example it will be demonstrated that the present method is applicable even to a combination of closed wedge osteotomy and open wedge osteotomy. As an example, a 15-year-old woman is used. This patient had a fracture on the left forearm at the age of 7. She complained of the restriction of the supination of the forearm and received an examination. Figure 168 shows an x-ray of the left forearm. As shown here, the ulna is internally curved as compared to the normal state (see the arrow). Figure 169 shows an x-ray of the normal side. As shown in Figure **170,** this patient could not perform supination of the left forearm. As shown in Figure 171, the pronation of the patient was slightly restricted.

(Three-dimensional computer simulation)

A three-dimensional computer simulation was performed in the same manner as shown in the above examples. As a result, it was found that the screw axis of the ulna was substantially vertical to the ulna and passed through the center of the ulna. A distal portion of the ulna exhibited radial deviation about the screw axis at 13 degrees. The screw axis of the radius was substantially parallel to the longer axis of the radius. A distal portion of the radius was internally deformed about the screw axis at 46 degrees. Figure 172 shows a three-dimensional model and the radius of the affected side. The screw axis of the ulna is shown with the substantially horizontal line. Figure 173 shows a mirror image model of the healthy side.

(Plan for correction osteotomy for ulna)

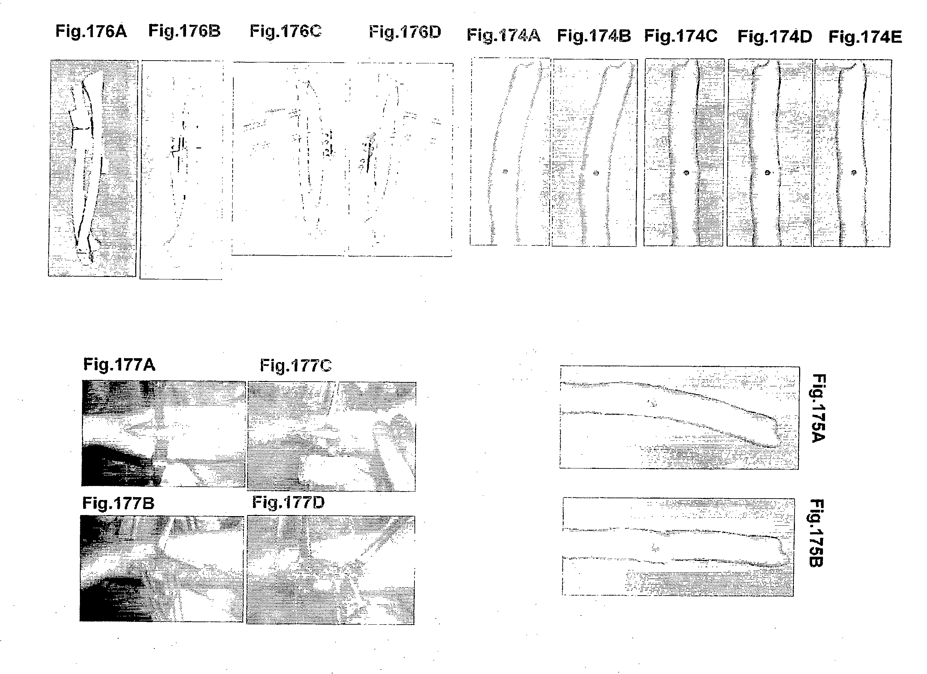

Next, a plan for correction osteotomy was made in the same manner as shown in the above examples. In the case where the screw axis passed through the ulna and was substantially vertical to the axis of the ulna, a combination of closed wedge osteotomy and open wedge osteotomy was planned. Figures **174A** through 174E show the plan. As shown in Figure **174A,** the closed wedge osteotomy was planned. The point represents the screw axis seen from the side thereof. As shown in Figure **174B,** the bone was cut along the plane passing through the screw axis and a wedge-shaped bone having an angle of 13 degrees was excised. As shown in Figure **174C,** the correction osteotomy was performed. As in Figures 174D and **174E,** a wedge-shaped graft was implanted in the defect which is generated after the correction.

Although not performed in this example, similar correction osteotomy can be realized by cutting the bone along an arch having the screw axis at the center as shown in Figures **175A** and **175B** to provide a dome-shaped bone and rotating the bone fragment. Figure **175** schematically shows such a procedure. As shown in Figure **175A,** the bone is cut along an arch of an appropriate radius having the screw axis at the center. As shown in Figure 175B, the upper bone fragment is rotated at 13 degrees. It is understood that correction is realized in this manner.

(Designing of an osteotomy template and a correction guide)

The radius was planned to be treated with rotational osteotomy as in Example 7, and a template was designed. Figures **176A** through **176D** schematically show the procedure. Figures **176A** and **176B,** respectively show the osteotomy template seen from the dorsal side and from the volar side. Figures **176C** and **176D,** respectively show the correction guide seen from the dorsal side and from the volar side.

Figures **177A** through **177D** show photos of correction osteotomy of ulna during the surgical operation. As shown in Figure **177A,** the deformed site of the ulna was developed. As shown in Figure **177B**, the osteotomy template was applied to the site and fixed with Kirschner wires. Notably, the wires are angled at 13 degrees. As shown in Figure 177C, after the bone was cut, the correction guide was outserted into the Kirschner wires for performing correction. The Kirschner wires were arranged to be parallel to each other. The bone fragments were fixed with a metal plate in the state shown Figure **177D.** A bone defect generated on the side closer to the operator was filled with a wedge-shaped bone excised from the deeper side.

(Results)

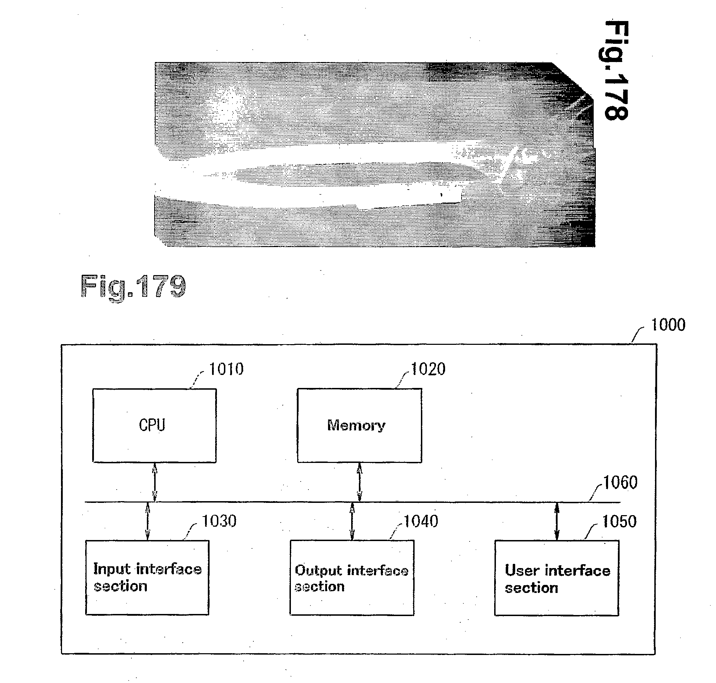

As shown in Figure **178,** the correction was performed as shown in the postoperative x-ray and also as simulated by a combination of closed wedge osteotomy and open wedge osteotomy.

(Example 12: Computer and computer program)

Figure **179** shows an exemplary structure of a computer **1000** for executing processing for determining a treatment process to be performed on a bone.

The computer **1000** includes a CPU **1010,** a memory **1020,** an input interface **1030,** an output interface **1040,** a user interface **1050,** and a bus **1060.**

The CPU **1010** executes a program.

The memory **1020** stores a program and other data required for executing the program.

The input interface **1030** acts as an interface for receiving data from an imaging apparatus (external apparatus) such as, for example a CT apparatus or an MRI apparatus.

The output interface **1040** acts as an interface for outputting data to an assisting member molding apparatus (external apparatus) such as a resin block molding apparatus.

The user interface **1050** acts as an interface for controlling interaction with the user. The user interface 1050 can be connected to an input device such as , for example, a keyboard or a mouse or an output device such as, for example, a display device or a printing device.

The bus **1060** is used for connecting the CPU **1010,** the memory **1020,** the input interface **1030,** the output interface **1040,** and the user interface **1050** to each other.

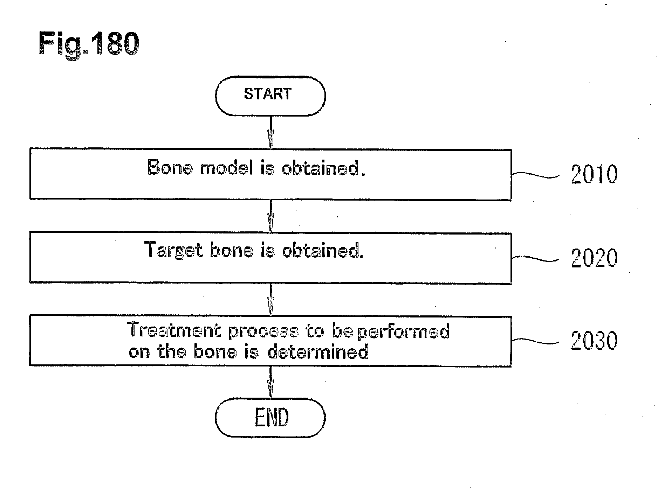

Figure **180** shows an exemplary procedure of processing for determining a treatment process to be performed on a bone. This processing is provided in the form of a program. The program is executed by the CPU **1010.**

In step **2010,** a bone model representing a bone which is a subject of treatment is obtained.

The bone model can be represented by a three-dimensional model which represents a three-dimensional structure of the bone. The bone which is a subject of treatment is representatively an affected bone, but is not limited to this. The bone model is obtained, for example, as follows. Data which is output from an imaging apparatus such as a CT apparatus or an MRI apparatus through the input interface **1030,** extracting data representing the bone from the received data, and generating a surface model of the bone based on the extracted data. The bone model may be obtained by other methods, and may be obtained by any appropriate method. For example, a bone model may be obtained by reading a surface model of the bone recorded on any type of recording medium, or by reading a surface model of the bone stored in the memory **1020.**

In step **2020,** a target bone model to which treatment aims is obtained.

The target bone model can be represented by a three-dimensional model which represents a three-dimensional structure of the bone. The target bone is representatively a normal bone, but is not limited to this. The target bone model is obtained by, for example generating a surface model of the bone using a mirror image of the bone model on the healthy side. The target bone model may be obtained in other methods, and may be obtained in any appropriate method. For example, a target bone model may be obtained by reading a surface model of the target bone recorded on any type of recording medium, or by reading a surface model of the target bone stored in the memory **1020.** Alternatively, a model arbitrarily created by the user (for example, the operator) may be used as a target bone model.

In steps **2010** and **2020 ,** software usable for generating a surface model of the bone, is for example, Virtual Place M (Medical Imaging Laboratory, Ltd., Tokyo), Mimics (Materalise, Belgium), Zview™ (ZviewInc., Huntington Beach, CA., USA), or Realize (Mayo Clinic), but is not limited to these. The surface model of the bone may be stored in the memory **1020** in the format of VTK, STL or the like.

In step **2030,** a treatment process to be performed on the bone is determined based on the bone model and the target bone model.

The treatment process to be performed on the bone includes, for example, cutting the bone along a cutting section, and moving one of a proximal bone fragment and a distal bone fragment obtained by cutting the bone in a certain direction by a certain amount. In this case, the treatment process to be performed on the bone is determined by determining the cutting section of the bone model and determining the direction and amount of moving one of the proximal bone fragment and the distal bone fragment.

The treatment process to be performed on the bone may or may not require an assisting member. In the case where the treatment process requires an assisting member, the step of determining a treatment process which is to be performed on the bone includes the step of determining the assisting member required for the treatment process. The required assistingmembermaybe, for example, at least one of a template assisting member, a correction position determination assisting member and a graft. As the assisting member required for the treatment process, an external fixation device may be used.

The computer **1000** may provide a method for determining a treatment process to be performed on the bone by the CPU 1010 executing the program for performing the processing for determining the treatment process to be performed on the bone.

The computer **1000** may act as an apparatus for determining a treatment process to be performed on the bone by the CPU **1010** executing the program for performing the processing for determining the treatment process to be performed on the bone. In the above example the steps **2010** through **2030** are implemented by software, but the present invention is not limited to this. The functions provided by steps **2010** through **2030** may be implemented by hardware (for example, circuits, boards, semiconductor chips), or by a combination of software and hardware. Accordingly, any apparatus including (A) means for obtaining a bone model representing a bone which is a subject of treatment; (E) means for obtaining a target bone model to which treatment aims; (C) means for determining a treatment process which is to be performed on the bone based on the bone model and the target bone model is encompassed in the scope of the present invention.

The program for executing the processing for determining the treatment process to be performed on the bone may be provided to the user in any form. For example, the program may be provided to the user by distributing a recording medium having the program recorded thereon or by the user downloading the program from a server to a terminal through a network. The program may be provided with or without charge. As the recording medium having the program recorded thereon, any recording medium such as a flexible disc, an MO disc, a DVD or the like is usable. As the network any network such as, for example, the Internet is usable.

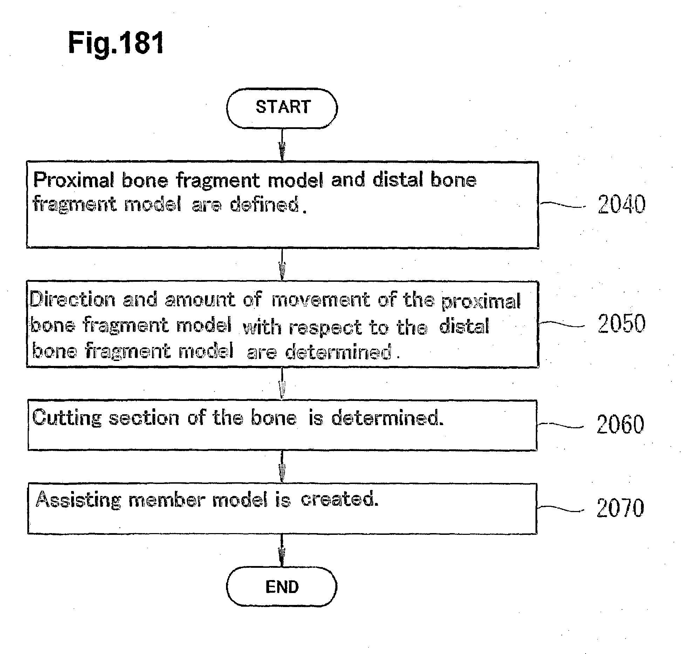

Figure **181** shows an exemplary procedure for performing step **2030** shown in Figure **180.** This procedure is performed by a program which is executed by the CPU **1010.**

In step **2040,** a proximal bone fragment model and a distal bone fragment model are defined from the bone model.

This is performed as follows. For example, a proximal portion of the bone model is overlapped on a proximal portion of the target bone model. A portion in which the proximal portions of the two bone models are distanced from each other by a prescribed distance (e.g., 1 mm) is ignored. A portion of the proximal portion of the bone model which matches the proximal portion of the target bone model within an error of a prescribed distance (e.g., 1 mm) is defined as a "proximal bone model".

For example, a distal portion of the bone model is overlapped on a distal portion of the target bone model. A portion in which the distal portions of the two bone models are distanced from each other by a prescribed distance (e.g., 1 mm) is ignored. A portion of the distal portion of the bone model which matches the distal portion of the target bone model within an error of a prescribed distance (e.g., 1 mm) is defined as a "distal bone model".

The proximal portion of the bone model and the proximal portion of the target bone model (or the distal portion of the bone model and the distal portion of the target bone model) can be overlapped by, for example, using a program for controlling and managing position information of the surface model. Such a program can be easily created based on an open source of, for example, VTK (The Visualization ToolKit) (http://public.kitware.com/VTK/).

In order to accurately match the two models, for example, a program referred to as an ICP (Iterative Closest Point) algorithm using a surface matching technique is used. In order to execute this program for the bone model and the target bone model, it is preferable to set parameters for the program such that portions in which the bone model and the target bone model are distanced from each other by a prescribed distance (e.g., 1 mm) are ignored and portions in which the bone model and the target bone model are within the prescribed distance are made effective. The reason is that where the bone is deformed, the bone model and the target bone model do not substantially match each other.

In step **2050,** the relative moving direction and the moving amount of the distal bone fragment model with respect to the proximal bone fragment model are determined.

The relative movement of the distal bone fragment model with respect to the proximal bone fragment model is represented in accordance with, for example, the Screw Displacement-Axis method.

The principle of the Screw Displacement-Axis method will be described with reference to Figure **8.** There is a unique axis, for one movement of an object in space, for which the following is true: When an object is rotated about the unique axis by an angle $\phi$ and moved parallel to the axis by distance **t,** the object can be moved to any position. Therefore, the relative movement of the distal bone fragment model with respect to the proximal bone fragment model can be represented by determining one axis, the rotating angle $\phi$ about the axis and the moving distance **t** along the axis. In many of the actual clinical uses, the moving distance **t** along the axis is sufficiently small to be negligible (e.g., within 1 mm). In such a case, it is sufficient to consider the rotating angle $\phi$ about the axis.

The relative movement of the distal bone fragment model with respect to the proximal bone fragment model may be represented in accordance with the affine transformation method.

In step **2060,** the cutting section of the bone model is determined.

The cutting section of the bone model may be determined by the computer 1000 by executing a program, or by an instruction of the user (e.g., a physician). Such an instruction is, for example, input to the CPU **1010** via the user interface **1050** by the user operating the mouse. The instruction may be of any type or any manner. For example, the user may instruct the position of the cutting section of the bone to the computer **1000** in any manner well known in three-dimensional graphics.

Alternatively, the computer **1000** may support the user instructing a cutting section of the bone model as follows. One or more candidates for the cutting section of the bone model are displayed on a display device together with the bone model and the target bone model, and the user is permitted to select one of the candidates (or the user is permitted to modify one of the candidates). Such a candidate or candidates can be displayed by storing the history of instructions issued by the user in the memory **1020.**

For example, it is assumed that the relative movement of the distal bone fragment model with respect to the proximal bone fragment model is represented by the screw axis **L,** the rotating angle $\phi$ about the screw axis **L,** and the moving distance **t** in accordance with the Screw Displacement-Axis method. In this case, it is desirable to determine the cutting section of the bone model in accordance with whether the screw axis **L** is substantially parallel or vertical to the longer axis of the bone model. The cutting section of the bone model may be determined by the computer **1000** by executing a program or in accordance with an instruction from the user (e.g., a physician) under the support of the computer **1000.**

For example, when the screw axis L is substantially parallel to the longer axis of the bone model, it is preferable to adopt rotational osteotomy and determine a plane vertical to the screw axis **L** as the cutting section of the bone model. For example, when the screw axis **L** is substantially vertical to the longer axis of the bone model, it is preferable to adopt closed/operation wedge osteotomy and determine a plane parallel to the screw axis **L** as the cutting section of the bone model.

Alternatively, one or more candidates for the cutting section of the bone model may be displayed together with the bone model and the target bone model in accordance with whether the screw axis **L** is substantially parallel or vertical to the longer axis of the bone model. By displaying the candidate or candidates for the cutting section of the bone model, the user can more easily determine the cutting section of the bone model. For example, when the screw axis **L** is substantially parallel to the longer axis of the bone model, it is conceivable to adopt rotational osteotomy and display one or more planes vertical to the screw axis **L** as the candidate (s) for the cutting section of the bone model. For example, when the screw axis **L** is substantially vertical to the longer axis of the bone model, it is conceivable to adopt closed/operation wedge osteotomy and display one or more planes parallel to the screw axis **L** as the candidate(s) for the cutting section of the bone model.

As described above, in step **2040**, the proximal bone fragment model and the distal bone fragment model are defined. In step **2050,** the direction and the amount (distance) of movement of the distal bone fragment model with respect to the proximal bone fragment model are determined. In step **2060,** the cutting section of the bone model is determined. Thus, the treatment process to be performed on the bone (bone cutting, movement of a bone fragment, etc.) can be determined.

In step **2070,** a model representing an assisting member (assisting member model) is created based on the direction and the amount of movement of the distal bone fragment model with respect to the proximal bone fragment model, and the cutting section of the bone model. When the assisting member is not required for the treatment process to be performed on the bone, the step **2070** may be omitted.

The assisting member model may be represented by three-dimensional data which represents a three-dimensional structure of the assisting member. The assisting member model may be stored in the memory **1020** in a format of, for example, STL. The STL format is commonly used for rapid prototyping such as photo printout or the like. By the STL format, the data representing the surface is polygonized using triangles. The assisting member model is output to an assisting member molding apparatus via the output interface **1040.** The assisting member molding apparatus produces an assisting member corresponding to the assisting member model by molding a material (e.g. , metal, plastics , ceramics) by any molding method such as, for example, photo printout.

The assisting member model may be, for example, a model representing a template assisting member (template assisting member model). The template assisting member model includes a fitting surface to be fit to the bone model, a cutting section guide for guiding the cutting section of the bone model, and a plurality of insertion guides for guiding a plurality of models (a plurality of rod models) representing a plurality of rods into the bone model. One example of the template assisting member model is shown in Figure **11** as the three-dimensional model **M1** of the osteotomy assisting member **1.** Alternatively, the template assisting member model may include at least one of the fitting surface, the cutting section guide and a plurality of insertion guides.

According to the invention the cutting section guide is a slit formed at such a position that corresponds to the cutting section of the bone model and the plurality of insertion guides are a plurality of guide holes, into which the plurality of rod models can be inserted. More specifically, the plurality of insertion guides are preferably a plurality of guide holes formed such that the plurality of rod models are substantially parallel to each other when the proximal bone fragment model and the distal bone fragment model are in a normal positional relationship.

For example, an initial model of the template assisting member is created so as to include a cutting section. By performing a Boolean operation on the initial model and the bone model (by subtracting the overlapping portion of the initial model and the bone model from the initial model), a template assisting member model having a fitting surface can be created.

For example, an initial model of the template assistingmember is created so as to include a cutting section. By performing a Boolean operation on the initial model and the cutting surface (by subtracting the overlapping portion of the initial model and the cutting section from the initial model), a template assisting member model having a slit at such a position as to correspond to the cutting section can be created.

For example, an initial model of the template assisting member is created so as to include a cutting section. By performing a Boolean operation on the initial model and the plurality of rod models (by subtracting the overlapping portion of the initial model and the plurality of rod models from the initial model), a template assisting member model having a plurality of guide holes into which the plurality of rod models can be inserted can be created.

As describe above, a template assisting member model having at least one of a slit or a plurality of guide holes can be created by performing a Boolean operation on the initial model of the template assisting mode and a prescribed model (a bone model, a cutting section, or a plurality of rod models) . The Boolean operation is one modeling technique in three-dimensional graphics. Usable software for performing a Boolean operation may be, for example, Magics RP (Materialise) which is commercially available, but examples are not limited to this. The initial model of the template may be created manually by instruction from the user, or semi-automaticallyby instruction from the user (for example, creating an initial model of a certain thickness including an area designated by the user as a fitting surface).

The assisting member model may be, for example, a model representing a correction position determination assisting member (position confirmation assisting member model). The correction position determination assisting member model is used for, after the bone model is corrected into the target bone model, confirming that the proximal bone fragment model and the distal bone fragment model are in a normal positional relationship. The correction position determination assisting member model has, for example, a plurality of guide holes which are formed such that, where the proximal bone fragment model and the distal bone fragment model are in a normal positional relationship, the plurality of rod models are substantially parallel to each other. One example of the correction position determination assisting member model is shown in Figure **12** as the three-dimensional block model **M3.**

For example, an initial model of the correction position determination assisting member is created at a position distanced from the bone model and the target bone model, and a Boolean operation is performed (i.e., an overlapping portion of the initial model and the plurality of rod members is subtracted from the initial model). Thus, a correction position determination assisting member model having a plurality of guide holes into which the plurality of rod models can be inserted can be created.

The assisting member model may be, for example, a model representing a graft (graft model). For example, the graft model can be created by obtaining a difference between the bone model and the target bone model.

In step **2070,** the method for using the assisting member may be determined instead of creating the assisting member model. For example when the external fixation device is used as the assisting member, the method for using the external fixation device (e.g., the position of the axis of the external fixation device, the position of the hinge) may be determined.

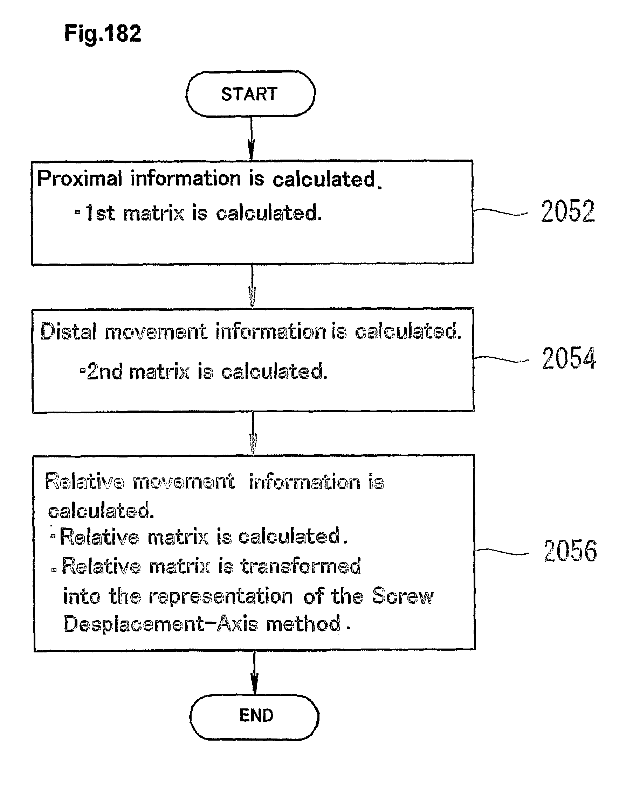

Figure **182** shows an exemplary procedure of the processing for determining the direction and the amount of movement of the distal bone fragment model with respect to the proximal bone fragment model. This processing is one example for performing the step **2050** shown in Figure **181.** The processing may be implemented by executing a program. The program is executed by the CPU **1010.**

In step **2052,** proximal movement information representing a direction and an amount of relative movement of the proximal fragment bone model which is required to match the proximal portion of the bone model to the proximal portion of the target bone model is found by calculation.

The proximal movement information can be found by, for example, matching the proximal bone fragment model of the bone model with the corresponding portion of the target bone model. Such a matching can be performed using a program adopting a surface matching technique referred to the ICP (Iterative Closest Point) algorithm mentioned above. The proximal movement information may be represented by a matrix (hereinafter, referred to as the "first matrix") in accordance with the affine transformation method.

In step **2054,** distal movement information representing a direction and an amount of relative movement of the distal fragment bone model which is required to match the distal portion of the bone model to the distal portion of the target bone model is found by calculation.

The distal movement information can be found by, for example matching the distal bone fragment model of the bone model with the corresponding portion of the target bone model. Such a matching can be performed using a program adopting a surface matching technique referred to the ICP (Iterative Closest Point) algorithm mentioned above. The distal movement information may be represented by a matrix (hereinafter, referred to as the "second matrix") in accordance with the affine transformation method.

In step **2056,** relative movement information representing the direction and the amount of movement of the proximal bond fragment model with respect to the distal bond fragment model is found by calculation through a difference between the proximal movement information and the distal movement information.

For example, in the case where the proximal movement information is represented by the first matrix in accordance with the affine transformation method and the distal movement information is represented by the second matrix in accordance with the affine transformation method, the relative movement information can be found by first finding a relative matrix by obtaining a difference between the first matrix and the second matrix and then transforming the relative matrix to the representation by the Screw Displacement-Axis method (i.e. , representation by the screw axis $L$, the rotating angle $\phi$, and the moving distance $t$ along the screw axis $L$).

The relative matrix represents the direction and the amount of movement of the distal bone fragment model with respect to the proximal bone fragment model in accordance with the affine transformation method. The screw axis $L$, the rotating angle $\phi$, and the moving distance $t$ along the screw axis $L$ represent the direction and the amount of movement of the distal bone fragment model with respect to the proximal bone fragment model in accordance with the Screw Displacement-Axis method. Accordingly, transformation of the relative matrix into the representation by the Screw Displacement-Axis method means transforming the relative movement information by the representation of the affine transformation method into the relative movement information by the representation of the Screw Displacement-Axis. Such transformation of the relative movement information is equivalent to representing a movement of an object in a space by a different coordinate system.

Thus, by the program according to the present invention, the treatment process (e.g., bone cutting, movement of bone fragments) to be performed on a bone for achieving a desired purpose (e.g., correction of a bone) can be determined. Thus, even a physician with little experience can perform the treatment appropriately.

Although certain preferred embodiments have been described herein, it is not intended that such embodiments be construed as limitations on the scope of the invention except as set forth in the appended claims. Various other modifications and equivalents will be apparent to and can be readily made by those skilled in the art, after reading the description herein, without departing from the scope of this invention. All patents , published patent applications and publications cited herein are incorporated by reference as if set forth fully herein.

INDUSTRIAL APPLICABILITY

As described so far, the present invention, when used for correction osteotomy surgical operations, significantly improves the accuracy and ease of the surgical operations and contributes to the remarkable technological development in the art.

**Claims**

1. An osteotomy assisting member (1) for assisting in cutting and dividing a bone deformed in an abnormal form into bone fragments and for changing and correcting a positional relationship of the bone fragments into a positional relationship of a bone in a normal form, the osteotomy assisting member comprising:

   a positioning element (1) having a fitting surface (11), which is formed to fit the surface of the bone to be cut and divided into bone fragments; and
   a cutting slit (12) formed at a position which corresponds to a cutting section along which the cutting and dividing of the bone are to be performed with the positioning element (1) applied to the surface of the bone, the cutting slit (12) having a shape capable of guiding a cutting jig towards the cutting section;

wherein the osteotomy assisting element (1) is provided with a plurality of guide holes (13) configured to guide attachment of a plurality of rods (2) to the bone fragments, said rods (2) formed as to pierce a bone; and wherein the plurality of guide holes (13) is arranged such that each bone fragment can be provided with at least two rods (2) pierced therein; and a block (3) provided with a plurality of insertion holes (31);

**characterised in that** each of said insertion holes (31) has a shape permitting one of the rods (2), which is attached to the respective bone fragment, to be inserted there through, when the bone fragments are rotated relative to each other about an axis vertical to the plane of the cutting section.

2. An apparatus for making an osteotomy assisting member for determining bone treatment for cutting and dividing a bone deformed in an abnormal form and changing and correcting a positional relationship of the divided bones into a positional relationship of a bone in a normal form, the apparatus comprising:

A) a bone model obtaining means for obtaining, from any imaging device for imaging a bone which is a subject of treatment, three-dimensional data of the bone, and based on the obtained three-dimensional data, obtaining a three-dimensional bone model representing the bone which is the subject of treatment;
B) a target bone model obtaining means for obtaining a target bone model which is a target of treatment upon correction of the bone; and
C) as a treatment determining means for determining, based on the bone model and the target bone model, a treatment process which is to be performed on the bone which is the subject of treatment:

C1) a bone fragment model movement determining means for setting a correction cutting section for the bone model and creating bone fragment models obtained by cutting the bone model at the cutting section, **characterised in that**
the target bone model obtaining means obtains the target bone mode by use of a mirror image of the bone model of a normal side;
the bone fragment model movement determining means determines, as a correction position, a position where a difference in shape between the bone fragment models and the target bone model is smallest; and the treatment determining means further comprises:
C2) a rod model attaching means for simulating a state in which at least one model of a rod (2) formed so as to pierce a bone is attached respectively to the bone fragment model at the correction position and a bone fragment model, on another side of the cutting section at the correction position, created by cutting the bone model,
C3) a rod model position calculating means for, based on positions of the respective rod models attached to the respective bone fragment models by the rod model attaching means, calculating positions of the respective rod models in a case where each of the bone fragment models is at a position of the bone model, and
C4) an osteotomy assisting member model creating means for creating an osteotomy assisting member model having a fitting surface fitting a surface of the bone model having the cutting section, the osteotomy assisting member model having a slit formed at a portion corresponding to the cutting section and having guide holes formed at portions corresponding to the respective rod models for inserting the respective rod models.

3. An apparatus according to claim 2, further including

C5) a block model creating means for creating a block model being formed with insertion holes (31) for inserting the respective rod models at portions corresponding to the respective rod models attached to the respective bone fragment models by the rod model attaching means at the corrected position.

4. A program for making an osteotomy assisting member for determining bone treatment for cutting and dividing a bone deformed in an abnormal form and changing and correcting a positional relationship of the divided bones into a positional relationship of a bone in a normal form, wherein the program makes a computer serve as:

A) a bone model obtaining means for obtaining, from any imaging device for imaging a bone which is a subject of treatment, three-dimensional data of the bone, and based on the obtained three-dimensional data, obtaining a three-dimensional bone model representing the bone which is the subject of treatment;
B) a target bone model obtaining means for obtaining, by use of a mirror image of the bone model of a normal side, a target bone model which is a target of treatment upon correction of the bone; and
C) as a treatment determining means for determining, based on the bone model and the target bone model, a treatment process which is to be performed on the bone which is the subject of treatment:

C1) a bone fragment model movement determining means for setting a correction cutting section for the bone model, creating bone fragment models obtained by cutting the bone model at the cutting section, and determining, as a correction position, a position where a difference in shape between the bone fragment models and the target bone model is smallest,

C2) a rod model attaching means for simulating a state in which at least one model of a rod (2) formed so as to pierce a bone is attached respectively to the bone fragment model at the correction position and a bone fragment model, on the other side of the cutting section at the correction position, created by cutting the bone model,

C3) a rod model position calculating means for, based on positions of the respective rod models attached to the respective bone fragment models by the rod model attaching means, calculating positions of the respective rod models in a case where each of the bone fragment models is at a position of the bone model, and

C4) an osteotomy assisting member model creating means for creating an osteotomy assisting member model having a fitting surface fitting a surface of the bone model having the cutting section, the osteotomy assisting member model having a slit formed at a portion corresponding to the cutting section and having guide holes formed at portions corresponding to the respective rod models for inserting the respective rod models.

5. The program according to claim 4, further making a computer serve as:

C5) a block model creating means for creating a block model being formed with guide holes (31) for inserting the respective rod models at portions corresponding to the respective rod models attached to the respective bone fragment models by the rod model attaching means at the correction position.

**Patentansprüche**

1. Osteotomieassistiervorrichtung (1) zum Assistieren beim Schneiden und Teilen eines Knochens, der in einer abnormen Form verformt ist, in Knochenfragmente und zum Ändern und Korrigieren einer Positionsbeziehung der Knochenfragmente in eine Positionsbeziehung eines Knochens in einer normale Form, wobei die Osteotomieassistiervorrichtung umfasst:

ein Positionierelement (1) mit einer passenden Oberfläche (11), welche ausgebildet ist, um zur Oberfläche des Knochens passen, der geschnitten und in Knochenfragmente geteilt werden soll; und

einen Schneidespalt (12) ausgebildet an einer Position, die einem Schneidebereich entspricht, entlang dem das Schneiden und Teilen des Knochens mit dem auf der Knochenoberfläche angebrachten Positionierelement (1) durchgeführt werden sollen, wobei der Schneidespalt (12) eine Form aufweist, die in der Lage ist, eine Schneidevorrichtung in Richtung des Schneidebereichs zu führen;

wobei die Osteotomieassistiervorrichtung (1) mit einer Vielzahl von Führungslöchern (13) versehen ist, die ausgelegt sind, die Befestigung einer Vielzahl von Stäben (2) an die Knochenfragmente zu führen, wobei die Stäbe (2) zum Durchbohren eines Knochens ausgebildet sind; und wobei die Vielzahl der Führungslöchern (13) so angeordnet sind, dass jedes Knochenfragment mit wenigstens zwei hineingebohrten Stäben (2) versehen werden kann; und einen Block (3) versehen mit einer Vielzahl von Insertionslöchern (31);

**dadurch gekennzeichnet, dass** jedes der Insertionslöcher (31) eine Form aufweist, welche es einem der Stäbe (2), der an dem jeweiligen Knochenfragment befestigt ist, ermöglicht, dort hindurchgeführt zu werden, wenn die Knochenfragmente im Verhältnis zueinander um eine Achse, die senkrecht zu der Ebene des Schneidebereichs steht, gedreht werden.

2. Apparat zum Herstellen einer Osteotomieassistiervorrichtung zum Festlegen einer Knochenbehandlung zum Schneiden und Teilen eines Knochens, der in einer abnormen Form verformt ist, und Verändern und Korrigieren einer Positionsbeziehung der geteilten Knochen in eine Positionsbeziehung eines Knochens in einer normale Form, wobei der Apparat umfasst:

A) ein Mittel zum Erhalten eines Knochenmodells zum Erhalten von dreidimensionalen Daten des Knochens von einer bildgebenden Vorrichtung zum Abbilden eines zu behandelnden Knochens, und basierend auf den erhaltenen dreidimensionalen Daten, Erhalten eines dreidimensionalen Knochenmodells, welches den zu behandelnden Knochen darstellt;

B) ein Mittel zum Erhalten eines Zielknochenmodells zu Erhalten eines Zielknochenmodells welches ein Behandlungsziel bei der Korrektur des Knochens ist; und

C) als ein Mittel zum Festlegen der Behandlung zum Festlegen basierend auf dem Knochenmodell und dem Zielknochenmodell eines Behandlungsprozesses, welcher an dem zu behandelnden Knochen durchgeführt werden soll:

C1) ein Mittel zum Festlegen der Bewegung eines Knochenfragmentmodells zum Festsetzen eines Korrekturschneidebereichs für das Knochenmodell und Erstellen von Knochenfragmentmodellen, welche durch Schneiden des Knochenmodells im Schneidebereich erhalten werden,

**dadurch gekennzeichnet, dass**

das Mittel zu Erhalten des Zielknochenmodells das Zielknochenmodell durch Verwenden eines Spiegelbildes des Knochenmodells einer normalen Seite erhält;

das Mittel zum Festlegen der Bewegung des Knochenfragmentmodells als eine Korrekturposition ein Position festlegt, wo ein Unterschied in der Form zwischen den Knochenfragmentmodellen und dem Zielknochenmodell am kleinsten ist; und

das Mittel zum Festlegen der Behandlung weiterhin umfasst:

C2) ein Mittel zum Befestigen eines Stabmodells zum Simulieren eines Zustandes worin wenigstens ein Modell eines zum Durchbohren eines Kochens ausgebildeten Stabs (2) jeweils an das Knochenfragmentmodell in der Korrekturposition und an ein Knochenfragmentmodell auf einer weiteren Seite des Schneidebereichs in der Korrekturposition befestigt ist, erstellt durch Schneiden des Knochenmodells,

C3) Mittel zum Berechnen einer Stabmodellposition zum Berechnen von Positionen der jeweiligen Stabmodelle im einem Fall, wo sich jedes der Knochenfragmentmodelle an der Position des Knochenmodells befindet, basierend auf Positionen der jeweiligen Stabmodelle befestigt an den jeweiligen Fragmentmodellen durch die Mittel zum Befestigen der Stabmodelle, und

C4) ein Mittel zum Erstellen eines Modells einer Osteotomieassistiervorrichtung zum Erstellen eines Modells einer Osteotomieassistiervorrichtung, welche eine passende Oberfläche aufweist, die zu einer Oberfläche des Knochenmodells passt, welches den Schneidebereich aufweist, wobei das Modell der Osteotomieassistiervorrichtung einen Spalt aufweist, der in einem Abschnitt ausgebildet ist, die dem Schneidebereich entspricht, und Führungsbohrungen aufweist, die in Abschnitten ausgebildet sind, die jenen der jeweiligen Stabmodellen zum Einführen der jeweiligen Stabmodelle entsprechen.

3. Einrichtung nach Anspruch 2 weiterhin umfassend

C5) ein Mittel zum Erstellen eines Blockmodells zum Erstellen eines Blockmodells, welches mit Insertionslöchern (31) zum Einführen der jeweiligen Stabmodelle in Abschnitten versehen ist, die jenen der jeweiligen Stabmodellen entsprechen, die an den jeweiligen Knochenfragmentmodellen mit den Mitteln zum Befestigen der Stabmodelle in der korrigierten Position befestigt sind.

4. Programm zum Herstellen einer Osteotomieassistiervorrichtung zum Festlegen einer Knochenbehandlung zum Schneiden und Teilen eines Knochens, der in einer abnormen Form verformt ist, und Verändern und Korrigieren einer Positionsbeziehung der geteilten Knochen in eine Positionsbeziehung eines Knochens in eine normale Form, wobei das Programm einen Computer veranlasst zu fungieren als:

A) ein Mittel zu Erhalten eines Knochenmodells zum Erhalten von dreidimensionalen Daten des Knochens von einer bildgebenden Vorrichtung zum Abbilden eines zu behandelnden Knochens, und basierend auf den erhaltenen dreidimensionalen Daten, Erhalten eines dreidimensionalen Knochenmodells, welches den zu behandelnden Knochen darstellt;

B) ein Mittel zum Erhalten eines Zielknochenmodells zu Erhalten eines Zielknochenmodells, welches ein Behandlungsziel bei der Korrektur des Knochens ist, durch Verwendung eines Spiegelbildes des Knochenmodells von einer normalen Seite; und

C) als ein Mittel zum Festlegen der Behandlung zum Festlegen eines Behandlungsprozesses, welcher an dem zu behandelnden Knochen durchgeführt werden soll, basierend auf dem Knochenmodell und dem Zielknochenmodell;

C1) ein Mittel zum Festlegen der Bewegung eines Knochenfragmentmodells zum Festsetzen eines Korrekturschneidebereichs für das Knochenmodell, wobei Knochenfragmentmodelle erstellt werden, welche durch Schneiden des Knochenmodells im Schneidebereich erhalten wurden, und Festlegen als eine Korrekturposition eine Position, wo ein Unterschied in der Form zwischen den Knochenfragmentmodell und dem Zielknochenfragment am kleinsten ist,

C2) ein Mittel zum Befestigen eines Stabmodells zum Simulieren eines Zustandes worin wenigstens ein Modell eines zum Durchbohren eines Knochens ausgebildeten Stabes (2) jeweils an das Knochenfragmentmodell in der Korrekturposition und an ein Knochenfragmentmodell auf der anderen Seite des Schneidebereichs in der Korrekturposition befestigt ist, erstellt durch Schneiden des Knochenmodells,

C3) Mittel zum Berechnen einer Stabmodellposition zum Berechnen von Positionen der jeweiligen Stabmodelle im einem Fall, wo sich jedes der Knochenfragmentmodelle an einer Position des Knochenmodells befindet, basierend auf Positionen der jeweiligen Stabmodelle befestigt an den jeweiligen Knochenfragmentmodellen durch Mittel zum Befestigen der Stabmodelle, und

C4) ein Mittel zum Erstellen eines Modells einer Osteotomieassistiervorrichtung zum Erstellen eines Modells einer Osteotomieassistiervorrichtung, welches eine passende Oberfläche aufweist, die zu einer Oberfläche des Knochenmodells passt, welches einen Schneidebereich aufweist, wobei das Modell der Osteotomieassistiervorrichtung einen Spalt aufweist, der in einem Abschnitt ausgebildet ist, der dem Schneidebereich entspricht, und Führungslöcher aufweist, die in Abschnitten ausgebildet sind, die jenen der jeweiligen Stabmodellen zum Einführen der jeweiligen Stabmodelle entsprechen.

5. Programm nach Anspruch 4 welches den Computer weiterhin veranlasst zu fungieren als:

C5) ein Mittel zum Erstellen eines Blockmodells zum Erstellen eines Blockmodells, welches mit Insertionslöchern (31) zum Einführen der jeweiligen Stabmodelle in Abschnitten versehen ist, die jenen der jeweiligen Stabmodellen entsprechen, die an den jeweiligen Knochenfragmentmodellen mit den Mitteln zum Befestigen der Stabmodelle in der korrigierten Position befestigt sind.

## Revendications

1. Elément d'assistance en ostéotomie (1) pour porter assistance dans la coupe et la division d'un os déformé sous forme anormale en fragments osseux et pour modifier et corriger une relation de position des fragments osseux en une relation de position d'un os sous forme normale, l'élément d'assistance en ostéotomie comprenant :

un élément de positionnement (1) ayant une surface d'ajustement (11) qui est formée pour s'ajuster à la surface de l'os à couper et à diviser en fragments osseux ; et

une fente de coupe (12) formée dans une position qui correspond à une section de coupe le long de laquelle la coupe et la division de l'os doivent être effectuées avec l'élément de positionnement (1) appliqué à la surface de l'os, la fente de coupe (12) ayant une forme capable de guider un gabarit de coupe vers la section de coupe ;

dans lequel l'élément d'assistance en ostéotomie (1) est pourvu d'une pluralité de trous de guidage (12) configurés pour guider la fixation d'une pluralité de tiges (2) aux fragments osseux, lesdites tiges (2) étant conformées pour percer un os ; et dans lequel la pluralité de trous de guidage (13) est aménagée de sorte que chaque fragment osseux puisse être pourvu d'au moins deux tiges (2) qui le traversent ; et

un bloc (3) pourvu d'une pluralité de trous d'insertion (31) ;

**caractérisé en ce que** chacun desdits trous d'insertion (31) a une forme permettant d'insérer l'une des tiges (2), qui est fixée au fragment osseux respectif, à travers ces trous lorsque les fragments osseux sont soumis à une rotation l'un par rapport à l'autre autour d'un axe vertical au plan de la section de coupe.

2. Appareil de fabrication d'un élément d'assistance en ostéotomie afin de déterminer un traitement osseux pour couper et diviser un os déformé sous forme anormale et modifier et corriger une relation de position des os divisés en une relation de position d'un os sous forme normale, l'appareil comprenant :

A) un moyen d'obtention d'un modèle d'os pour obtenir, à partir de n'importe quel dispositif d'imagerie pour imager un os qui fait l'objet du traitement, des données tridimensionnelles de l'os et pour, sur la base des données tridimensionnelles obtenues, obtenir un modèle d'os tridimensionnel représentant l'os qui fait l'objet du traitement ;

B) un moyen d'obtention d'un modèle d'os cible pour obtenir un modèle d'os cible qui est une cible de traitement lors de la correction de l'os ; et

C) un moyen de détermination de traitement pour déterminer, sur la base du modèle d'os et du modèle d'os cible, un procédé de traitement qui doit être effectué sur l'os qui fait l'objet du traitement ;

C1) un moyen de détermination du mouvement du modèle de fragment osseux pour régler une section de coupe de correction pour le modèle d'os et créer des modèles de fragments osseux obtenus en coupant le modèle d'os à la section de coupe,
**caractérisé en ce que** :

le moyen d'obtention d'un modèle d'os cible donne le modèle d'os cible en utilisant une image symétrique du modèle d'os d'un côté normal ;
le moyen de détermination du mouvement de modèle de fragment osseux détermine, comme position de correction, une position dans laquelle la différence de forme entre les modèles de fragments osseux et le modèle d'os cible est la plus faible ; et
le moyen de détermination de traitement comprend en outre :

C2) un moyen de fixation de modèle de tige pour simuler un état dans lequel au moins un modèle de tige (2) formé de manière à percer un os est fixé respectivement au modèle de fragment osseux dans la position de correction et à un modèle de fragment osseux, sur un autre côté de la section de coupe dans la position de correction, créé en coupant le modèle d'os,
C3) un moyen de calcul de position du modèle de tige pour, sur la base des positions des modèles de tiges respectifs fixés aux modèles de fragments osseux respectifs par le moyen de fixation de modèle de tige, calculer les positions des modèles de tiges respectifs dans un cas où chacun des modèles de fragments osseux se trouve dans une position du modèle d'os, et
C4) un moyen de création de modèle d'élément d'assistance en ostéotomie pour créer un modèle d'élément d'assistance en ostéotomie ayant une surface d'ajustement s'ajustant sur une surface du modèle d'os ayant la section de coupe, le modèle d'élément d'assistance en ostéotomie ayant une fente formée dans une partie correspondant à la section de coupe et ayant des trous de guidage formés dans des parties correspondant aux modèles de tiges respectifs pour insérer les modèles de tiges respectifs.

3.   Appareil selon la revendication 2, comprenant en outre :

C5) un moyen de création de modèle de bloc pour créer un modèle de bloc formé avec des trous d'insertion (31) pour insérer des modèles de tiges respectifs dans des parties correspondant aux modèles de tiges respectifs fixés aux modèles de fragments osseux respectifs par le moyen de fixation de modèles de tiges dans la position corrigée.

4.   Programme de fabrication d'un élément d'assistance en ostéotomie pour déterminer un traitement osseux pour couper et diviser un os déformé sous forme anormale et modifier et corriger une relation de position des os divisés en une relation de position d'un os sous forme normale, dans lequel le programme dessert l'ordinateur par les moyens suivants :

A) un moyen d'obtention d'un modèle d'os pour obtenir, à partir de n'importe quel dispositif d'imagerie permettant d'imager un os qui fait l'objet du traitement, des données tridimensionnelles de l'os et, sur la base des données tridimensionnelles obtenues, obtenir un modèle d'os tridimensionnel représentant l'os qui fait l'objet du traitement ;
B) un moyen d'obtention de modèle d'os cible pour obtenir, par l'utilisation d'une image symétrique du modèle d'os d'un côté normal, un modèle d'os cible qui est une cible de traitement lors de la correction de l'os ; et
C) un moyen de détermination de traitement pour déterminer, sur la base du modèle d'os et du modèle d'os cible, un procédé de traitement qui doit être effectué sur l'os qui fait l'objet de traitement ;

C1) un moyen de détermination de mouvement de modèle de fragment osseux pour régler une section de coupe de correction pour le modèle d'os, en créant des modèles de fragments osseux obtenus en coupant le modèle d'os dans la section de coupe et pour déterminer, comme position de correction, une position dans laquelle la différence de forme entre les modèles de fragments osseux et le modèle d'os cible est la plus petite,
C2) un moyen de fixation de modèle de tige pour simuler un état dans lequel au moins un modèle de tige (2) formé de manière à percer un os est fixé respectivement au modèle de fragment osseux dans la position de correction et à un modèle de fragment osseux, sur l'autre côté de la section de coupe dans la position de correction, créé en coupant le modèle d'os,

C3) un moyen de calcul de position du modèle de tige pour, sur la base des positions des modèles de tiges respectifs fixés aux modèles de fragments osseux respectifs par le moyen de fixation de modèle de tige, calculer les positions des modèles de tiges respectifs dans un cas où chacun des modèles de fragments osseux se trouve dans une position du modèle d'os, et

C4) un moyen de création de modèle d'élément d'assistance en ostéotomie pour créer un modèle d'élément d'assistance en ostéotomie ayant une surface d'ajustement s'ajustant sur une surface du modèle d'os ayant la section de coupe, le modèle d'élément d'assistance en ostéotomie ayant une fente formée dans une partie correspondant à la section de coupe et ayant des trous de guidage formés dans des parties correspondant aux modèles de tiges respectifs pour insérer les modèles de tiges respectifs.

5. Programme selon la revendication 4, desservant en outre l'ordinateur par le moyen suivant :

C5) un moyen de création de modèle de bloc pour créer un modèle de bloc formé avec des trous d'insertion (31) pour insérer les modèles de tiges respectifs dans des parties correspondant aux modèles de tiges respectifs fixés aux modèles de fragments osseux respectifs par le moyen de fixation de modèles de tiges dans la position corrigée.

**Fig.1**

1

13

13

12

13

13

**Fig.2**

1

13

13

12

13

13

**Fig.3**

2

**Fig.4**

3

31

31

31

31

**Fig.5**

M3

L

M2

M21

M22

**Fig.6**

M2a(MT)

L

M21

M22

**Fig.7**

M2

L

M21

P

M22

**Fig.8**

L

z

t

θ

y

x

**Fig.9**

M2a

M21

MR

P

MR

M22

**Fig.10**

M2

M21

MR

P

MR

M22

**Fig.11**

M2

M21

MR

P

M1

MR

M22

**Fig.12**

M2a

M21

M3

MR

MR

M22

Fig.13

Fig.14

Fig.15

Fig.16

**Fig.17**

**Fig.18**

**Fig.19**

**Fig.20**

**Fig.21**

**Fig. 22A**

**Fig. 22C  Fig. 22D**

**Fig. 22B**

**Fig.23**

Mirror image model of the healthy side

Malunion Model

Amount of deformity

Screw axis

**Fig.24**

## Fig.25A   Fig.25B   Fig.25C

## Fig.26

| Result | Group A | Group B |
|---|---|---|
| No. of cases | 3 | 3 |
| Age | 19.1 years | 19.2 years |
| Injury to operation term | 12.5 months | 10.7 months |
| Follow-up term | 10.3 months | 3.0 months |
| Deformity angle | | |
| Preoperative | 25° | 20.7° |
| Postoperative | 9.3° | 0.3° |
| Pronation/supination movable range | | |
| Preoperative | 73.3° | 120° |
| Postoperative | 146.7° | 180° (only one case was evaluated) |

Fig.27

Fig.28

Fig.29

Fig.30

Fig.32

Fig.34

Fig.31

Fig.33

Fig.36

Fig.38

Fig.35

Fig.37

Fig.40

Fig.42

Fig.39

Fig.41

Fig.43

Fig.44

Fig.45

Fig.46

Fig.48

Fig.50

Fig.47

Fig.49

Fig.52

Fig.54

Fig.51

Fig.53

Fig.56

Fig.58

Fig.55

Fig.57

Fig.59

Fig.60

Fig.61

Fig.62

Fig.63

Fig.64

Fig.65

Fig.66

Fig.68

Fig.70

Fig.67

Fig.69

Fig.71

Fig.72

Fig.73

Fig.74

Fig.76

Fig.75

Fig.78

Fig.77

Fig.80

Fig.82

Fig.79

Fig.81

Fig.84

Fig.86

Fig.83

Fig.85

Fig.89

Fig.88

Fig.87

Fig.91

Fig.90

EP 1 624 812 B1

Fig.93

Fig.95

Fig.92

Fig.94

Fig.97

Fig.96

Fig.99

Fig.98

72

Fig.100

Fig.101

Fig.102

Fig.103

EP 1 624 812 B1

Fig.105

Fig.104

Fig.107

Fig.106

Fig.108

Fig.109

Fig.110

Fig.111

Fig.113

Fig.112

Fig.114

Fig.115

Fig.117

Fig.119

Fig.116

Fig.118

77

Fig.121

Fig.120

Fig.122

Fig.123

EP 1 624 812 B1

Fig.125

Fig.124

Fig.127

Fig.126

Fig.129

Fig.131

Fig.128

Fig.130

Fig.133

Fig.135

Fig.132

Fig.134

Fig.137

Fig.136

Fig.139

Fig.138

Fig.141

Fig.143

Fig.140

Fig.142

**Fig.144**

**Fig.145**

**Fig.146**

**Fig.147**

Fig.148

Fig.149

Fig.150

Fig.151

Fig.153

Fig.154

Fig.152

Fig.155

**Fig.156**

**Fig.157**

**Fig.158**

**Fig.159**

Fig.160

Fig.161

**Fig.162A**

**Fig.162B**

**Fig.163A**      **Fig.163B**

**Fig.162C**  **Fig.162D**

**Fig.164A Fig.164B Fig.164C**

**Fig.165**

**Fig.164F**

**Fig.164D**      **Fig.164E**

**Fig.164G**      **Fig.164H**      **Fig.164I**

**Fig.166A**　　**Fig.166B**　　　　**Fig.167**

**Fig.168**　　　　　　　　　　　　**Fig.169**

**Fig.170**

**Fig.171**

Fig.172

Fig.173

EP 1 624 812 B1

**Fig.176A**  **Fig.176B**  **Fig.176C**  **Fig.176D**  **Fig.174A**  **Fig.174B**  **Fig.174C**  **Fig.174D**  **Fig.174E**

**Fig.177A**  **Fig.177C**

**Fig.177B**  **Fig.177D**

**Fig.175A**

**Fig.175B**

# Fig.178

Fig.179

# Fig.180

```
                    ┌─────────┐
                    │  START  │
                    └─────────┘
                         │
                         ▼
    ┌──────────────────────────────────────────┐
    │         Bone model is obtained.           │────  2010
    └──────────────────────────────────────────┘
                         │
                         ▼
    ┌──────────────────────────────────────────┐
    │         Target bone is obtained.          │────  2020
    └──────────────────────────────────────────┘
                         │
                         ▼
    ┌──────────────────────────────────────────┐
    │    Treatment process to be performed      │────  2030
    │      on the bone is determined            │
    └──────────────────────────────────────────┘
                         │
                         ▼
                    ┌─────────┐
                    │   END   │
                    └─────────┘
```

# Fig.181

```
        ┌─────────────┐
        │    START    │
        └──────┬──────┘
               │
               ▼
┌──────────────────────────────────┐
│ Proximal bone fragment model and  │
│ distal bone                       │       2040
│ fragment model are defined.       │
└──────────────┬───────────────────┘
               │
               ▼
┌──────────────────────────────────┐
│ Direction and amount of movement  │
│ of the proximal                   │
│ bone fragment model with respect  │       2050
│ to the distal                     │
│ bone fragment model are           │
│ determined.                       │
└──────────────┬───────────────────┘
               │
               ▼
┌──────────────────────────────────┐
│ Cutting section of the bone is    │       2060
│ determined.                       │
└──────────────┬───────────────────┘
               │
               ▼
┌──────────────────────────────────┐
│ Assisting member model is         │       2070
│ created.                          │
└──────────────┬───────────────────┘
               │
               ▼
        ┌─────────────┐
        │     END     │
        └─────────────┘
```

## Fig.182

START

Proximal information is calculated.
    ▫ 1st matrix is calculated.

2052

Distal movement information is calculated.
    ▫ 2nd matrix is calculated.

2054

Relative movement information is calculated.
    ▫ Relative matrix is calculated.
    ▫ Relative matrix is transformed into the representation of the Screw Desplacement-Axis method.

2056

END

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 4565191 A
- WO 0237935 A
- US 5021056 A

## Non-patent literature cited in the description

- Zoki Ishoku. Shin Gekagaku Taikei. Nakayama Shoten, vol. 12
- **S. OHWOVORIOLE ; B. ROTH.** An extension of screw theory. *Journal of Mechanical Design,* October 1981, vol. 103, 725-735
- **COONEY, WP ; LINSCHEID RL. ; DOBYNS JH. ; WOOD MB.** *J Hand Surg,* 1988, vol. 13A, 635-650
- **TSUYUGUCHI Y ; MURASE T ; HIDAKA N ; OHNO H ; KAWAI H.** *J Hand Surg,* 1995, vol. 20B, 194-200
- **AMADIO PC. ; BERQUIST TH. ; SMITH DK. ; ILSTRUP DM. ; COONEY WP. ; LINSCHEID RL.** *JHandSurg,* 1989, vol. 14A, 679-687
- **BURGRESS RC.** The effect of simulated scaphoid malunion on wrist motion. *J Hand Surg,* 1987, vol. 12A, 774-6
- **FISK GR. ; ANN R.** *Surg Engl,* 1970, vol. 46, 63-76
- **FERNANDEZ DL.** *J Hand Surg,* 1984, vol. 9A, 733-737
- **NAKAMURA R. ; HORI M. ; HORII E ; MIURA T.** *J Hand Surg,* 1987, vol. 12A, 1000-1005
- **TOMANIO MM. ; KING J. ; PIZILLO M.** *J Hand Surg,* 2000, vol. 25A, 322-329
- **NAKAMURA R. ; IMAEDA T. ; MIURA T.** Scaphoid malunion. *J Bone Joint Surg [Bg,* 1991, vol. 73B, 134-137
- **BELSOLE RJ. ; HILBERLINK DR. ; LLEWELLYN JA. ; DALE M. ; GREENE TL. ; RAYHACK JM.** *J Hand Surg,* 1991, vol. 16A, 899-906